(19) Europäisches Patentamt
European Patent Office
Office européen des brevets

(11) **EP 3 091 981 B1**

(12) # EUROPEAN PATENT SPECIFICATION

(45) Date of publication and mention of the grant of the patent:
**10.03.2021 Bulletin 2021/10**

(21) Application number: **15701875.5**

(22) Date of filing: **09.01.2015**

(51) Int Cl.:
***A61K 31/506*** *(2006.01)* ***A61K 9/16*** *(2006.01)*
***A61K 9/20*** *(2006.01)* ***A61P 11/00*** *(2006.01)*
***A61P 15/00*** *(2006.01)* ***A61P 35/00*** *(2006.01)*
***A61P 43/00*** *(2006.01)*

(86) International application number:
**PCT/US2015/010810**

(87) International publication number:
**WO 2015/106096 (16.07.2015 Gazette 2015/28)**

(54) **COMPOSITIONS AND METHODS FOR TREATMENT OF ABNORMAL CELL GROWTH**

ZUSAMMENSETZUNGEN UND VERFAHREN ZUR BEHANDLUNG VON ANORMALEM ZELLWACHSTUM

COMPOSITIONS ET MÉTHODES POUR LE TRAITEMENT D'UNE CROISSANCE CELLULAIRE ANORMALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.01.2014 US 201461925467 P**

(43) Date of publication of application:
**16.11.2016 Bulletin 2016/46**

(73) Proprietors:
- **Verastem, Inc.**
**Needham, MA 02494 (US)**
- **Pfizer Inc.**
**New York, NY 10017 (US)**

(72) Inventors:
- **PADVAL, Mahesh**
**Needham, MA 02494 (US)**
- **NKANSAH, Paul, Okwabi**
**Gaithersburg MD 20879-3492 (US)**

(74) Representative: **Harris, Jennifer Lucy et al**
**Kilburn & Strode LLP**
**Lacon London**
**84 Theobalds Road**
**London WC1X 8NL (GB)**

(56) References cited:
**WO-A1-2008/129380**

- **Y. KANG ET AL: "Role of Focal Adhesion Kinase in Regulating YB-1-Mediated Paclitaxel Resistance in Ovarian Cancer", JNCI JOURNAL OF THE NATIONAL CANCER INSTITUTE, vol. 105, no. 19, 2 October 2013 (2013-10-02), pages 1485-1495, XP055172119, ISSN: 0027-8874, DOI: 10.1093/jnci/djt210**
- **SHAMMA REHAB N ET AL: "Soluplus TM : A novel polymeric solubilizer for optimization of Carvedilol solid dispersions: Formulation design and effect of method of preparation", POWDER TECHNOLOGY, ELSEVIER SEQUOIA, LAUSANNE, CH, vol. 237, 29 December 2012 (2012-12-29), pages 406-414, XP028991212, ISSN: 0032-5910, DOI: 10.1016/J.POWTEC.2012.12.038**
- **WILLIAM CURATOLO ET AL: "Utility of Hydroxypropylmethylcellulose Acetate Succinate (HPMCAS) for Initiation and Maintenance of Drug Supersaturation in the GI Milieu", PHARMACEUTICAL RESEARCH, KLUWER ACADEMIC PUBLISHERS-PLENUM PUBLISHERS, NL, vol. 26, no. 6, 10 March 2009 (2009-03-10) , pages 1419-1431, XP019686188, ISSN: 1573-904X, DOI: 10.1007/S11095-009-9852-Z**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- JAMES C. DINUNZIO ET AL: "Production of advanced solid dispersions for enhanced bioavailability of itraconazole using KinetiSol Dispersing", DRUG DEVELOPMENT AND INDUSTRIAL PHARMACY, vol. 36, no. 9, 1 September 2010 (2010-09-01), pages 1064-1078, XP055172122, ISSN: 0363-9045, DOI: 10.3109/03639041003652973
- SHU LI ET AL: "Enhanced Bioavailability of a Poorly Water-Soluble Weakly Basic Compound Using a Combination Approach of Solubilization Agents and Precipitation Inhibitors: A Case Study", MOLECULAR PHARMACEUTICS, 13 April 2012 (2012-04-13), XP055172154, ISSN: 1543-8384, DOI: 10.1021/mp200352q
- Dwayne T. Friesen ET AL: "Hydroxypropyl Methylcellulose Acetate Succinate-Based Spray-Dried Dispersions: An Overview", Molecular Pharmaceutics, vol. 5, no. 6, 1 December 2008 (2008-12-01), pages 1003-1019, XP055056966, ISSN: 1543-8384, DOI: 10.1021/mp8000793
- Anonymous: "Pharma Ingredients & Services = Registered trademark of BASF group Soluplus Technical Information Supersedes issue dated May 2010 03_090801e-04/Page 1 of 8", , 4 July 2010 (2010-07-04), XP055420529, Retrieved from the Internet: URL:https://industries.basf.com/bin/bws/documentDownload.en.8805242743253 [retrieved on 2017-10-31]
- Edward T. Hellriegel ET AL: "Interpatient variability in bioavailability is related to the extent of absorption: Implications for bioavailability and bioequivalence studies", Clinical Pharmacology and Therapeutics, vol. 60, no. 6, 1 December 1996 (1996-12-01), pages 601-607, XP055584813, US ISSN: 0009-9236, DOI: 10.1016/S0009-9236(96)90208-8

## Description

### Field of Invention

This invention relates to compositions, for example oral dosage forms, and said compositions for use in the treatment of abnormal cell growth, such as cancer, in mammals, especially humans.

### Background of Invention

Convincing evidence suggests that focal adhesion kinase (FAK), a cytoplasmic, non-receptor tyrosine kinase, plays an essential role in cell-matrix signal transduction pathways (Clark and Brugge 1995, Science 268: 233-239) and its aberrant activation is associated with an increase in the metastatic potential of tumors (Owens et al. 1995, Cancer Research 55: 2752-2755). FAK was originally identified as a 125 kDa protein highly tyrosine-phosphorylated in cells transformed by v-Src. FAK was subsequently found to be a tyrosine kinase that localizes to focal adhesions, which are contact points between cultured cells and their underlying substratum and sites of intense tyrosine phosphorylation. FAK is phosphorylated and, thus, activated in response to extracellular matrix (ECM)-binding to integrins. Recently, studies have demonstrated that an increase in FAK mRNA levels accompanied invasive transformation of tumors and attenuation of the expression of FAK (through the use of antisense oligonucleotides) induces apoptosis in tumor cells (Xu et al. 1996, Cell Growth and Diff. 7: 413-418). In addition to being expressed in most tissue types, FAK is found at elevated levels in most human cancers, for example in highly invasive metastases.

Accordingly, a need exists for compounds (*e.g.*, inhibitors), compositions (*e.g,* formulations, *e.g.*, oral formulations (*e.g.*, oral dosage forms)), and methods of use of inhibitors of the non-receptor tyrosine kinase, FAK, for use in the treatment of abnormal cell growth. U.S. Pat. No. 8,247,411 relates to a broad class of novel pyrimidine derivatives that are kinase inhibitors, and more specifically, inhibitors of FAK. Compounds such as these may be useful in the treatment of abnormal cell growth.

WO 2008/129380 relates to sulfonyl amide derivatives for the treatment of abnormal cell growth. Kang et al. 2013, JNCI Journal of the National Cancer Institute 105: 1485-1495 relates to the "role of focal adhesion kinase in regulating yb-1-mediated paclitaxel resistance in ovarian cancer". Rehab et al. 2012, Powder Technology 237 : 406-414 relates to "Solupluse (R): a novel polymeric solubilizer for optimization of Carvedilol solid dispersions: formulation design and effect of method of preparation". Curatolo et al. 2009, Pharmaceutical Research 26 : 1419-1431 relates to the "utility of hydroxypropylmethylcellulose acetate succinate (HPMCAS) for initiation and maintenance of drug supersaturation in the GI milieu". Dinuzio et al. 2010, Drug Development and Industrial Pharmacy 36: 1064-1078 relates to the "production of advanced solid dispersions for enhanced bioavailability of itraconazole using KinetiSol® Dispersing". Friesen et al. 2008, Molecular Pharmaceuticals 5: 1003-1019 relates to "Hydroxypropyl Methylcellulose Acetate Succinate-based spray-dried dispersions". Soluplus® *Technical Information* 2010 discloses Soluplus. Hellriegel et al. 1996, Clinical Pharmacology and Therapeutics 60: 601-607 relates to "interpatient variability in bioavailability is related to the extent of absorption: implications for bioavailability and bioequivalence studies".

### Summary of the Invention

In one aspect, the present invention provides a pharmaceutical composition comprising VS-6063, or a pharmaceutically acceptable salt thereof, admixed with a polymer excipient, wherein the polymer excipient is hydroxypropylmethyl cellulose acetate succinate (HPMCAS), wherein the pharmaceutical composition is configured in an oral dosage form in the form of a tablet, wherein the tablet is provided by direct compression or dry granulation of a physical blend or mixture of VS-6063, or a pharmaceutically acceptable salt thereof, admixed with the polymer excipient.

In another aspect, the invention provides an oral dosage form comprising:

VS-6063, or a pharmaceutically acceptable salt thereof, optionally wherein VS-6063 is VS-6063 free base and/or the pharmaceutically acceptable salt is VS-6063 hydrochloride, in an amount from about 5 to 50% by weight on a dry weight basis,
a pharmaceutically acceptable filler, for example microcrystalline cellulose, optionally wherein the filler is microcrystalline cellulose PH 102, in an amount from about 10 to 30% by weight on a dry weight basis,
a second pharmaceutically acceptable filler, for example lactose, optionally wherein the filler is lactose monohydrate, in an amount from about 10 to 30% by weight on a dry weight basis,
HPMCAS, optionally wherein the HPMCAS is HPMCAS-HF, in an amount from about 25 to 55% by weight on a dry weight basis,
a pharmaceutically acceptable disintegrant, for example starch, optionally wherein the disintegrant is modified starch, optionally wherein the disintegrant is sodium starch glycolate, in an amount from about 0 to 5% by weight on a dry

weight basis, and
a pharmaceutically acceptable lubricant, for example magnesium stearate, in an amount from about 0.1 to 2% by weight on a dry weight basis;
optionally wherein the oral dosage form comprises:

VS-6063, or a pharmaceutically acceptable salt thereof, in an amount from about 10 to 15% by weight on a dry weight basis,
the pharmaceutically acceptable filler in an amount from about 15 to 25% by weight on a dry weight basis,
the second pharmaceutically acceptable filler in an amount from about 15 to 25% by weight on a dry weight basis,
the HPMCAS in an amount from about 35 to 55% by weight on a dry weight basis,
the pharmaceutically acceptable disintegrant in an amount from about 2.5 to 3.5% by weight on a dry weight basis, and
the pharmaceutically acceptable lubricant in an amount from about 0.2 to 1.5% by weight on a dry weight basis;
optionally wherein the oral dosage form is a compressed pharmaceutical tablet or a direct compressed pharmaceutical tablet, wherein the tablet is provided by direct compression or dry granulation of a physical blend or mixture of VS-6063, or a pharmaceutically acceptable salt thereof, admixed with the polymer excipient.

In another aspect, the invention provides an oral dosage form a pharmaceutical composition as described herein for use in treating a subject having been identified with a cancer disorder, for example mesothelioma, triple negative breast cancer, ovarian cancer, or lung cancer; optionally wherein the cancer disorder is surgical resectable malignant pleural mesothelioma, advanced ovarian cancer, non-small cell lung cancer (NSCLC), KRAS mutant NSCLC, or non-hematologic malignancies; optionally wherein:

a) the oral dosage form comprises 10 to 500 mg of VS-6063, or a pharmaceutically acceptable salt thereof; or
b) the oral dosage form is administered in combination with administration of an additional agent; optionally wherein the additional agent is a cancer therapeutic, optionally wherein the cancer therapeutic is taxane, optionally wherein the taxane is paclitaxel.

In another aspect, the invention provides a method for preparing a direct compressed tablet in unit dosage form, which comprises:

(a) blending as a % by weight on a dry weight basis:

(i) 5 to 50% by weight on a dry weight basis of VS-6063;
(ii) HPMCAS; and
(iii) at least one excipient selected from a filler, a disintegrant, and a lubricant;
to form a VS-6063 formulation in the form of a tableting powder, capable of being directly compressed into a tablets; and

(b) compressing the formulation prepared during step (a) to form the compressed VS-6063 tablet in unit dosage form.

Also disclosed herein is a composition, *e.g.*, pharmaceutical composition comprising VS-6063 *(e.g.,* VS-6063 free base), or a pharmaceutically acceptable salt thereof *(e.g.,* VS-6063 hydrochloride), *e.g.,* admixed with an excipient *(e.g.,* a binder, *e.g.,* a polymer, *e.g.,* a precipitation inhibitor, *e.g.,* Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF); a *e.g.,* polyol *(e.g.,* mannitol-starch *(e.g.,* Pearlitol Flash), mannitol, sorbitol)). In some embodiments, the excipient is a binder. In some embodiments, the excipient is a polymer. In some embodiments, the polymer is a precipitation inhibitor. In some embodiments, the precipitation inhibitor is a HPMCAS. In some embodiments, the precipitation inhibitor is HPMCAS-HF. In some embodiments, the excipient reduces precipitation *(e.g.,* of VS-6063 in solution, *e.g.,* in vivo) when administered orally. In some embodiments, the excipient enhances bioavailability (*e.g.*, improves absorption) of VS-6063 *(e.g.,* VS-6063 free base), or a pharmaceutically acceptable salt thereof *(e.g.,* VS-6063 hydrochloride), when administered orally. In some embodiments, the ratio (*e.g.*, relative amount) by weight of VS-6063 *(e.g.,* VS-6063 free base), or a pharmaceutically acceptable salt thereof *(e.g.,* VS-6063 hydrochloride), to the polymer is about 1 to 1, 1 to 2, 1 to 3, 1 to 4, 1 to 5, 1 to 6, 1 to 7, 1 to 8, or 1 to 9. In some embodiments, the polymer is a precipitation inhibitor, *e.g.*, Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF). In some embodiments, the VS-6063 *(e.g.,* VS-6063 free base), or a pharmaceutically acceptable salt thereof *(e.g.,* VS-6063 hydrochloride), is present in the composition in an amount that is about 10, 25, 30, 31, 32, 32.5, 33, 34, 35,

40, 45, 50% or more weight per weight (w/w) relative to an amount of polymer, *e.g.,* a precipitation inhibitor, provided. In some embodiments, the composition is admixed with 5 to 50%, 5 to 30%, 10 to 30%, 10 to 20%, 12 to 15%, or 13% weight of VS-6063, or a pharmaceutically acceptable salt thereof, per weight of the composition. In some embodiments, the polymer is present at 25 to 55%, 35 to 45%, 38 to 43%, or 40% weight of polymer per weight of the oral dosage form. In some embodiments, the polymer is a precipitation inhibitor, *e.g.*, Hydroxypropyl Methylcellulose (HPMC) or Hypromellose (*e.g.,* Hydroxypropyl Methylcellulose Acetate Succinate (HPMC-AS) or Hypromellose Acetate Succinate, *e.g.*, HPMCAS-H, HPMCAS-M, HPMCAS-HF). In some embodiments, the precipitation inhibitor is a HPMCAS. In some embodiments, the precipitation inhibitor is HPMCAS-HF.

In some embodiments, the composition is configured in an oral dosage form. In some embodiments, the oral dosage form comprises 5 to 50%, 5 to 30%, 10 to 30%, 10 to 20%, 12 to 15%, or 13% weight of VS-6063, or a pharmaceutically acceptable salt thereof, per weight of the oral dosage form. In some embodiments, the VS-6063 (*e.g.*, VS-6063 free base), or a pharmaceutically acceptable salt thereof (*e.g.*, VS-6063 hydrochloride), is present at 13% weight of VS-6063, or a pharmaceutically acceptable salt thereof, per weight of the oral dosage form.

In some embodiments, the oral dosage form is a tablet. In some embodiments, the tablet is provided by direct compression of a physical blend or mixture. In some embodiments, the physical blend or mixture is provided by a dry granulation process.

In some embodiments, the tablet has a tablet hardness of about 5 to 20 kP, 7 to 17 kP, 5 to 15 kP, or 10 to 15 kP. In some embodiments, the tablet has a tablet hardness of about 5, 6, 7, 8, 9, 10, 11, 12, 13 or more kP. In some embodiments, the tablet has a disintegration time of about 1 to 20 mins, 3 to 17 mins, 5 to 15 mins, or 9 to 15 mins. In some embodiments, the tablet has a disintegration time of about 1, 2, 3, 4, 5, 6, 7, 8, 9, 10, 11, 12, 13, 14, 15, 16, 17, 18, 19, 20 or more minutes.

In some embodiments, the pharmaceutical composition further comprises one or more fillers (*e.g.,* microcrystalline cellulose *(e.g.,* microcrystalline cellulose PH 102, *e.g.,* Avicel PH 102); lactose (*e.g.,* lactose monohydrate *(e.g.,* FastFlo 316)). In some embodiments, the filler is present at 5 to 80%, 10 to 70%, 20 to 60%, 30 to 60%, 30 to 50%, or 42% weight of filler per weight of the oral dosage form. In some embodiments, the filler is a mixture of two fillers. In some embodiments, one filler is a microcrystalline cellulose, microcrystalline cellulose PH 102 *(e.g.,* Avicel PH 102). In some embodiments, one filler is lactose monohydrate (*e.g*., FastFlo 316). In some embodiments, the filler is present at 10 to 30%, 15 to 25%, or 20% weight of filler per weight of the oral dosage form. In some embodiments, the fillers are present at a 1:1 w/w ratio to one another.

In some embodiments, the oral dosage form comprises a disintegrant *(e.g.,* a polymer, *e.g.,* a crosslinked polymer, *e.g.*, crosslinked polyvinylpyrrolidone or crospovidone, carboxymethyl cellulose or croscarmellose sodium; starch, *e.g.,* modified starch, *e.g.,* sodium starch glycolate). In some embodiments, the disintegrant is sodium starch glycolate. In some embodiments, the disintegrant is present at 0 to 5%, 1 to 5%, 2.5 to 5%, or 3% weight of disintegrant per weight of the oral dosage form.

In some embodiments, the oral dosage form comprises a lubricant *(e.g.,* talc, silica, fats, *e.g.,* magnesium stearate). In some embodiments, the lubricant is magnesium stearate. In some embodiments, the lubricant is present at 0.1 to 2% or 0.2 to 1.5% weight of lubricant per weight of the oral dosage form. In some embodiments, the lubricant is present at 1% w/w. In some embodiments, the lubricant is present at 0.5% w/w.

In an embodiment, an oral dosage form described herein has a greater $C_{max}$ value than a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose (*e.g.,* Hydroxypropyl Methylcellulose Acetate Succinate (HPMC-AS) or Hypromellose Acetate Succinate, *e.g.*, HPMCAS-H, HPMCAS-M, HPMCAS-HF), in an admixture. In an embodiment, the $C_{max}$ value of an oral dosage form described herein is at least 1.1 times (*e.g*., at least 1.25 times, at least 1.5 times, at least 2 times, at least 3 times, at least 5 times) greater than a reference dosage form of the same dosage amount (*e.g*., a dosage form having 100 mg of VS-6063 relative to a reference dosage form also having 100 mg of VS-6063). In an embodiment, the $C_{max}$ value of an oral dosage form described herein is at least 1.6 times greater, at least 1.7 times greater, at least 1.8 times greater, at least 1.9 times greater, at least 2 times greater, at least 3 times greater, or at least 4 times greater than a reference dosage form of the same dosage amount.

In an embodiment, an oral dosage form described herein has a reduced food effect relative to a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g*., HPMCAS-H, HPMCAS-M, HPMCAS-HF) in an admixture. In an embodiment, the difference between the $T_{max}$ value of the fed and fasted states for subjects administered an oral dosage form described herein is less that the difference between the $T_{max}$ value of the fed and fasted states for subjects administered a reference oral dosage form of the same dosage amount, *e.g.,* a dosage form that does not include an excipient (*e.g.,* a cellulosic polymer and derivatives thereof, *e.g.,* Hydroxypropyl Methylcellulose (HPMC) or Hypromellose (*e.g*., Hydroxypropyl Methylcellulose Acetate Succinate (HPMC-AS) or Hypromellose Acetate Succinate, *e.g*., HPMCAS-H, HPMCAS-M, HPMCAS-HF) in an admixture.

In an embodiment, an oral dosage form described herein has consistent exposure when administered to a subject. In an embodiment, an oral dosage form described herein, when administered to a subject, provides an exposure across multiple subjects of less than 50% of the variability associated with exposure compared with that of a reference oral dosage form of the same dosage amount. In an embodiment, an oral dosage form described herein, when administered to a subject, provides an exposure across multiple subjects of less than 40%, 30%, 20%, or 10% of the variability associated with exposure compared with that of a reference oral dosage form of the same dosage amount.

Also disclosed herein is an oral dosage form comprising: VS-6063 (*e.g.*, VS-6063 free base), or a pharmaceutically acceptable salt thereof (*e.g.*, VS-6063 hydrochloride) in an amount from about 5 to 50% by weight on a dry weight basis, a pharmaceutically acceptable excipient (*e.g.,* a binder, *e.g.,* a polymer, *e.g.,* a precipitation inhibitor, *e.g.,* Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF), in an amount from about 25 to 55% by weight on a dry weight basis, a pharmaceutically acceptable filler *(e.g.,* microcrystalline cellulose, *e.g.,* microcrystalline cellulose PH 102, *e.g.,* Avicel PH102) in an amount from about 10 to 30% by weight on a dry weight basis, a second pharmaceutically acceptable filler *(e.g.,* lactose, *e.g.,* lactose monohydrate, *e.g.,* FastFlo 316) in an amount from about 10 to 30% by weight on a dry weight basis, a pharmaceutically acceptable disintegrant *(e.g.,* starch, *e.g.,* modified starch, *e.g,* sodium starch glycolate) in an amount from about 0 to 5% by weight on a dry weight basis, and a pharmaceutically acceptable lubricant (*e.g*., magnesium stearate) in an amount from about 0.1 to 2% by weight on a dry weight basis. In some embodiments, disclosed herein is an oral dosage form comprising: VS-6063 (*e.g.*, VS-6063 free base), or a pharmaceutically acceptable salt thereof (*e.g.*, VS-6063 hydrochloride) in an amount from about 10 to 15% by weight on a dry weight basis, a pharmaceutically acceptable excipient (*e.g.,* a binder, *e.g.,* polymer, *e.g.,* a precipitation inhibitor, *e.g.,* Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF), in an amount from about 35 to 55% by weight on a dry weight basis, a pharmaceutically acceptable filler *(e.g.,* microcrystalline cellulose, *e.g.,* microcrystalline cellulose PH 102, *e.g.,* Avicel PH102) in an amount from about 15 to 25% by weight on a dry weight basis, a second pharmaceutically acceptable filler *(e.g.,* lactose, *e.g.,* lactose monohydrate, *e.g.*, FastFlo 316) in an amount from about 15 to 25% by weight on a dry weight basis, a pharmaceutically acceptable disintegrant *(e.g.,* starch, *e.g.,* modified starch, *e.g.,* sodium starch glycolate) in an amount from about 2.5 to 3.5% by weight on a dry weight basis, and a pharmaceutically acceptable lubricant (*e.g*., magnesium stearate) in an amount from about 0.2 to 1.5% by weight on a dry weight basis. In some embodiments, the oral dosage form is a compressed pharmaceutical tablet or a direct compressed pharmaceutical tablet.

In some embodiments, the excipient is one or more binders *(e.g.,* polyvinylpyrrolidone *(e.g.,* polyvinylpyrrolidone vinyl acetate copolymer (PVP/VA), Copovidone), other copolymers (*e.g.*, comprising polyethylene glycol, polyvinylcaprolactam, and polyvinylactate (*e.g.*, Soluplus))). In some embodiments, the binder is a polymer, *e.g.,* a precipitation inhibitor. In some embodiments, the binder comprises two binders *(e.g.,* polyvinylpyrrolidone *(e.g.,* polyvinylpyrrolidone vinyl acetate copolymer (PVP/VA), Copovidone), other copolymers (*e.g.*, comprising polyethylene glycol, polyvinylcaprolactam, and polyvinylactate *(e.g.,* Soluplus))). In some embodiments, the binder is a polymer, *e.g.,* a precipitation inhibitor. In some embodiments, the binder is present at about 37.5% weight of binder per weight of the oral dosage form. In some embodiments, the binder is present at about 25% weight of binder per weight of the oral dosage form. In some embodiments, the filler is a polyol *(e.g.,* mannitol-starch *(e.g.,* Pearlitol Flash), mannitol, sorbitol).

In some embodiments, the disintegrant is a polyvinylpolypyrrolidone (*e.g*., crospovidone). In some embodiments, the disintegrant is present at 0 to 30%, 5 to 25%, 5 to 20%, 5 to 15%, or 10% weight of disintegrant per weight of the oral dosage form.

In an embodiment, an oral dosage form described herein has a greater $C_{max}$ value than a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g.*, HPMCAS-H, HPMCAS-M, HPMCAS-HF), in an admixture. In an embodiment, the $C_{max}$ value of an oral dosage form described herein is at least 1.1 times (*e.g.*, at least 1.25 times, at least 1.5 times, at least 2 times, at least 3 times, at least 5 times) greater than a reference dosage form of the same dosage amount (*e.g.*, a dosage form having 100 mg of VS-6063 relative to a reference dosage form also having 100 mg of VS-6063). In an embodiment, the $C_{max}$ value of an oral dosage form described herein is at least 1.6 times greater, at least 1.7 times greater, at least 1.8 times greater, at least 1.9 times greater, at least 2 times greater, at least 3 times greater, or at least 4 times greater than a reference dosage form of the same dosage amount.

In an embodiment, an oral dosage form described herein has a reduced food effect relative to a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g.*, HPMCAS-H, HPMCAS-M, HPMCAS-HF) in an admixture. In an embodiment, the difference between the $T_{max}$ value of the fed and fasted states for subjects administered an oral dosage form described herein is less that the difference between the $T_{max}$ value of the fed and fasted states for subjects

administered a reference oral dosage form of the same dosage amount, *e.g.,* a dosage form that does not include an excipient (*e.g.,* a cellulosic polymer and derivatives thereof, *e.g.,* Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF) in an admixture.

In an embodiment, an oral dosage form described herein has consistent exposure when administered to a subject. In an embodiment, an oral dosage form described herein, when administered to a subject, provides an exposure across multiple subjects of less than 50% of the variability associated with exposure compared with that of a reference oral dosage form of the same dosage amount. In an embodiment, an oral dosage form described herein, when administered to a subject, provides an exposure across multiple subjects of less than 40%, 30%, 20%, or 10% of the variability associated with exposure compared with that of a reference oral dosage form of the same dosage amount.

Also disclosed herein is an oral dosage form comprising: VS-6063 (*e.g.*, VS-6063 free base), or a pharmaceutically acceptable salt thereof (*e.g.*, VS-6063 hydrochloride) in an amount from about 10 to 15% by weight on a dry weight basis, a pharmaceutically acceptable excipient *(e.g.,* a binder *(e.g.,* polyvinyl pyrrolidone (*e.g.*, polyvinyl pyrrolidone vinyl acetate copolymer (PVP/VA), Copovidone))) in an amount from about 15 to 25% by weight on a dry weight basis, a second pharmaceutically acceptable excipient *(e.g.,* a binder *(e.g.,* polyvinylactate *(e.g.,* Soluplus))) in an amount from about 15 to 25% by weight on a dry weight basis, a pharmaceutically acceptable filler *(e.g.,* polyol *(e.g.,* mannitol-starch *(e.g.,* Pearlitol Flash))) in an amount from about 30 to 40% by weight on a dry weight basis, a pharmaceutically acceptable disintegrant (*e.g.*, crospovidone) in an amount from about 5 to 15% by weight on a dry weight basis, and a pharmaceutically acceptable lubricant *(e.g.,* magnesium stearate) in an amount from about 0.2 to 1.5% by weight on a dry weight basis.

In some embodiments, the VS-6063 (*e.g.*, VS-6063 free base), or a pharmaceutically acceptable salt thereof *(e.g.,* VS-6063 hydrochloride), is present at 10% weight of VS-6063 *(e.g.,* VS-6063 free base), or a pharmaceutically acceptable salt thereof (*e.g.*, VS-6063 hydrochloride), per weight of the oral dosage form. In some embodiments, the VS-6063 (*e.g.*, VS-6063 free base), or a pharmaceutically acceptable salt thereof (*e.g.*, VS-6063 hydrochloride), is present at 25% weight of VS-6063, or a pharmaceutically acceptable salt thereof, per weight of the oral dosage form. In some embodiments, the VS-6063 *(e.g.,* VS-6063 free base), or a pharmaceutically acceptable salt thereof *(e.g.,* VS-6063 hydrochloride), is present at 50% weight of VS-6063 (*e.g.*, VS-6063 free base), or a pharmaceutically acceptable salt thereof (*e.g.*, VS-6063 hydrochloride), per weight of the oral dosage form.

In some embodiments, the physical blend or mixture is provided by a spray dried dispersion process. In some embodiments, the process provides a solid dispersion comprising at least 10% by weight (*e.g.*, 10, 20, 25, 40, 50% or more) of VS-6063 (*e.g.*, VS-6063 free base), or a pharmaceutically acceptable salt thereof (*e.g.*, VS-6063 hydrochloride). In some embodiments, the process provides a solid dispersion comprising 10% by weight of VS-6063 (*e.g.*, VS-6063 free base), or a pharmaceutically acceptable salt thereof (*e.g.*, VS-6063 hydrochloride). In some embodiments, the process provides a solid dispersion comprising 25% by weight of VS-6063 (*e.g.*, VS-6063 free base), or a pharmaceutically acceptable salt thereof (*e.g.*, VS-6063 hydrochloride). In some embodiments, the dispersion further comprises a polymer, *e.g.,* a polymer matrix *(e.g.,* Hydroxypropyl Methylcellulose Acetate Succinate (HPMACS) or Hypromellose Acetate Succinate, *e.g.*, HPMCAS-H, HPMCAS-M; Hydroxypropyl Methylcellulose Phthalate (HPMCP) or Hypromellose Phthalate, *e.g.*, HPMCP-HP55, HPMCP-HP55S, Methocol E3LV, PVP-VA, Soluplus, PVAP, polymethacrylic acid (*e.g.,* Eudragit, *e.g.,* Eudragit L100-55), Soluplus:PVP-VA mixture, Soluplus, HPMCAS (*e.g.*, HPMCAS-H) mixture). In some embodiments, the dispersion further comprises a polymer matrix *(e.g.,* polymethacrylic acid *(e.g.,* Eudragit, *e.g.,* Eudragit L100-55)).

Also disclosed herein is an oral dosage form comprising VS-6063 (*e.g.*, VS-6063 free base), or a pharmaceutically acceptable salt thereof (*e.g.*, VS-6063 hydrochloride), for use in treating a subject having been identified with a cancer disorder *(e.g.,* mesothelioma *(e.g.,* malignant pleural mesothelioma, *e.g.,* surgical resectable malignant pleural mesothelioma), breast cancer *(e.g.,* triple negative breast cancer), ovarian cancer *(e.g.,* advanced ovarian cancer), lung cancer *(e.g.,* non-small cell lung cancer (NSCLC), *e.g.*, KRAS mutant NSCLC)), or a non-hematolotic malignancy.

Also disclosed herein is a method of treating a disorder in a patient in need thereof, the method comprising administering an oral dosage form comprising 10 to 500 mg of VS-6063 (*e.g.*, VS-6063 free base), or a pharmaceutically acceptable salt thereof (*e.g.*, VS-6063 hydrochloride), to thereby treat the disorder. In some embodiments, the disorder is a cancer disorder *(e.g.,* mesothelioma *(e.g.,* surgical resectable malignant pleural mesothelioma), breast cancer *(e.g.,* triple negative breast cancer, ovarian cancer *(e.g.,* advanced ovarian cancer)), lung cancer *(e.g.,* non-small cell lung cancer (NSCLC), *e.g.,* KRAS mutant NSCLC)), or a non-hematolotic malignancy.

In some embodiments, the administration is performed in combination with administration of an additional agent (*e.g.,* an anti-cancer or anti-tumor agent, *e.g.,* taxane, *e.g.,* paclitaxel).

Also disclosed herein is a method for preparing a direct compressed tablet in unit dosage form, which comprises: (a) admixing (*e.g.*, blending) as a % by weight on a dry weight basis: (i) 5 to 50% by weight on a dry weight basis of VS-6063; (ii) a pharmaceutically acceptable excipient *(e.g.,* a binder, *e.g.,* polymer, *e.g.,* a precipitation inhibitor, *e.g.,* HPMC, *e.g.,* HPMC-AS, *e.g.,* HPMCAS-H, HPMCAS-M, HPMCAS-HF); and (iii) at least one pharmaceutically acceptable excipient selected from a pharmaceutically acceptable filler, a pharmaceutically acceptable disintegrant, and a pharmaceutically acceptable lubricant; to form a VS-6063 formulation in the form of a tabletting powder, capable of being directly compressed into tablets; and (b) compressing the formulation prepared during step (a) to form the compressed VS-6063 tablet in unit dosage form. In some embodiments, a pharmaceutically acceptable filler is present at 30 to 60% by weight on a dry weight basis. In some embodiments, a pharmaceutically acceptable distintegrant is present at 2.5 to 5% by weight on a dry weight basis. In some embodiments, a pharmaceutically acceptable lubricant is present at 0.5 to 2% by weight on a dry weight basis.

Also disclosed herein is a method of treating a subject having mesothelioma, *e.g.*, malignant pleural mesothelioma, *e.g.,* surgical resectable malignant pleural mesothelioma (*e.g.,* non-metastatic, unresectable), comprising: providing an effective amount of VS-6063, or a pharmaceutically acceptable salt thereof, or composition (*e.g.,* a composition as described herein, *e.g.,* a composition comprising VS-6063, or a pharmaceutically acceptable salt thereof); to said subject, thereby treating said subject. In some embodiments, the method comprises providing an effective course of VS-6063, or a pharmaceutically acceptable salt thereof, or composition (*e.g.*, a composition as described herein, *e.g.,* a composition comprising VS-6063, or a pharmaceutically acceptable salt thereof); to said subject.

In some embodiments, said subject carries a mutation in a tumor suppressor gene. In some embodiments, said subject carries a mutation in Neurofibromin 2 (NF2). NF2 is a tumor suppressor gene and encodes the protein Merlin, which can function in restricting cellular proliferation and promoting apoptosis. Merlin regulates FAK, at least in part through the attenuation of FAK phosphorylation, which disrupts the ability of FAK to interact with certain binding partners; resulting in the inhibition of FAK mediated signaling pathways. Thus mutations in the NF2 gene which result in decreased expression and/or activity of Merlin, or decreased Merlin expression and/or activity in the absence of NF2 mutations, may lead to increased sensitivity to FAK inhibitors. Therefore cancer patients with one or more alterations in the NF2 gene or an alteration in the level of Merlin expression and/or activity, *e.g.*, decreased level of Merlin expression; may have a cancer characterized by increased sensitivity to FAK inhibitors, and thus may benefit from treatment with a compound as described herein *(e.g.,* VS-6063, *e.g.,* VS-6063 free base, or a pharmaceutically acceptable salt thereof (*e.g.*, VS-6063 hydrochloride salt)). In some embodiments, said subject has aberrant levels of NF2 and or Merlin, *e.g.*, relative to a reference standard, *e.g.*, a subject without a mutation in a tumor suppressor gene, a subject without a mutation in NF2, or a subject without cancer. In some embodiments, said subject has aberrant levels (*e.g.*, lower levels or dysfunctional levels) of Merlin protein. In some embodiments, the method comprises acquiring, *e.g.,* directly acquiring or indirectly acquiring, information on the NF2 and or Merlin (*i.e.,* Merlin protein) status of the subject. In some embodiments, the NF2 and or Merlin status is determined by immunohistochemistry. In some embodiments, the NF2 and or Merlin status is determined by DNA sequence analysis. In some embodiments, the method comprises testing said subject for NF2 and or Merlin status of the subject. In some embodiments, the method comprises, responsive to NF2 and or Merlin status, *e.g.*, a mutant NF2, administering VS-6063, or a pharmaceutically acceptable salt thereof, or composition *(e.g.,* a composition as described herein, *e.g.,* a composition comprising VS-6063, or a pharmaceutically acceptable salt thereof); to said subject.

In some embodiments, the level of Merlin expression is low, decreased, or absent. In some embodiments, the level of Merlin expression is compared to a reference standard. In some embodiments, the level of Merlin expression is low, decreased, or absent compared to the reference standard. In some embodiments, the level of Merlin expression is evaluated by the level of Merlin RNA expression. In some embodiments, the level of Merlin expression is measured by the evaluation of the level of RNA that encodes the Merlin. In some embodiments, the level of Merlin expression is evaluated by the level of Merlin protein expression. In some embodiments, the level of Merlin protein expression is assayed by immunohistochemistry or western blot. Immunohistochemistry or IHC refers to the process of localizing antigens (e.g. proteins) in cells of a tissue section exploiting the principle of antibodies binding specifically to antigens in biological tissues. In a preferred embodiment, the level of Merlin protein expression is assayed by immunohistochemistry.

In some embodiments, the patient has one or more alterations in the NF2 gene.

In some embodiments, the alterations in the NF2 gene include mutations, chromosomal deletions, or alterations to the NF2 gene or NF2 promoter. In some embodiments, the alterations to the NF2 promoter include changes in methylation, *e.g.*, hypermethylation. In some embodiments, the evaluation includes acquiring information regarding the NF2 genotype of the patient. In some embodiments, the NF2 genotype of the patient is compared to a reference standard.

In some embodiments, the alterations in the NF2 gene include mutations, chromosomal deletions, or alterations to the NF2 gene. The NF2 gene includes any regulatory sequences, *e.g.*, promoter, and enhancer sequences. In some embodiments, the alterations in the NF2 gene include mutations, chromosomal deletions, or alterations to the NF2 gene. The NF2 gene includes any regulatory sequences, *e.g.*, promoter, and enhancer sequences. In certain embodiments the alteration in the NF2 gene is one or more mutations. In certain embodiments the alteration is a single nucleotide polymorphism. In certain embodiments the alteration is a point mutation. In certain embodiments the alteration is an inactivating mutation. In certain embodiments the alteration in the NF2 gene is a chromosomal deletion. In certain embodiments the alteration is an inactivating chromosomal deletion. In certain embodiments the alteration in the NF2 gene is hypermethylation of the promoter region of the gene.

Cancer patients with one or more alterations in the NF2 gene or an alteration in the level of Merlin expression and/or activity, *e.g*., decreased level of Merlin expression; may have a cancer characterized by sensitivity or responsiveness to FAK inhibtiors, and thus may benefit from treatment with a FAK inhibitor.

In some embodiments, the method comprises co-administering a course of a second therapy, e.g. a second anti-tumor or anti-cancer therapy, *e.g.,* one or more anti-tumor or anti-cancer agents. In some embodiments, said course of second therapy is provided after a diagnosis of mesothelioma. In some embodiments, the course of second therapy ends prior to initiation of provision of the course of VS-6063, or a pharmaceutically acceptable salt thereof, or composition (*e.g*., a composition as described herein, *e.g.,* a composition comprising VS-6063, or a pharmaceutically acceptable salt thereof). In some embodiments, the method comprises providing a first course of said second therapy; administering VS-6063, or a pharmaceutically acceptable salt thereof, or composition (*e.g*., a composition as described herein, *e.g.,* a composition comprising VS-6063, or a pharmaceutically acceptable salt thereof); and providing a second course of said second therapy. In some embodiments, the first course is initiated prior to the administration of VS-6063, or a pharmaceutically acceptable salt thereof, or composition (*e.g*., a composition as described herein, *e.g*., a composition comprising VS-6063, or a pharmaceutically acceptable salt thereof). In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second thereaputic agent in the subject, *e.g*., in the blood of a subject, is reduced in the second course as compared with the first course. In some embodiments, said second agent is not administered to said subject after the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during the course of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition *(e.g.,* a composition as described herein, *e.g.,* a composition comprising VS-6063, or a pharmaceutically acceptable salt thereof).

In some embodiments, said subject has previously received a course of a second therapy, *e.g.,* a second anti-cancer or anti-tumor therapy, *e.g.,* one or more anti-cancer or anti-tumor agents. In some embodiments, said course of second therapy is administered after a diagnosis of mesothelioma. In some embodiments, said course of second therapy ends prior to, at the same time as, or after, the initial administration of VS-6063, or a pharmaceutically acceptable salt thereof, or composition (*e.g*., a composition as described herein, *e.g*., a composition comprising VS-6063, or a pharmaceutically acceptable salt thereof). In some embodiments, said second therapy is not administered after the first administration of VS-6063, or a pharmaceutically acceptable salt thereof, or composition (*e.g*., a composition as described herein, *e.g*., a composition comprising VS-6063, or a pharmaceutically acceptable salt thereof). In some embodiments, the method comprises providing a second course of said second therapy. In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the second course is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second therapeutic agent in the subject, *e.g*., in the blood of a subject, is reduced in the second course as compared with the first course. In some embodiments, said second agent is not administered to said subject after the initial administration of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, said second agent is not administered to said subject during the course of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition *(e.g.,* a composition as described herein, *e.g.,* a composition comprising VS-6063, or a pharmaceutically acceptable salt thereof).

In some embodiments, said second therapy comprises the administration of a therapeutic agent, *e.g.,* an anti-tumor or anti-cancer agent. In some embodiments, said second therapy comprises the administration of an additional agent, *e.g.,* an anti-cancer or anti-tumor agent, *e.g.,* an anti-cancer or antitumor agent selected from the group comprising mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens. In some embodiments, said therapeutic agent comprises a platin drug, *e.g*., cisplatin or carboplatin. In some

embodiments, said therapeutic agent comprises a drug other than a platin drug, *e.g.,* it comprises a taxane, *e.g.,* docetaxel, paclitaxel; a plant derived anti-tumor substance, *e.g.,* an alkaloid, *e.g.,* vinorelbine; an anti-metabolite, *e.g.,* gemcitabine; or a cytotoxic topoisomerase inhibiting agent, *e.g.,* irinotecan. In some embodiments, said second therapy comprises the administration of a first and a second therapeutic agent. In some embodiments, said the first and the second therapeutic agent is each selected from a group of additional therapeutic agents, *e.g.,* an anti-tumor or anti-cancer agent, *e.g.,* an anti-tumor or anti-cancer agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens. In some embodiments, said therapeutic agent comprises a platin drug, *e.g.*, cisplatin or carboplatin. In some embodiments, said therapeutic agent comprises a drug other than a platin drug, *e.g.,* it comprises a taxane, *e.g.,* docetaxel, paclitaxel; a plant derived anti-cancer or anti-tumor substance, *e.g.,* an alkaloid, *e.g.,* vinorelbine; an anti-metabolite, *e.g.,* gemcitabine; or a cytotoxic topoisomerase inhibiting agent, *e.g.,* irinotecan.

Also disclosed herein is a method of treating a subject having cancer, *e.g.,* lung cancer, *e.g.,* non-small cell lung cancer (NSCLC), *e.g.*, KRAS mutant NSCLC, comprising: providing an effective amount of VS-6063, or a pharmaceutically acceptable salt thereof, or composition (*e.g.*, a composition as described herein, *e.g.,* a composition comprising VS-6063, or a pharmaceutically acceptable salt thereof); to said subject, thereby treating said subject. In some embodiments, the method comprises providing an effective course of VS-6063, or a pharmaceutically acceptable salt thereof, or composition (*e.g.*, a composition as described herein, *e.g.*, a composition comprising VS-6063, or a pharmaceutically acceptable salt thereof); to said subject. In some embodiments, said cancer is metastatic. In some embodiments, the subject has a locally recurrent disease.

Also disclosed herein is a method of evaluating a subject having cancer, *e.g.,* lung cancer, *e.g.,* non-small cell lung cancer (NSCLC), for treatment with VS-6063, or a pharmaceutically acceptable salt thereof, or composition (*e.g.,* a composition as described herein, *e.g.,* a composition comprising VS-6063, or a pharmaceutically acceptable salt thereof); comprising: acquiring, *e.g.*, directly or indirectly acquiring, the mutation status of a gene in said NSCLC, thereby evaluating said subject. In some embodiments, the gene is selected from the group comprising KRAS, INK4a/Arf, and p53. In some embodiments, the method comprises acquiring the mutation status of KRAS in said NSCLC. In some embodiments, the method comprises acquiring the mutation status of KRAS and INK4a/Arf in said NSCLC. In some embodiments, the method comprises acquiring the mutation status of KRAS and p53 in said NSCLC. In some embodiments, the method comprises acquiring the mutation status of KRAS, INK4a/Arf, and p53 in said NSCLC. In some embodiments, the method comprises, responsive to said mutation status, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, responsive to a determination that the NSCLC comprises a KRAS mutant, selecting said subject for treatment with VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the method comprises, responsive to a determination that the NSCLC comprises a KRAS mutant and a INK4a/Arf mutant, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, responsive to a determination that the NSCLC comprises a KRAS mutant and a p53 mutant, selecting said subject for treatment with VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the method comprises, responsive to a determination that the NSCLC comprises a KRAS mutant, a INK4a/Arf mutant, and a p53 mutant, selecting said subject for treatment with VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the method comprises testing the subject for a mutation in a gene in said NSCLC. In some embodiments, the method comprises testing the subject for a mutation in a gene from the group comprising KRAS, INK4a/Arf, and p53 in said NSCLC. In some embodiments, the method comprises testing the subject for a mutation of KRAS in said NSCLC. In some embodiments, the method comprises testing the subject for a mutation of KRAS and INK4a/Arf in said NSCLC. In some embodiments, the method comprises testing the subject for a mutation of KRAS and p53 in said NSCLC. In some embodiments, the method comprises testing the subject for a mutation of KRAS, INK4a/Arf, and p53 in said NSCLC. In some embodiments, a cell of said subject *(e.g.,* a subject having NSCLC) comprises a KRAS mutant; KRAS mutant and INK4a/Arf mutant; or KRAS mutant, INK4a/Arf mutant, and p53 mutant. In some embodiments, the said cancer is metastatic. In some embodiments, the subject has locally recurrent disease.

In some embodiments, the method comprises acquiring mutation status of a gene in said subject's NSCLC. In some embodiments, the gene is selected from the group comprising KRAS, INK4a/Arf, and p53. In some embodiments, the method comprises acquiring the mutation status of KRAS in said subject's NSCLC. In some embodiments, the method comprises acquiring the mutation status of KRAS and INK4a/Arf in said subject's NSCLC. In some embodiments,

the method comprises acquiring the mutation status of subject's KRAS and p53 in said subject's NSCLC. In some embodiments, the method comprises acquiring the mutation status of KRAS, INK4a/Arf, and p53 in said NSCLC. In some embodiments, the method comprises, responsive to said mutation status, selecting said subject for treatment with VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the method comprises, responsive to a determination that the NSCLC comprises a KRAS mutant, selecting said subject for treatment with VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the method comprises, responsive to a determination that the NSCLC comprises a KRAS mutant and a INK4a/Arf mutant, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, responsive to a determination that the NSCLC comprises a KRAS mutant and a p53 mutant, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, responsive to a determination that the NSCLC comprises a KRAS mutant, a INK4a/Arf mutant, and a p53 mutant, selecting said subject for treatment with VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the method comprises testing the subject for a mutation in a gene in said NSCLC. In some embodiments, the method comprises testing the subject for a mutation in a gene from the group comprising KRAS, INK4a/Arf, and p53 in said NSCLC. In some embodiments, the method comprises directly testing the subject for a mutation of KRAS in said NSCLC. In some embodiments, the method comprises testing the subject for a mutation of KRAS and INK4a/Arf in said NSCLC. In some embodiments, the method comprises testing the subject for a mutation of KRAS and p53 in said NSCLC. In some embodiments, the method comprises testing the subject for a mutation of KRAS, INK4a/Arf, and p53 in said NSCLC. In some embodiments, a cell in the subject *(e.g.,* the subject with lung cancer, *e.g.,* NSCLC) comprises a KRAS mutant; KRAS mutant and INK4a/Arf mutant; or KRAS mutant, INK4a/Arf mutant, and p53 mutant.

In some embodiments, the method comprises co-administering a course of a second therapy, e.g. a second anti-tumor or anti-cancer therapy, *e.g.,* one or more anti-tumor or anti-cancer agents. In some embodiments, said course of second therapy is provided after a diagnosis of NSCLC. In some embodiments, the course of second therapy ends prior to initiation of provision of the course of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the method comprises providing a first course of said second therapy; administering VS-6063, or a pharmaceutically acceptable salt thereof, or composition (*e.g.*, a composition as described herein, *e.g.,* a composition comprising VS-6063, or a pharmaceutically acceptable salt thereof); and providing a second course of said second therapy. In some embodiments, the first course is initiated prior to the administration of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second thereaputic agent in the subject, *e.g.*, in the blood of a subject, is reduced in the second course as compared with the first course. In some embodiments, said second agent is not administered to said subject after the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during the course of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition *(e.g.,* a composition as described herein, *e.g.,* a composition comprising VS-6063, or a pharmaceutically acceptable salt thereof).

In some embodiments, said subject has previously received a course of a second therapy, *e.g.,* a second anti-tumor or anti-cancer therapy, *e.g.,* one or more anti-tumor or anti-cancer agents. In some embodiments, said course of second therapy was administered after a diagnosis of NSCLC. In some embodiments, said course of second therapy ends prior to, at the same time as, or after, the initial administration of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, said second therapy is not administered after the first administration of VS-6063, or a pharmaceutically acceptable salt thereof, or composition (*e.g.*, a composition as described herein, *e.g.,* a composition comprising VS-6063, or a pharmaceutically acceptable salt thereof). In some embodiments,

the method comprises providing a second course of said second therapy. In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the second course is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second therapeutic agent in the subject, *e.g.*, in the blood of a subject, is reduced in the second course as compared with the first course. In some embodiments, said second agent is not administered to said subject after the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during the course of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition *(e.g.,* a composition as described herein, *e.g.,* a composition comprising VS-6063, or a pharmaceutically acceptable salt thereof).

In some embodiments, said second therapy comprises the administration of a therapeutic agent, *e.g.,* an anti-tumor or anti-cancer agent. In some embodiments, said second therapy comprises the administration of and additional agent, *e.g.,* an anti-tumor or anti-cancer agent, *e.g.,* an anti-tumor or anticancer agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens. In some embodiments, said therapeutic agent comprises a platin drug, *e.g.*, cisplatin or carboplatin. In some embodiments, said therapeutic agent comprises a drug other than a platin drug, *e.g.,* it comprises a taxane, *e.g.,* docetaxel, paclitaxel; a plant derived anti-tumor substance, *e.g.,* an alkaloid, *e.g.,* vinorelbine; an anti-metabolite, *e.g.,* gemcitabine; or a cytotoxic topoisomerase inhibiting agent, *e.g.,* irinotecan. In some embodiments, said second therapy comprises the administration of a first and a second therapeutic agent. In some embodiments, said the first and the second therapeutic agent is each selected from a group of additional therapeutic agents, *e.g.,* an anti-tumor or anti-cancer agent, *e.g.,* an anti-tumor or anti-cancer agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens. In some embodiments, said therapeutic agent comprises a platin drug, *e.g.*, cisplatin or carboplatin. In some embodiments, said therapeutic agent comprises a drug other than a platin drug, *e.g.,* it comprises a taxane, *e.g.,* docetaxel, paclitaxel; a plant derived anti-tumor or anti-cancer substance, *e.g.,* an alkaloid, *e.g.,* vinorelbine; an anti-metabolite, *e.g.,* gemcitabine; or a cytotoxic topoisomerase inhibiting agent, *e.g.,* irinotecan.

In some embodiments, VS-6063, or a pharmaceutically acceptable salt thereof, or composition *(e.g.,* a composition as described herein, *e.g.,* a composition comprising VS-6063, or a pharmaceutically acceptable salt thereof); is administered for a cycle of at least 5, 10, 15, 20, 25, 30 or 35 days. In some embodiments, VS-6063, or a pharmaceutically acceptable salt thereof, or composition (*e.g.*, a composition as described herein, *e.g.,* a composition comprising VS-6063, or a pharmaceutically acceptable salt thereof); is administered for a cycle of 5 to 40, 10 to 40, 10 to 30, or 10 to 25 days. In some embodiments, VS-6063, or a pharmaceutically acceptable salt thereof, or composition (*e.g.*, a composition as described herein, *e.g.,* a composition comprising VS-6063, or a pharmaceutically acceptable salt thereof); is administered for a cycle of 15 to 40, 15 to 30, or 15 to 25 days. In some embodiments, VS-6063, or a pharmaceutically acceptable salt thereof, or composition (*e.g.*, a composition as described herein, *e.g.,* a composition comprising VS-6063, or a pharmaceutically acceptable salt thereof); is administered for a cycle of 21 +/-5, 21 +/-4, 21 +/-3, 21 +/-2, or 21 +/-1 days. In some embodiments, VS-6063, or a pharmaceutically acceptable salt thereof, or composition (*e.g.*, a composition as described herein, *e.g.,* a composition comprising VS-6063, or a pharmaceutically acceptable salt thereof); is administered for a cycle of 21 days. In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered 1, 2, 3, or 4 times per day. In some embodiments, VS-6063, or a pharmaceutically acceptable salt thereof, or composition (*e.g.*, a composition as described herein, *e.g.,* a composition comprising VS-6063, or a pharmaceutically acceptable salt thereof); is administered 2 times per day. In some embodiments, said VS-6063, or a pharmaceutically acceptable salt thereof, or composition *(e.g.,* a composition as described herein, *e.g.,* a composition comprising VS-6063, or a pharmaceutically acceptable salt thereof) is administered orally. In some embodiments, 50 to 800, 50 to 600, 50 to 500, 50 to 400, 50 to 300, or 50 to 200 mg/day of VS-6063, or a pharmaceutically acceptable salt thereof, or composition (*e.g.*, a composition as described herein, *e.g.,* a composition comprising VS-6063, or a pharmaceutically acceptable salt thereof); is administered. In some embodiments, 100 to 800, 100 to 600, 100 to 500, 100 to 400, 100 to 300, or 100 to 200 mg/day of VS-6063, or a pharmaceutically acceptable salt thereof, or

composition (*e.g.*, a composition as described herein, *e.g.,* a composition comprising VS-6063, or a pharmaceutically acceptable salt thereof); is administered. In some embodiments, 200 to 800, 200 to 600, 200 to 500, 200 to 400, or 200 to 300 mg/day of VS-6063, or a pharmaceutically acceptable salt thereof, or composition (*e.g.*, a composition as described herein, *e.g.,* a composition comprising VS-6063, or a pharmaceutically acceptable salt thereof); is administered. In some embodiments, 250 to 800, 250 to 600, 250 to 500, 250 to 400, or 250 to 300 mg/day of VS-6063, or a pharmaceutically acceptable salt thereof, or composition (*e.g.*, a composition as described herein, *e.g.,* a composition comprising VS-6063, or a pharmaceutically acceptable salt thereof); is administered. In some embodiments, the range does not include one or both endpoints, *e.g.*, wherein the range does not include the lower endpoint. In some embodiments, 100 +/- 50, 150 +/- 50, 200 +/- 50, 250 +/- 50, 300 +/- 50, 400 +/- 50, 500 +/- 50, 550 +/- 50 or 600 +/- 50 mg/day of VS-6063, or a pharmaceutically acceptable salt thereof, or composition (*e.g.*, a composition as described herein, *e.g.,* a composition comprising VS-6063, or a pharmaceutically acceptable salt thereof); is administered. In some embodiments, 100 mg/day of VS-6063, or a pharmaceutically acceptable salt thereof, or composition *(e.g.,* a composition as described herein, *e.g.,* a composition comprising VS-6063, or a pharmaceutically acceptable salt thereof); is administered. In some embodiments, 200 mg/day of VS-6063, or a pharmaceutically acceptable salt thereof, or composition (*e.g.*, a composition as described herein, *e.g.,* a composition comprising VS-6063, or a pharmaceutically acceptable salt thereof); is administered. In some embodiments, 400 mg/day of VS-6063, or a pharmaceutically acceptable salt thereof, or composition *(e.g.,* a composition as described herein, *e.g.,* a composition comprising VS-6063, or a pharmaceutically acceptable salt thereof); is administered.

In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered in multiple cycles (*e.g.*, more than one cycle). In some embodiments, the amount of VS-6063, or a pharmaceutically acceptable salt thereof, or composition (*e.g.*, a composition as described herein, *e.g.,* a composition comprising VS-6063, or a pharmaceutically acceptable salt thereof) is administered in each cycle is constant (*e.g.*, the amount of VS-6063, or a pharmaceutically acceptable salt thereof, or composition (*e.g.*, a composition as described herein, *e.g.,* a composition comprising VS-6063, or a pharmaceutically acceptable salt thereof) administered is the same). In some embodiments, the amount of VS-6063, or a pharmaceutically acceptable salt thereof, or composition *(e.g.,* a composition as described herein, *e.g.,* a composition comprising VS-6063, or a pharmaceutically acceptable salt thereof) is administered in a cycle is different than the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in another cycle (*e.g.,* a second cycle). In some embodiments, the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in a cycle is lower than the amount of VS-6063, or a pharmaceutically acceptable salt thereof, or composition *(e.g.,* a composition as described herein, *e.g.,* a composition comprising VS-6063, or a pharmaceutically acceptable salt thereof) that is administered in a subsequent cycle *(e.g.,* a second cycle). In some embodiments, the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in a cycle is greater than the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in a subsequent cycle *(e.g.,* a second cycle).

In some embodiments, a unit dosage formulation of 50 +/-25, 100 +/-50, 150 +/-50, 200 +/-50, 250+/-50, 300+/-50, 400+/-50, 500+/-50, 550+/-50 or 600+/-50 mg of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, 100+/-50, 100+/-25, 100+/-10, or 100+/-5 mg/day of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, a unit dosage formulation of 100+/-20, 100+/-10 or 100+/-5 mg of VS-6063, or a pharmaceutically acceptable salt thereof, or composition (*e.g.*, a composition as described herein, *e.g.,* a composition comprising VS-6063, or a pharmaceutically acceptable salt thereof) is administered. In some embodiments, a unit dosage formulation of 100 mg of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, 200+/-50, 200+/- 25, 200+/-10, or 200+/-5 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, a unit dosage formulation of 200+/-20, 200+/-10 or 200+/-5 mg of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, a unit dosage formulation of 200 mg of VS-6063, or a pharmaceutically acceptable salt thereof, or composition (*e.g.*, a composition as described herein, *e.g.*, a composition comprising VS-6063, or a pharmaceutically acceptable salt thereof) is administered. In some embodiments, 400+/-50, 400+/-25, 400+/-10, or 400+/- 5 mg/day of VS-6063, or a pharmaceutically acceptable salt thereof, or composition (*e.g.*, a composition as described herein, *e.g.,* a composition comprising VS-6063, or a pharmaceutically acceptable salt thereof) is administered. In some embodiments, a unit dosage formulation of 400+/-20, 400+/-10 or 400+/-5 mg of VS-6063, or a pharmaceutically acceptable salt thereof, or composition (*e.g.*, a composition as described herein, *e.g.*, a composition comprising VS-6063, or a pharmaceutically acceptable salt thereof) is administered. In some embodiments, a unit dosage formulation of 400 mg of VS-6063, or a pharmaceutically acceptable salt thereof, or composition (*e.g.*, a composition as described herein, *e.g.,* a composition comprising VS-6063, or a pharmaceutically acceptable salt thereof) is administered.

In an embodiment, an oral dosage form described herein has a greater $C_{max}$ value than a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g.*, HPMCAS-H, HPMCAS-M, HPMCAS-HF), in an admixture. In an embodiment, the $C_{max}$ value of an oral dosage form described herein is at least 1.1 times (*e.g.*, at least 1.25 times, at least 1.5 times, at least 2 times, at least 3 times, at least 5 times) greater than a reference dosage form of the same dosage amount (*e.g.*, a dosage form having 100 mg of VS-6063 relative to a reference dosage form also having 100 mg of VS-6063). In an embodiment, the $C_{max}$ value of an oral dosage form described herein is at least 1.6 times greater, at least 1.7 times greater, at least 1.8 times greater, at least 1.9 times greater, at least 2 times greater, at least 3 times greater, or at least 4 times greater than a reference dosage form of the same dosage amount.

In an embodiment, an oral dosage form described herein has a reduced food effect relative to a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose (*e.g.,* Hydroxypropyl Methylcellulose Acetate Succinate (HPMC-AS) or Hypromellose Acetate Succinate, *e.g.*, HPMCAS-H, HPMCAS-M, HPMCAS-HF) in an admixture. In an embodiment, the difference between the $T_{max}$ value of the fed and fasted states for subjects administered an oral dosage form described herein is less that the difference between the $T_{max}$ value of the fed and fasted states for subjects administered a reference oral dosage form of the same dosage amount, *e.g.,* a dosage form that does not include an excipient (*e.g.,* a cellulosic polymer and derivatives thereof, *e.g.*, Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF) in an admixture.

In an embodiment, an oral dosage form described herein has consistent exposure when administered to a subject. In an embodiment, an oral dosage form described herein, when administered to a subject, provides an exposure across multiple subjects of less than 50% of the variability associated with exposure compared with that of a reference oral dosage form of the same dosage amount. In an embodiment, an oral dosage form described herein, when administered to a subject, provides an exposure across multiple subjects of less than 40%, 30%, 20%, or 10% of the variability associated with exposure compared with that of a reference oral dosage form of the same dosage amount.

Also disclosed herein is a unit dosage form having 50 to 800 mg of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the unit dosage form has 50+/-25 mg of VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the unit dosage form has 50+/-10 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 50 mg of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, disclosed herein is a unit dosage form having 100 to 800 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 100+/-50 mg of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the unit dosage form has 100 +/-10 mg of VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the unit dosage form has 100 mg of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the unit dosage form has 200 +/-50 mg of VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the unit dosage form has 200 +/-10 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 200 mg of VS-6063, or a pharmaceutically acceptable salt thereof, or composition *(e.g.,* a composition

as described herein, *e.g.,* a composition comprising VS-6063, or a pharmaceutically acceptable salt thereof). In some embodiments, the unit dosage form has 400 +/-50 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 400 +/-10 mg of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the unit dosage form has 400 mg of VS-6063, or a pharmaceutically acceptable salt thereof, or composition (*e.g.*, a composition as described herein, *e.g.,* a composition comprising VS-6063, or a pharmaceutically acceptable salt thereof).

Also disclosed herein is a method of treating a subject having ovarian cancer, *e.g.*, advanced or metastatic ovarian cancer, comprising: providing an effective amount of VS-6063, or a pharmaceutically acceptable salt thereof, or composition (*e.g.*, a composition as described herein, *e.g.,* a composition comprising VS-6063, or a pharmaceutically acceptable salt thereof) to said subject, and optionally, providing an effective amount of a second therapeutic agent, *e.g.,* an anti-cancer or anti-tumor agent, *e.g.,* mitotic inhibitor, *e.g.,* a taxane, *e.g.,* paclitaxel, to said subject; said amounts, alone or in combination, being effective to treat said subject, thereby treating said subject, wherein, said subject receives more than 100 or 250 mg/day of VS-6063, or a pharmaceutically acceptable salt thereof, or composition *(e.g.,* a composition as described herein, *e.g.,* a composition comprising VS-6063, or a pharmaceutically acceptable salt thereof) for at least one day, *e.g.,* for an entire cycle or course of VS-6063, or a pharmaceutically acceptable salt thereof, or composition (*e.g.*, a composition as described herein, *e.g.,* a composition comprising VS-6063, or a pharmaceutically acceptable salt thereof); said subject receives VS-6063, or a pharmaceutically acceptable salt thereof, or composition (*e.g.*, a composition as described herein, *e.g.,* a composition comprising VS-6063, or a pharmaceutically acceptable salt thereof) for at least 5, 10, or 15 days; said second therapeutic agent is provided, *e.g.,* on a different course regimen, *e.g.*, not always on the same day as said VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, providing a course of VS-6063, or a pharmaceutically acceptable salt thereof, or composition *(e.g.,* a composition as described herein, *e.g.,* a composition comprising VS-6063, or a pharmaceutically acceptable salt thereof) to said subject, and optionally, providing a course of a second therapeutic agent, *e.g.,* an anti-cancer or anti-tumor agent, *e.g.,* mitotic inhibitor, *e.g.,* a taxane, *e.g.,* paclitaxel, to said subject, said courses, alone or in combination, being effective to treat said subject.

Also disclosed herein is a method of evaluating a subject having ovarian cancer for treatment with VS-6063, or a pharmaceutically acceptable salt thereof, or composition (*e.g.*, a composition as described herein, *e.g.,* a composition comprising VS-6063, or a pharmaceutically acceptable salt thereof), comprising: acquiring, e.g. directly acquiring or indirectly acquiring, the mutation status of a gene in said ovarian cancer, thereby evaluating said subject. In some embodiments, the method comprises, responsive to said mutation status, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises, testing the subject for a mutation in a gene in said ovarian cancer.

In some embodiments, the method comprises acquiring mutation status of a gene in said subject's ovarian cancer. In some embodiments, the method comprises, responsive to said mutation status, selecting said subject for treatment with VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the method comprises testing the subject for a mutation in a gene in said subject's ovarian cancer. In some embodiments, said cancer is metastatic. In some embodiments, said cancer is locally recurrent. In some embodiments, said cancer is advanced.

In some embodiments, the method comprising co-administering a course of a second therapy, e.g. a second anti-cancer or anti-tumor therapy, *e.g.*, one or more anti-cancer or anti-tumor agents. In some embodiments, a course of VS-6063, or a pharmaceutically acceptable salt thereof, or composition (*e.g.*, a composition as described herein, *e.g.*, a composition comprising VS-6063, or a pharmaceutically acceptable salt thereof) and a course of a second therapy, *e.g.,* the administration of an anti-cancer or anti-tumor agent, *e.g.,* mitotic inhibitor, *e.g.,* a taxane, *e.g.,* paclitaxel, are co-administered. In some embodiments, a course of VS-6063, or a pharmaceutically acceptable salt thereof, or composition (*e.g.*, a composition as described herein, *e.g.*, a composition comprising VS-6063, or a pharmaceutically acceptable salt thereof) and a course of a second therapy, *e.g.,* the administration of an anti-cancer or anti-tumor agent, *e.g.,* mitotic inhibitor, *e.g.,* a taxane, *e.g.,* paclitaxel, overlap. In some embodiments, said second therapy comprises the administration of paclitaxel. In some embodiments, said paclitaxel is administered at least twice in a course, *e.g.*, every 7+/-3, 7+/-2, 7+/-1 or 7 days. In some embodiments, said paclitaxel is administered at 80+/-50, 80+/-40, 180+/-30, 80+/-20, 80+/-10, or 80 $mg/m^2$. In some embodiments, said course of second therapy is provided after a diagnosis of ovarian cancer. In some embodiments, the course of second therapy ends prior to initiation of providing VS-6063, or a pharmaceutically acceptable salt thereof, or composition (*e.g.*, a composition as described herein, *e.g.,* a composition comprising VS-6063, or a pharmaceutically acceptable salt thereof), *e.g.,* a course of VS-6063, or a pharmaceutically acceptable

salt thereof, or composition (*e.g.*, a composition as described herein, *e.g.,* a composition comprising VS-6063, or a pharmaceutically acceptable salt thereof). In some embodiments, the method comprises, providing a first course of said second therapy; administering VS-6063, or a pharmaceutically acceptable salt thereof, or composition (*e.g.*, a composition as described herein, *e.g.,* a composition comprising VS-6063, or a pharmaceutically acceptable salt thereof); and providing a second course of said second therapy. In some embodiments, the first course is initiated prior to the administration of VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second therapeutic agent in the subject, *e.g.*, in the blood of a subject, is reduced in the second course as compared with the first course. In some embodiments, said second agent is not administered to said subject after the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during the administration of, during the course of administration of, VS-6063, or a pharmaceutically acceptable salt thereof, or composition (*e.g.*, a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition *(e.g.,* a composition as described herein, *e.g.,* a composition comprising VS-6063, or a pharmaceutically acceptable salt thereof).

In some embodiments, said subject has previously received a course of a second therapy, *e.g.,* a second anti-cancer or anti-tumor therapy, *e.g.,* one or more anti-cancer or anti-tumor agents. In some embodiments, said course of second therapy is administered after a diagnosis of ovarian cancer. In some embodiments, said course of second therapy ends prior to, at the same time as, or after, the initial administration of VS-6063, or a pharmaceutically acceptable salt thereof, or composition (*e.g.*, a composition as described herein, *e.g.*, a composition comprising VS-6063, or a pharmaceutically acceptable salt thereof).

In some embodiments, said second therapy is not administered after the first administration of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the method comprises, providing a second course of said second therapy. In some embodiments, the second course replaces the first course and is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the second course is initiated prior to, at the same time as, or after, the initial administration of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, the number of administrations, frequency of administration, amount of second therapeutic agent delivered in an administration, or level of second therapeutic agent in the subject, *e.g.*, in the blood of a subject, is reduced in the second course as compared with the first course. In some embodiments, said second agent is not administered to said subject after the initial administration of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, said second agent is not administered to said subject during the course of VS-6063, or a pharmaceutically acceptable salt thereof, or composition *(e.g.,* a composition as described herein, *e.g.,* a composition comprising VS-6063, or a pharmaceutically acceptable salt thereof). In some embodiments, said second agent is not administered to said subject during a cycle of VS-6063, or a pharmaceutically acceptable salt thereof, or composition (*e.g.*, a composition as described herein, *e.g.*, a composition comprising VS-6063, or a pharmaceutically acceptable salt thereof).

In some embodiments, said second therapy comprises the administration of a therapeutic agent, *e.g.,* an anti-cancer or anti-tumor agent. In some embodiments, said therapeutic agent comprises *e.g.,* mitotic inhibitor, *e.g.,* a taxane, *e.g.,* paclitaxel. In some embodiments, said second therapy comprises the administration of a first and a second therapeutic agent. In some embodiments, said second therapy comprises the administration of and additional agent, *e.g.,* an anti-cancer or anti-tumor agent, *e.g.,* an anti-cancer or anti-tumor agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens.

In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered for a cycle of equal to or greater than 5, 10, 15, 20, 21, 25, 28, 30, 35, 40, 45, or 50 days. In some

embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered for a cycle of 5 to 40, 10 to 40, 10 to 30, or 10 to 25 days. In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered for a cycle of 15 to 40, 15 to 30, or 15 to 25 days. In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered for a cycle of 21 +/-5, 21 +/-4, 21 +/-3, 21 +/-2, or 21 +/-1 days. In some embodiments, VS-6063, or a pharmaceutically acceptable salt thereof, or composition (*e.g*., a composition as described herein, *e.g*., a composition comprising VS-6063, or a pharmaceutically acceptable salt thereof) is administered for a cycle of 21 days. In some embodiments, VS-6063, or a pharmaceutically acceptable salt thereof, or composition (*e.g*., a composition as described herein, *e.g.,* a composition comprising VS-6063, or a pharmaceutically acceptable salt thereof) is administered for a cycle of 28 +/-5, 28 +/-4, 28 +/-3, 28 +/-2, or 28 +/-1 days. In some embodiments, VS-6063, or a pharmaceutically acceptable salt thereof, or composition (*e.g*., a composition as described herein, *e.g.,* a composition comprising VS-6063, or a pharmaceutically acceptable salt thereof) is administered for a cycle of 28 days. In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered 1, 2, 3, or 4 times per day. In some embodiments, VS-6063, or a pharmaceutically acceptable salt thereof, or composition (*e.g*., a composition as described herein, *e.g.,* a composition comprising VS-6063, or a pharmaceutically acceptable salt thereof) is administered 2 times per day. In some embodiments, said VS-6063, or a pharmaceutically acceptable salt thereof, or composition *(e.g.,* a composition as described herein, *e.g.,* a composition comprising VS-6063, or a pharmaceutically acceptable salt thereof) is administered orally. In some embodiments, 50 to 800, 50 to 600, 50 to 500, 50 to 400, 50 to 300, or 50 to 200 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 100 to 800, 100 to 600, 100 to 500, 100 to 400, 100 to 300, or 100 to 200 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 200 to 800, 200 to 600, 200 to 500, 200 to 400, or 200 to 300 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 250 to 800, 250 to 600, 250 to 500, 250 to 400, or 250 to 300 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, the range *(e.g.,* a range as described herein) does not include one or both endpoints, *e.g*., wherein the range does not include the lower endpoint. In some embodiments, 50 +/-25, 100 +/-50, 150 +/-50, 200 +/-50, 250+/-50, 300+/-50, 400+/-50, 500+/-50, 550+/-50 or 600+/-50 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 50 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, 100+/-50, 100+/- 25, 100+/-10, or 100+/-5 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, 100 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 200+/-50, 200+/-25, 200+/-10, or 200+/-5 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, 200 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, 400+/-50, 400+/-25, 400+/-10, or 400+/- 5 mg/day of VS-6063, or a pharmaceutically acceptable salt thereof, or composition (*e.g*., a composition as described herein, *e.g.,* a composition comprising VS-6063, or a pharmaceutically acceptable salt thereof) is administered. In some embodiments, 400 mg/day of VS-6063, or a pharmaceutically acceptable salt thereof, or composition (*e.g.,* a composition as described herein, *e.g.,* a composition comprising VS-6063, or a pharmaceutically acceptable salt thereof) is administered.

In some embodiments, VS-6063, or a pharmaceutically acceptable salt thereof, or composition *(e.g.,* a composition as described herein, *e.g.,* a composition comprising VS-6063, or a pharmaceutically acceptable salt thereof) is administered in multiple cycles (*e.g*., more than one cycle). In some embodiments, the amount of VS-6063, or a pharmaceutically acceptable salt thereof, or composition (*e.g*., a composition as described herein, *e.g.,* a composition comprising VS-6063, or a pharmaceutically acceptable salt thereof) is administered in each cycle is constant (*e.g*., the amount

of VS-6063, or a pharmaceutically acceptable salt thereof, or composition (*e.g.*, a composition as described herein, *e.g.,* a composition comprising VS-6063, or a pharmaceutically acceptable salt thereof) administered is the same). In some embodiments, the amount of VS-6063, or a pharmaceutically acceptable salt thereof, or composition *(e.g.,* a composition as described herein, *e.g.,* a composition comprising VS-6063, or a pharmaceutically acceptable salt thereof) is administered in a cycle is different than the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in another cycle *(e.g.,* a second cycle). In some embodiments, the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in a cycle is smaller than the amount of VS-6063, or a pharmaceutically acceptable salt thereof, or composition *(e.g.,* a composition as described herein, *e.g.,* a composition comprising VS-6063, or a pharmaceutically acceptable salt thereof) that is administered in a subsequent cycle *(e.g.,* a second cycle). In some embodiments, the amount of VS-6063, or a pharmaceutically acceptable salt thereof, or composition *(e.g.,* a composition as described herein, *e.g.,* a composition comprising VS-6063, or a pharmaceutically acceptable salt thereof) that is administered in a cycle is greater than the amount of VS-6063, or a pharmaceutically acceptable salt thereof, or composition (*e.g.*, a composition as described herein, *e.g.,* a composition comprising VS-6063, or a pharmaceutically acceptable salt thereof) that is administered in a subsequent cycle *(e.g.,* a second cycle).

In some embodiments, a unit dosage formulation of 50 +/-25, 100 +/-50, 150 +/-50, 200 +/-50, 250+/-50, 300+/-50, 400+/-50, 500+/-50, 550+/-50 or 600+/-50 mg of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, 200+/-50, 200+/-25, 200+/-10, or 200+/-5 mg/day of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, a unit dosage formulation of 200+/-20, 200+/-10 or 200+/-5 mg of VS-6063, or a pharmaceutically acceptable salt thereof, or composition (*e.g.*, a composition as described herein, *e.g.,* a composition comprising VS-6063, or a pharmaceutically acceptable salt thereof) is administered. In some embodiments, a unit dosage formulation of 200 mg of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, 100+/-50, 100+/- 25, 100+/-10, or 100+/-5 mg/day of VS-6063, or a pharmaceutically acceptable salt thereof, or composition (*e.g.,* a composition as described herein, *e.g.,* a composition comprising VS-6063, or a pharmaceutically acceptable salt thereof) is administered. In some embodiments, a unit dosage formulation of 100+/-20, 100+/-10 or 100+/-5 mg of VS-6063, or a pharmaceutically acceptable salt thereof, or composition *(e.g.,* a composition as described herein, *e.g.,* a composition comprising VS-6063, or a pharmaceutically acceptable salt thereof) is administered. In some embodiments, a unit dosage formulation of 100 mg of VS-6063, or a pharmaceutically acceptable salt thereof, or composition (*e.g.*, a composition as described herein, *e.g.*, a composition comprising VS-6063, or a pharmaceutically acceptable salt thereof) is administered. In some embodiments, 50+/-25, 50+/-10, or 50+/-5 mg/day of VS-6063, or a pharmaceutically acceptable salt thereof, or composition (*e.g.*, a composition as described herein, *e.g.,* a composition comprising VS-6063, or a pharmaceutically acceptable salt thereof) is administered. In some embodiments, a unit dosage formulation of 50+/-20, 50+/-10 or 50+/-5 mg of VS-6063, or a pharmaceutically acceptable salt thereof, or composition (*e.g.*, a composition as described herein, *e.g.,* a composition comprising VS-6063, or a pharmaceutically acceptable salt thereof) is administered. In some embodiments, a unit dosage formulation of 50 mg of VS-6063, or a pharmaceutically acceptable salt thereof, or composition (*e.g.*, a composition as described herein, *e.g.,* a composition comprising VS-6063, or a pharmaceutically acceptable salt thereof) is administered.

In an embodiment, an oral dosage form described herein has a greater $C_{max}$ value than a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g.*, HPMCAS-H, HPMCAS-M, HPMCAS-HF), in an admixture. In an embodiment, the $C_{max}$ value of an oral dosage form described herein is at least 1.1 times (*e.g.*, at least 1.25 times, at least 1.5 times, at least 2 times, at least 3 times, at least 5 times) greater than a reference dosage form of the same dosage amount (*e.g.*, a dosage form having 100 mg of VS-6063 relative to a reference dosage form also having 100 mg of VS-6063). In an embodiment, the $C_{max}$ value of an oral dosage form described herein is at least 1.6 times greater, at least 1.7 times greater, at least 1.8 times greater, at least 1.9 times greater, at least 2 times greater, at least 3 times greater, or at least 4 times greater than a reference dosage form of the same dosage amount.

In an embodiment, an oral dosage form described herein has a reduced food effect relative to a reference oral dosage form, *e.g.,* a dosage form that does not include an excipient (*e.g.,* a cellulosic polymer and derivatives thereof, *e.g.,* Hydroxypropyl Methylcellulose (HPMC) or Hypromellose *(e.g.,* Hydroxypropyl Methylcellulose Acetate Succinate (HPMC-AS) or Hypromellose Acetate Succinate, *e.g.*, HPMCAS-H, HPMCAS-M, HPMCAS-HF) in an admixture. In an embodiment, the difference between the $T_{max}$ value of the fed and fasted states for subjects administered an oral dosage

form described herein is less that the difference between the $T_{max}$ value of the fed and fasted states for subjects administered a reference oral dosage form of the same dosage amount, *e.g.,* a dosage form that does not include an excipient (*e.g.,* a cellulosic polymer and derivatives thereof, *e.g.*, Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M, HPMCAS-HF) in an admixture.

In an embodiment, an oral dosage form described herein has consistent exposure when administered to a subject. In an embodiment, an oral dosage form described herein, when administered to a subject, provides an exposure across multiple subjects of less than 50% of the variability associated with exposure compared with that of a reference oral dosage form of the same dosage amount. In an embodiment, an oral dosage form described herein, when administered to a subject, provides an exposure across multiple subjects of less than 40%, 30%, 20%, or 10% of the variability associated with exposure compared with that of a reference oral dosage form of the same dosage amount.

Also disclosed herein is a unit dosage form having 50 to 800 mg of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, the unit dosage form has 100 +/-50 mg of VS-6063, or a pharmaceutically acceptable salt thereof, or composition (*e.g.*, a composition as described herein, *e.g.*, a composition comprising VS-6063, or a pharmaceutically acceptable salt thereof). In some embodiments, the unit dosage form has 100 +/-10 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 100 mg of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, the unit dosage form has 200 +/-50 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 200 +/-10 mg of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, the unit dosage form has 200 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 400 +/-50 mg of VS-6063, or a pharmaceutically acceptable salt thereof, or composition (*e.g.,* a composition as described herein, *e.g.,* a composition comprising VS-6063, or a pharmaceutically acceptable salt thereof). In some embodiments, the unit dosage form has 400 +/-10 mg of VS-6063, or a pharmaceutically acceptable salt thereof, or composition (*e.g.*, a composition as described herein, *e.g.,* a composition comprising VS-6063, or a pharmaceutically acceptable salt thereof). In some embodiments, the unit dosage form has 400 mg of VS-6063, or a pharmaceutically acceptable salt thereof, or composition *(e.g.,* a composition as described herein, *e.g.,* a composition comprising VS-6063, or a pharmaceutically acceptable salt thereof).

## Detailed Description of the Disclosure

This disclosure is not limited in its application to the details of the compositions, *e.g.*, formulations, *e.g.*, oral dosage forms, or the specific order of preparation or administration of the composition, *e.g.,* formulations, *e.g.,* oral dosage forms. The compositions, *e.g.,* dosage forms described herein may be suitably prepared using other techniques and/or administered in various ways. Also, the phraseology and terminology used herein is for the purpose of description and should not be regarded as limiting.

### *Definitions*

As used herein, the articles "a" and "an" refer to one or to more than one *(e.g.,* to at least one) of the grammatical object of the article.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

As used herein, an amount of a compound effective to treat a disorder (*e.g.*, a disorder as described herein), "effective amount" or " effective course" refers to an amount of the compound which is effective, upon single or multiple dose administration(s) to a subject, in treating a subject, or in curing, alleviating, relieving or improving a subject with a disorder *(e.g.,* a disorder as described herein) beyond that expected in the absence of such treatment (*e.g.*, placebo treatment).

The term "pharmaceutically acceptable," as used herein, refers to a compound or carrier (*e.g.*, excipient) that may be administered to a subject, together with a compound described herein (*e.g.*, VS-6063, VS-6063 free base, VS-6063 hydrochloride), and which does not destroy the pharmacological activity thereof and is nontoxic when administered

in doses sufficient to deliver a therapeutic amount of the compound.

The term, "pharmaceutically acceptable salts," as used herein, refers to derivatives of a compound described herein *(e.g.,* VS-6063, *e.g.,* VS-6063 free base), wherein the compound is modified by converting an existing acid or base moiety to its salt form (*e.g.*, VS-6063 hydrochloride). Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the disclosure include the conventional non-toxic salts of a compound described herein (*e.g.*, VS-6063), formed, for example, from non-toxic inorganic or organic acids. The pharmaceutically acceptable salts of the disclosure can be synthesized from a compound described herein (*e.g.*, VS-6063, VS-6063 free base), which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418 and Journal of Pharmaceutical Science, 66, 2 (1977).

The phrase, "pharmaceutically acceptable derivative or prodrug," as used herein refers to any pharmaceutically acceptable salt, ester, salt of an ester, or other derivative of a compound, VS-6063, which, upon administration to a recipient, is capable of providing (directly or indirectly) a therapeutic agent. Particularly favored derivatives and prodrugs are those that increase the bioavailability of a compound described herein (*e.g.*, VS-6063, VS-6063 free base, VS-6063 hydrochloride) when such compounds are administered to a mammal *(e.g.,* by allowing an orally administered compound to be more readily absorbed into the blood) or which enhance delivery of a compound described herein (*e.g.*, VS-6063, VS-6063 free base, VS-6063 hydrochloride) to a biological compartment, *e.g.*, relative to a compound described herein (*e.g.*, VS-6063, VS-6063 free base, VS-6063 hydrochloride). Preferred prodrugs include derivatives where a group which enhances aqueous solubility or active transport through the gut membrane is appended to the structure of formulae described herein.

The term, "oral dosage form," as used herein, refers to a composition or medium used to administer an agent, *e.g.*, VS-6063, to a subject. Typically, an oral dosage form is administered via the mouth, however, "oral dosage form" is intended to cover any substance which is administered to a subject and is absorbed across a membrane, *e.g.*, a mucosal membrane, of the gastrointestinal tract, including, *e.g.,* the mouth, esophagus, stomach, small intestine, large intestine, and colon. For example, "oral dosage form" covers a solution which is administered through a feeding tube into the stomach.

The term, "treat" or "treatment," as used herein, refers to the application or administration of a compound, alone or in combination with, an additional agent to a subject, *e.g.,* a subject who has a disorder *(e.g.,* a disorder as described herein), a symptom of a disorder, or a predisposition toward a disorder, with the purpose to cure, heal, alleviate, relieve, alter, remedy, ameliorate, improve or affect the disorder.

Co-administration, co-administering or co-providing, as used herein in the context of the administration of therapies, refers to administration at the same time, administration of one therapy before (*e.g.*, immediately before, less than 5, 10, 15, 30, 45, 60 minutes; 1, 2, 3, 4, 6, 8, 10, 12, 16, 20, 24, 48, 72 or more hours before) administration of a secondary therapy.

Course of therapy, as referred to herein, comprises one or more separate administrations of a therapeutic agent. A course of therapy can comprise one or more cycles of a therapeutic agent.

A cycle, as used herein in the context of a cycle of administration of a drug, refers to a period of time for which a drug is administered to a patient. For example, if a drug is administered for a cycle of 21 days, the periodic administration, *e.g.*, daily or twice daily, is given for 21 days. A drug can be administered for more than one cycle. In some embodiments, a first and second or subsequent cycle are the same in terms of one or both of duration and periodic administration. In embodiments, a first and second or subsequent cycle differ in terms of one or both of duration and periodic administration. Rest periods may be interposed between cycles. A rest cycle may be 1, 2, 4, 6, 8, 10, 12, 16, 20, 24 hours, 1, 2, 3, 4, 5, 6, 7 days, or 1, 2, 3, 4 or more weeks in length.

Numerous ranges, *e.g.*, ranges for the amount of a drug administered per day, are provided herein. In some embodiments, the range includes both endpoints. In other embodiments, the range excludes one or both endpoints. By way of example, the range can exclude the lower endpoint. Thus, in such an embodiment, a range of 250 to 400 mg/day, excluding the lower endpoint, would cover an amount greater than 250 that is less than or equal to 400 mg/day.

As used herein, the term "subject" is intended to include human and non-human animals. Exemplary human subjects include a human subject having a disorder, *e.g.,* a disorder described herein. The term "non-human animals" of the invention includes all vertebrates, *e.g.*, non-mammals (such as chickens, amphibians, reptiles) and mammals, such as non-human primates, domesticated and/or agriculturally useful animals, *e.g.*, sheep, dog, cat, cow, pig, etc.

***Compound VS-6063***

The active agent (*e.g.,* active ingredient) is the compound VS-6063 *(e.g.,* VS-6063 free base):

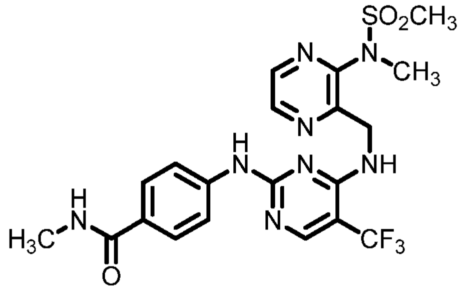

or a pharmaceutically acceptable salt thereof (*e.g.*, VS-6063 hydrochloride), as disclosed in US 7,928,109. VS-6063 is also known as defactinib or PF-04554878.

VS-6063 is a potent inhibitor of the FAK protein tyrosine kinases, and may be adapted to therapeutic use as antiproliferative agents (*e.g.*, anticancer), antitumor (*e.g.,* effective against solid tumors), antiangiogenesis (*e.g.*, stop or prevent proliferation of blood vessels) in mammals, particularly in humans. VS-6063 may be useful in the prevention and treatment of non-hematolotic malignancies. Moreover, VS-6063 may be useful in the prevention and treatment of a variety of human hyperproliferative disorders such as malignant and benign tumors of the liver, kidney, bladder, breast, gastric, ovarian, colorectal, prostate, pancreatic, lung, vulval, thyroid, hepatic carcinomas, sarcomas, glioblastomas, head and neck, and other hyperplastic conditions such as benign hyperplasia of the skin *(e.g.,* psoriasis) and benign hyperplasia of the prostate *(e.g.,* BPH). VS-6063 may also be useful in the prevention and treatment of disorders such as mesothelioma.

In some embodiments, VS-6063 or a pharmaceutically acceptable salt thereof is present in the composition in the amount of 5, 10, 11, 12, 12.5, 13, 14, 15, 20, 25, 30, 35, 40, 45, 50, 55, 60% w/w or greater. In some embodiments, VS-6063 or a pharmaceutically acceptable salt thereof is present in the composition in the amount of about 10 to 50% w/w. In some embodiments, VS-6063 or a pharmaceutically acceptable salt thereof is present in the composition in the amount of about 10% w/w. In some embodiments, VS-6063 or a pharmaceutically acceptable salt thereof is present in the composition in greater than 10% w/w. In some embodiments, VS-6063 or a pharmaceutically acceptable salt thereof is present in the composition in the amount of about 13% w/w. In some embodiments, VS-6063 or a pharmaceutically acceptable salt thereof is present in the composition in the amount of about 25% w/w. In some embodiments, VS-6063 or a pharmaceutically acceptable salt thereof is present in the composition in the amount of about 50% w/w.

***Pharmaceutical compositions and oral dosage forms***

Pharmaceutical compositions are provided which may, for example, be in a form suitable for oral administration such as a tablet, capsule, pill, powder, sustained release formulations, solution, and suspension. The pharmaceutical composition may be in unit dosage forms suitable for single administration of precise dosages. Pharmaceutical compositions may comprise, in addition to VS-6063 or a pharmaceutically acceptable salt thereof, a pharmaceutically acceptable carrier, and may optionally further comprise one or more pharmaceutically acceptable excipients, such as, for example, stabilizers (*e.g.,* a binder, *e.g.,* polymer, *e.g.,* a precipitation inhibitor, for example, Hydroxypropyl Methylcellulose (HPMC) or Hypromellose (*e.g.*, Hydroxypropyl Methylcellulose Acetate Succinate (HPMC-AS) or Hypromellose Acetate Succinate, *e.g.*, HPMCAS-H, HPMCAS-M)), diluents, binders, and lubricants. In some embodiments, the composition provides a free-flowing, cohesive, tableting powder, capable of being directly compressed into a tablet. In addition, the tablet may include other medicinal or pharmaceutical agents, carriers, and or adjuvants. Exemplary pharmaceutical compositions include compressed tablets (*e.g.*, directly compressed tablets), *e.g.*, comprising VS-6063 or a pharmaceutically acceptable salt thereof.

Tablets are also provided comprising the active or therapeutic ingredient (*e.g.*, a compound described herein, *e.g.,* VS-6063 (*e.g.,* VS-6063 free base), or a pharmaceutically acceptable salt thereof (*e.g.*, VS-6063 hydrochloride)). In addition to the active or therapeutic ingredients, tablets may contain a number of inert materials such as carriers. Pharmaceutically acceptable carriers can be sterile liquids, such as water and oils, including those of petroleum, animal, vegetable or synthetic origin, such as peanut oil, sesame oil and the like. Saline solutions and aqueous dextrose can also be employed as liquid carriers. Oral dosage forms for use in accordance with the present disclosure thus may be formulated in conventional manner using one or more pharmaceutically acceptable carriers comprising excipients and auxiliaries, which facilitate processing of the active ingredients into preparations which, can be used pharmaceutically.

Excipients can impart good powder flow and compression characteristics to the material being compressed. Desirable characteristics of excipients can include high-compressibilities as to allow for strong tablets to be made at low compression forces; good powder flow properties that can improve the powder flow of other excipients in the composition; and cohesiveness, for example to prevent a tablet from crumbling during processing, shipping, and handling. Such properties are imparted to these excipients through pretreatment steps, such as dry granulation (*e.g.*, by roller compaction, slugging), wet granulation, spray drying spheronization (*e.g.*, spray dried dispersion, solid nanodispersions) or crystallization (*e.g.*, salt forms) of a pharmaceutical composition. They may be classified according to the role that they play in the final tablet. Some excipients can serve multiple functions. For example, a filler can be a binder and or serve as a precipitation inhibitor. Other excipients which give physical characteristics to a finished tablet are coloring and flavoring agents (*e.g.*, in the case of chewable tablets). Examples of excipients are described, for example, in the Handbook of Pharmaceutical Excipients (5th edition), Edited by Raymond C Rowe, Paul J. Sheskey, and Sian C. Owen; Publisher: Pharmaceutical Press.

For oral administration, the active ingredients, *e.g.,* the compounds described herein *(e.g.,* VS-6063, *e.g.*, VS-6063 free base or VS-6063 hydrochloride), can be formulated readily by combining the active ingredients with pharmaceutically acceptable carriers well known in the art. Such carriers enable the active ingredients described herein to be formulated as tablets, pills, capsules, liquids, gels, syrups, slurries, powders or granules, suspensions or solutions in water or non-aqueous media, and the like, for oral ingestion by a subject. Pharmacological preparations for oral use can be made using a solid excipient, optionally grinding the resulting mixture, and processing the mixture of granules, after adding suitable auxiliaries if desired, to obtain, for example, tablets. Suitable excipients such as diluents, binders or disintegrants may be desirable.

Tablets typically contain diluents or fillers, which are added, for example, to increase the bulk weight of the blend resulting in a practical size for compression. Diluents or fillers that may be used include one or more of calcium salts such as calcium phosphate dibasic and sugars such as lactose, sucrose, dextrose, microcrystalline cellulose, mannitol, and maltodextrin. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. In some embodiments, the diluent or filler is microcrystalline cellulose, which can be manufactured by the controlled hydrolysis of alpha-cellulose. Suitable microcrystalline cellulose will have an average particle size of from about 20 nm to about 200 nm. Suitable microcrystalline cellulose includes Avicel PH 101, Avicel PH 102, Avicel PH 103, Avicel PH 105 and Avicel PH 200, *e.g.*, manufactured by FMC Corporation. In some embodiments, the diluents or filler is lactose. In some embodiments, the lactose has an average particle size of between about 50 $\mu$m and about 500 $\mu$m prior to formulating.

The pharmaceutical composition will generally also include a lubricant. Lubricants are typically added to prevent the tabletting materials from sticking to punches, minimize friction during tablet compression, and to allow for removal of the compressed tablet from the die. Such lubricants are commonly included in the final tablet mix in amounts usually less than 1% by weight per weight of a composition. Examples of lubricants include, but are not limited to, colloidal silica, magnesium trisilicate, talc, magnesium carbonate, magnesium oxide, glycerylbehaptate, polyethylene glycol, ethylene oxide polymers (*e.g.*, Carbowax), sodium lauryl sulphate, magnesium stearate, aluminum stearate, calcium stearate, sodium stearyl fumarate, stearic acid, magnesium lauryl stearate, and mixtures of magnesium stearate with sodium lauryl sulphate. Preferred lubricants include calcium stearate, magnesium stearate and sodium stearyl fumarate. In some embodiments, the lubricant is magnesium stearate. In some embodiments, the amount of lubricant employed is from about 0.1 to about 2.0%, preferably about 0.5 to 1.5% w/w.

Glidants are substances added to a powder that can improve its flowability. Examples of glidants include magnesium stearate, colloidal silicon dioxide (such as the grades sold as Aerosil), starch and talc. Glidants may be present in the pharmaceutical composition at a level of from 0 to about 5% w/w. Again, it should be noted that excipients may serve multiple functions. The lubricant, for example magnesium stearate, may also function as a glidant.

In addition to the diluent(s)/filler(s) and lubricant(s), other conventional excipients may also be present in the pharmaceutical compositions described herein. Such additional excipients include disintegrants, binders, flavouring agents, colours and glidants. Some excipients can serve multiple functions, for example as both binder and tablet disintegrant.

A tablet disintegrant may be present in an amount necessary to expedite dissolution (*e.g.*, increase the rate of tablet disintegration). Disintegrants are excipients which can oppose the physical forces of particle bonding in a tablet or capsule when the dosage form is placed in an aqueous environment. Disintegrants include starch derivatives and salts of carboxymethylcellulose. Examples of pharmaceutically acceptable disintegrants include, but are not limited to, starches, *e.g.*, sodium starch glycolate, pregelatinized starch; clays; celluloses; alginates; gums; cross-linked polymers, *e.g.*, cross-linked polyvinyl pyrrolidone (*e.g.*, polyvinyl polypyrrolidone, PVPP, crospovidone, crospolividone), cross-linked calcium carboxymethylcellulose and cross-linked sodium carboxymethylcellulose (sodium croscarmellose); and soy polysaccharides. In some embodiments, the disintegrant used in the composition is sodium starch glycolate. Generally the amount of disintegrant can be from 0 to about 25% w/w, more commonly from about 1% to about 15% w/w,

and usually less than 15%, less than 10%, or less than 5% w/w, of the composition. In some embodiments, the amount of disintegrant is about 10% w/w of the composition. In some embodiments, the amount of disintegrant is about 3% w/w of the composition.

Binders are excipients which contribute to particle adhesion in a solid formulation. Examples of binders include, but are not limited to, polymers, *e.g.,* starches *(e.g.,* mannitol-starch, *e.g.,* Pearlitol Flash); corn syrup; polysaccharides; gelatin; celluloses and derivatives thereof, *e.g*., microcrystalline cellulose, carboxymethylcellulose, Hydroxypropyl Methylcellulose (HPMC) or Hypromellose (*e.g*., Hydroxypropyl Methylcellulose Acetate Succinate (HPMC-AS) or Hypromellose Acetate Succinate, *e.g*., HPMCAS-H, HPMCAS-M, HPMCAS-HF), HPMC phthalate (HPMCP), (*e.g*., HPMCP-HP55, HPMCP-HP55S), Methocel (*e.g*., Methocel E3LV)), hydroxypropyl cellulose, hydroxyethylcellulose, ethylcellulose, microcrystalline cellulose; and sugars such as lactose, sucrose, dextrose, glucose, maltodextrin, mannitol, xylitol; polymethacrylates (*e.g*., Eudragit, *e.g*., Eudragit L100-55), polyvinyl pyrrolidone (*e.g*., polyvinyl pyrrolidone vinyl acetate copolymer (PVP/VA), Copovidone), other copolymers *(e.g.,* comprising polyethylene glycol, polyvinylcaprolactam, and polyvinylacetate *(e.g.,* polyvinyl phthalate (PVAP); polyvinyl caprolactam - polyvinyl acetate - polyethylene glycol graft copolymer, Soluplus)), sorbitol, pregelatinized starch, alginic acids, and salts thereof such as sodium alginate, magnesium aluminum silicate, polyethylene glycol, and the like. Generally, the amount of binder can vary widely, e.g. from 0% to 95% w/w of the composition. As noted above, excipients may serve multiple functions. For instance, the tabletting diluent may also serve as a binder. In some embodiments, the binder is a precipitation inhibitor. A precipitation inhibitor is generally an excipient that mediates supersaturation stabilization (*e.g*., stabilization of a supersaturated solution) and or slows and or inhibits (*e.g*., prevents rapid) precipitation, *e.g*., in solution or in the GI tract. In some embodiments, the binder is selected from the group consisting of: starches *(e.g.,* mannitol-starch, *e.g.,* Pearlitol Flash); celluloses and derivatives thereof, *e.g*., microcrystalline cellulose, carboxymethylcellulose, hydroxypropyl methylcellulose (HPMC) (*e.g*., HPMC acetate succinate (HPMCAS), (*e.g*., HPMCAS-H, HPMCAS-M), HPMC phthalate (HPMCP), (*e.g.,* HPMCP-HP55, HPMCP-HP55S), Methocel (*e.g.,* Methocel E3LV)), hydroxypropyl cellulose, hydroxyethylcellulose, ethylcellulose, microcrystalline cellulose; and sugars such as lactose, sucrose, dextrose, glucose, maltodextrin, mannitol, xylitol; polymethacrylates (*e.g*., Eudragit, *e.g*., Eudragit L100-55), polyvinyl pyrrolidone (*e.g*., polyvinyl pyrrolidone vinyl acetate copolymer (PVP/VA), Copovidone), other copolymers (*e.g*., comprising polyethylene glycol, polyvinylcaprolactam, and polyvinylactate (*e.g*., Soluplus)). In some embodiments, the binder is polyvinylpyrrolidone (*e.g*., polyvinylpyrrolidone vinyl acetate copolymer (PVP/VA), Copovidone). In some embodiments, the binder is mannitol-starch, *e.g*., Pearlitol Flash. In some embodiments, the binder is a copolymer (*e.g.,* a copolymer comprising polyethylene glycol, polyvinylcaprolactam, and polyvinylactate (*e.g*., Soluplus). In some embodiments, the binder is a polymer. In some embodiments, the polymer is a precipitation inhibitor. In some embodiments, the precipitation inhibitor is a HPMC *(e.g.,* HPMCAS, *e.g.,* HPMCAS-H, HPMCAS-M, or HPMCAS-HF).

In some embodiments, more than one binder is used in the composition. In some embodiments, the amount of binder is from about 0 to 60% w/w of the composition. In some embodiments, the amount of binder present in the composition is 0, 5, 10, 15, 20, 25, 30, 35, 40, 45, 50, 55, 60, 65, 70, 75, 80% or greater weight per weight of composition. In some embodiments, the amount of binder is about 20 to about 80% w/w of the composition. In some embodiments, the amount of binder is about 50% w/w of the composition. In some embodiments, the amount of binder is about 80% w/w of the composition.

The dosage may vary depending upon the dosage form employed and the route of administration utilized. The exact formulation, route of administration and dosage can be chosen by the individual physician in view of the patient's condition. (See *e.g.,* Fingl, et al., 1975, in "The Pharmacological Basis of Therapeutics"). Lower or higher doses than those recited above may be required. Specific dosage and treatment regimens for any particular subject will depend upon a variety of factors, including the activity of the specific compound employed, the age, body weight, general health status, sex, diet, time of administration, rate of excretion, drug combination, the severity and course of the disease, condition or symptoms, the subject's disposition to the disease, condition or symptoms, and the judgment of the treating physician.

Tablets may be plain, film, or sugar coated bisected, embossed, layered, or sustained-release. They can be made in a variety of sizes, shapes, and colors. Tablets may be swallowed, chewed, or dissolved in the buccal cavity or beneath the tongue.

Three processes are generally used for making compressed tablets: wet granulation, direct compression and dry granulation. The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

Direct compression is a relatively quick process where powdered materials are compressed directly without changing the physical and chemical properties of the drug. The active ingredient(s) (*e.g*., a compound as described

herein, *e.g.,* VS-6063, *e.g.,* VS-6063 free base or VS-6063 hydrochloride), direct compression excipients and other auxiliary substances, such as a glidant and lubricant are blended (*e.g.,* in a twin shell blender or similar low shear apparatus) before being compressed into tablets. The method typically consists of blending of the ingredients, dry screening, lubrication and compression.

The dry granulation method may be used where one of the constituents, either the drug or the diluent, has sufficient cohesive properties to be tableted. The method consists of blending, slugging (or roller compaction of) the ingredients, dry screening, lubrication and compression. In some embodiments, dry granulation is used to improve *e.g.,* processibility, *e.g.,* flow properties of the blend *(e.g.,* a composition as described herein, *e.g.,* a composition comprising VS-6063).

The wet granulation method may be used to, *e.g.,* convert a powder mixture into granules having improved flow and cohesive properties for tableting. The procedure consists of mixing the powders, *e.g.*, in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

Oral dosage forms may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

### *Methods of treatment and administration*

Disclosed herein are methods for treating abnormal cell growth, *e.g.*, cancer, comprising administering an oral dosage form comprising VS-6063, or a pharmaceutically acceptable salt thereof.

*Abnormal cell growth*

Abnormal cell growth, as used herein and unless otherwise indicated, refers to cell growth that is independent of normal regulatory mechanisms (*e.g.*, loss of contact inhibition). This includes the abnormal growth of: (1) tumor cells (tumors) that proliferate for example by expressing a mutated tyrosine kinase or overexpression of a receptor tyrosine kinase; (2) benign and malignant cells of other proliferative diseases for example in which aberrant tyrosine kinase activation occurs; (3) any tumors that proliferate for example by receptor tyrosine kinases; (4) any tumors that proliferate for example by aberrant serine/threonine kinase activation; and (5) benign and malignant cells of other proliferative diseases for example in which aberrant serine/threonine kinase activation occurs. Abnormal cell growth can refer to cell growth in epithelial *(e.g.,* carcinomas, adenocarcinomas); mesenchymal *(e.g.,* sarcomas (e.g. leiomyosarcoma, Ewing's sarcoma)); hematopoetic (*e.g.*, lymphomas, leukemias, myelodysplasias (*e.g.*, pre-malignant)); or other (*e.g.*, melanoma, mesothelioma, and other tumors of unknown origin) cells.

Abnormal cell growth can refer to cancer, which includes, but is not limited to, lung cancer *(e.g.,* non-small cell lung cancer (NSCLC), *e.g.*, KRAS mutant NSCLC; metastatic cancer), bone cancer, pancreatic cancer, skin cancer, cancer of the head or neck, cutaneous or intraocular melanoma, uterine cancer, ovarian cancer (*e.g.*, advanced or metastatic ovarian cancer), rectal cancer, cancer of the anal region, stomach cancer, colon cancer, breast cancer (*e.g.,* triple-negative breast cancer *(e.g.,* breast cancer which does not express the genes for the estrogen receptor, progesterone receiptor, and Her2/neu)), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, mesothelioma (*e.g.,* malignant pleural mesothelioma, *e.g.,* surgical resectable malignant pleural mesothelioma) or a combination of one or more of the foregoing cancers. In some embodiments, the cancer is metastatic. In some embodiments, the abnormal cell growth is locally recurring (*e.g.*, the subject has a locally recurrent disease, *e.g.*, cancer).

In some embodiments, the method comprises administering to a mammal an oral dosage form described herein that is effective in treating non-hematolotic malignancies. In some embodiments, the method is effective in treating

breast, lung, and ovarian cancer. In an embodiment, the breast cancer is triple-negative breast cancer (*e.g.*, breast cancer which does not express the genes for the estrogen receptor, progesterone receiptor, and Her2/neu). In an embodiment, the lung cancer is non-small cell lung cancer (NSCLC), *e.g.*, KRAS mutant NSCLC. In an embodiment, the ovarian cancer is advanced ovarian cancer (*e.g.*, advanced ovarian cancer or metastatic ovarian cancer).

In an embodiment, the method comprises administering a mammal a composition, *e.g.*, an oral dosage form described herein that is effective in treating mesothelioma (*e.g.*, malignant pleural mesothelioma, *e.g.*, surgical resectable malignant pleural mesothelioma).

Methods of the present disclosure contemplate single as well as multiple administrations of a therapeutically effective amount of a composition as described herein. Compositions, *e.g.*, a composition as described herein, can be administered at regular intervals, depending on the nature, severity and extent of the subject's condition. In some embodiments, a composition described herein is administered in a single dose. In some embodiments, a composition described herein is administered in multiple doses. In some embodiments, a therapeutically effective amount of a composition, *e.g.*, a composition as described herein, may be administered orally and periodically at regular intervals (*e.g.*, 1, 2, 3, 4, 5, 6, 7, 8, 9, 10 or more times every 1, 2, 3, 4, 5, or 6 days, or every 1, 2, 3, 4, 5, 6, 7, 8, or 9 weeks, or every 1, 2, 3, 4, 5, 6, 7, 8, 9 months or longer).

In some embodiments, a compositions described herein is administered at a predetermined interval (*e.g.*, 1, 2, 3, 4, 5, 6, 7, 8, 9, 10 or more times every 1, 2, 3, 4, 5, or 6 days, or every 1, 2, 3, 4, 5, 6, 7, 8, or 9 weeks, or every 1, 2, 3, 4, 5, 6, 7, 8, 9 months or longer).

*Combinations*

The oral dosage forms and methods described herein may be administered in combination with an additional (*e.g.*, a second, secondary) agent (*e.g.*, therapeutic agent). The additional agent can include an anti-tumor or anti-cancer agent, *e.g.,* an anti-tumor agent selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, antibodies, cytotoxics, anti-hormones, and anti-androgens.

An oral dosage form described herein may be applied as a sole therapy or may involve one or more other anti-tumor substances, for example those selected from, for example, mitotic inhibitors, for example taxanes, *e.g.*, docetaxel (Taxotere), paclitaxel; alkaloids, *e.g.*, vinblastine; alkylating agents, for example platinum-coordinated alkylating compounds, *e.g.,* platins, *e.g.,* cis-platin, oxaliplatin, and carboplatin; and cyclophosphamide; anti-metabolites, for example gemcitabine, 5-fluorouracil, capecitabine, cytosine arabinoside and hydroxyurea, growth factor inhibitors; cell cycle inhibitors; intercalating antibiotics, for example adriamycin and bleomycin; enzymes, for example interferon; and anti-hormones, for example anti-estrogens such as Nolvadex (tamoxifen) or, for example anti-androgens such as Casodex (4'-cyano-3-(4-fluorophenylsulphonyl)-2-hydroxy-2-methyl-3'-(trifluoromethyl)propionanilide).

An oral dosage form described herein may be used alone or in combination with one or more of a variety of anti-cancer agents or supportive care agents. For example, the oral dosage forms described herein may be used with cytotoxic agents, *e.g.,* one or more selected from the group consisting of a camptothecin, irinotecan HCl (Camptosar), edotecarin, SU-11248, epirubicin (Ellence); taxanes, *e.g.*, docetaxel (Taxotere), paclitaxel; rituximab (Rituxan) bevacizumab (Avastin), imatinib mesylate (Gleevac), Erbitux, gefitinib (Iressa), and combinations thereof. Also disclosed herein is the use of the oral dosage forms of the invention together with hormonal therapy, *e.g.*, exemestane (Aromasin), Lupron, anastrozole (Arimidex), tamoxifen citrate (Nolvadex), Trelstar, and combinations thereof. Also disclosed herein is an oral dosage forms described herein alone or in combination with one or more supportive care products, *e.g.,* a product selected from the group consisting of Filgrastim (Neupogen), ondansetron (Zofran), Fragmin, Procrit, Aloxi, Emend, or combinations thereof. Such conjoint treatment may be achieved by way of the simultaneous, sequential or separate dosing of the individual components of the treatment.

Oral dosage forms described herein may be used with the following, which is a non-limiting list of examples of additional or secondary agents, e.g., anti-tumor agents, anti-cancer agents, that may be used with the oral dosage forms described herein.

- **Alkylating agents** include, but are not limited to, nitrogen mustard N-oxide, cyclophosphamide, ifosfamide, melphalan, busulfan, mitobronitol, carboquone, thiotepa, ranimustine, nimustine, temozolomide, AMD-473, altretamine, AP-5280, apaziquone, brostallicin, bendamustine, carmustine, estramustine, fotemustine, glufosfamide, ifosfamide, KW-2170, mafosfamide, and mitolactol; platinum-coordinated alkylating compounds, e.g., platins, such as cisplatin, carboplatin, eptaplatin, lobaplatin, nedaplatin, oxaliplatin or satrplatin;
- **Anti-metabolites** include but are not limited to, methotrexate, 6-mercaptopurine riboside, mercaptopurine, gemcitabine, 5-fluorouracil (5-FU) alone or in combination with leucovorin, tegafur, UFT, doxifluridine, carmofur, cytarabine, cytarabine ocfosfate, enocitabine, S-1, gemcitabine, fludarabin, 5-azacitidine, capecitabine, cladribine, clofarabine, decitabine, eflornithine, ethynylcytidine, cytosine arabinoside, hydroxyurea, TS-1, melphalan, nelarabine, nolatrexed, ocfosfate, disodium premetrexed, pentostatin, pelitrexol, raltitrexed, triapine, trimetrexate, vidarabine, vincristine, vinorelbine;

◦ **Antibiotics** include but are not limited to: aclarubicin, actinomycin D, amrubicin, annamycin, bleomycin, daunorubicin, doxorubicin, elsamitrucin, epirubicin, galarubicin, idarubicin, mitomycin C, nemorubicin, neocarzinostatin, peplomycin, pirarubicin, rebeccamycin, stimalamer, streptozocin, valrubicin or zinostatin;

◦ **Hormonal therapy agents,** *e.g.*, exemestane (Aromasin), Lupron, anastrozole (Arimidex), doxercalciferol, fadrozole, formestane, anti-estrogens such as tamoxifen citrate (Nolvadex) and fulvestrant, Trelstar, toremifene, raloxifene, lasofoxifene, letrozole (Femara), or anti-androgens such as bicalutamide, flutamide, mifepristone, nilutamide, Casodex® (4'-cyano-3-(4-fluorophenylsulphonyl)-2-hydroxy-2-methyl-3'-(trifluoromethyl)propionanilide) and combinations thereof;

◦ **Plant derived anti-tumor substances** include for example those selected from mitotic inhibitors, for example alkaloids, *e.g.,* vinblastine; taxanes, *e.g.*, docetaxel (Taxotere), paclitaxel;

◦ **Cytotoxic topoisomerase inhibiting agents** include one or more agents selected from the group consisting of aclarubicin, amonafide, belotecan, camptothecin and camptothecin derivatives, including but are not limited to 10-hydroxycamptothecin, 9-aminocamptothecin, diflomotecan, irinotecan, irinotecan HCl (Camptosar), edotecarin, epirubicin (Ellence), etoposide, exatecan, gimatecan, lurtotecan, mitoxantrone, pirarubicin, pixantrone, rubitecan, sobuzoxane, SN-38, tafluposide, and topotecan, and combinations thereof;

◦ **Immunologicals** include interferons and numerous other immune enhancing agents. Interferons include interferon alpha, interferon alpha-2a, interferon, alpha-2b, interferon beta, interferon gamma-1a or interferon gamma-nl. Other agents include filgrastim, lentinan, sizofilan, TheraCys, ubenimex, WF-10, aldesleukin, alemtuzumab, BAM-002, dacarbazine, daclizumab, denileukin, gemtuzumab ozogamicin, ibritumomab, imiquimod, lenograstim, lentinan, melanoma vaccine (Corixa), molgramostim, OncoVAX-CL, sargramostim, tasonermin, tecleukin, thymalasin, tositumomab, Virulizin, Z-100, epratuzumab, mitumomab, oregovomab, pemtumomab, and Provenge;

◦ **Biological response modifiers** are agents that modify defense mechanisms of living organisms or biological responses, such as survival, growth, or differentiation of tissue cells to direct them to have anti-tumor activity. Such agents include krestin, lentinan, sizofuran, picibanil, or ubenimex;

◦ **Other anticancer agents** include alitretinoin, ampligen, atrasentan, bexarotene, bortezomib, Bosentan, calcitriol, exisulind, finasteride, fotemustine, ibandronic acid, miltefosine, mitoxantrone, 1-asparaginase, procarbazine, dacarbazine, hydroxycarbamide, pegaspargase, pentostatin, tazarotene, TLK-286, Velcade, Tarceva, tretinoin, CTLA4 inhibitors, or a farnesyl protein transferase inhibitors;

◦ **Other anti-angiogenic compounds** include acitretin, fenretinide, thalidomide, zoledronic acid, angiostatin, aplidine, cilengtide, combretastatin A-4, endostatin, halofuginone, rebimastat, removab, Revlimid, squalamine, ukrain and Vitaxin;

◦ **Platinum-coordinated compounds** include but are not limited to, cisplatin, carboplatin, nedaplatin, or oxaliplatin;

◦ **Tyrosine kinase inhibitors** are Iressa or SU5416;

◦ **Antibodies** include Herceptin, Erbitux, Avastin, Rituximab, or a cytotoxic lymphocyte antigen 4 (CTLA4); and

◦ **Interferons** include interferon alpha, interferon alpha-2a, interferon, alpha-2b, interferon beta, interferon gamma-la or interferon gamma-nl.

## BRIEF DESCRIPTION OF THE DRAWINGS

**FIG. 1** shows exemplary mean dissolution profiles of 100mg VS-6063 IR tablet formulations

**FIG. 2** shows exemplary mean dissolution profiles of 100mg VS-6063 IR tablet formulations

**FIG. 3** shows exemplary mean dissolution profiles of 100mg VS-6063 IR tablet formulations

**FIG. 4** shows exemplary mean dissolution profiles of 100mg VS-6063 IR tablet formulations in 0.1M HCl

**FIG. 5** shows exemplary mean (n=4) VS-6063 Concentrations in Plasma Following an Oral Gavage Dose of VS-6063 in Various Formulations to Male Beagle Dogs at 10 mg API/kg - Rectilinear Scale

**FIG. 6** shows exemplary mean (n=4) VS-6063 Concentrations in Plasma Following an Oral Gavage Dose of VS-6063 in Various Formulations to Male Beagle Dogs at 10 or 20 mg API/kg - Rectilinear Scale

## EXAMPLES

The disclosure is further described in the following examples, which do not limit the scope of the claims.

## Example 1. Dry Granulation of VS-6063 formulations

VS-6063 immediate release (IR) formulations were prepared using a dry granulation approach. The composition of the formulations is shown on **Table 1:**

**Table 1: Composition of 100mg VS-6063 IR tablet formulation**

| Composition | HPMC-AS Formulation | | | PVP VA/Soluplus Forumlation | | |
|---|---|---|---|---|---|---|
| | % w/w | mg/ tablet | g/per 200g batch | % w/w | mg/ tablet | g/per 200g batch |
| **Intragranular blend** | | | | | | |
| VS-6063* (free base) | 13.529 | 108.23 (100.00) | 27.058 | 13.529 | 108.23 (100.00) | 27.058 |
| HPMC AS-HF | 40.586 | 324.69 | 81.172 | - | - | - |
| PVP VA | - | - | - | 20.293 | 162.35 | 40.586 |
| Soluplus | - | - | - | 20.293 | 162.35 | 40.586 |
| Avicel PH 102 | 20.943 | 167.54 | 41.886 | - | - | - |
| Lactose Monohydrate (FastFlo 316) | 20.943 | 167.54 | 41.886 | - | - | - |
| Pearlitol® Flash | - | - | - | 34.885 | 279.08 | 69.770 |
| Crospovidone | - | - | - | 10.000 | 80.00 | 20.000 |
| Sodium starch glycolate | 3.000 | 24.00 | 6.000 | - | - | - |
| Magnesium | 0.500 | 4.00 | 1.000 | 0.500 | 4.00 | 1.000 |
| Stearate | | | | | | |
| **Sub-total** | **99.50** | **796.00** | **199.00** | **99.50** | **796.01** | **199.00** |
| Extragranular blend | | | | | | |
| Magnesium stearate | 0.500 | 4.00 | Final blend for tableting based on yield of granulate available | 0.500 | 4.00 | Final blend for tableting based on yield of granulate available |
| **Total** | **100.00** | **800.00** | | **100.00** | **800.00** | |

The intragranular blend was prepared by screening VS-6063 and all excipients through 600$\mu$m sieves before use. The required amount of the functional excipients and VS-6063 was weighed out and pre-blended for 1 minute using a large plastic spatula, followed by mixing on the Turbula mixer at a setting of 32rpm for 5 minutes. The remaining screened components (except the magnesium stearate) were added to the pre-blend mixed for 1 minute using a large plastic spatula, followed by mixing on the Turbula mixer at a setting of 32rpm for 15 minutes. Final blending on the Turbula mixer was conducted at a setting of 32rpm for 3 minutes after addition of magnesium stearate. The intragranular blend was then roller compacted. The granule properties for two IR tablet formulations were characterized and compared to the direct compression batch of the same composition; shown in **Table 2:**

**Table 2: Powder characterisation for 100mg VS-6063 tablet formulations**

| Test | HPMC-AS Dry granulation | HPMC-AS Direct Compression | PVP VA/Soluplus Dry granulation | PVP VA/Soluplus Direct Compression |
|---|---|---|---|---|
| Bulk Density (g/mL) | 0.522 | 0.282 | 0.572 | Not tested |
| Tapped Density (g/mL) | 0.679 | 0.570 | 0.748 | Not tested |

(continued)

| Test | HPMC-AS Dry granulation | HPMC-AS Direct Compression | PVP VA/Soluplus Dry granulation | PVP VA/Soluplus Direct Compression |
|---|---|---|---|---|
| Hausner Ratio | 1.30 | 2.02 | 1.31 | Not tested |
| Carr's Index (%) | 23.20 | 50.60 | 23.49 | Not tested |
| Angle of Repose (°) | 17.54 | 38.37 | 36.49 | Not tested |

The particle size analysis data indicate bi-modal particle size distribution with approximately 20% of the particle size distribution >710μm and approximately 20% of the particle size distribution < 75μm.

The physical properties of the blends were determined and compared to the direct compression formulation (HPMC-AS formulation only); **(Table 2).** The Hausner ratio for the blends tested was >1.3 and the Carr's index was **>23**% indicating the blends may have poor powder flow, cohesiveness and high interparticulate friction. However, the angle of repose does indicate passable powder flow.

In comparison to a direct compression approach, a higher compression force may be required to achieve the desired tablet hardness for the dry granulated blends.

**Table 3: Tablet compression data for 100mg VS-6063 tablet formulations**

| Test | HPMC-AS Dry granulation | HPMC-AS Direct Compression | PVP VA/Soluplus Dry granulation | PVP VA/Soluplus Direct Compression |
|---|---|---|---|---|
| Average Hardness (kP) | 12.7 %RSD 8.7 Min 11.9 Max 14.0 | 13.3 %RSD 3.8 Min 12.8 Max 14.1 | 11.7 %RSD 8.8 Min 10.4 Max 12.7 | 11.2 |
| Average Tablet Weight (mg) | 805.4 %RSD 0.9 Min 797.8 Max 821.6 | 804.7 %RSD 0.7 Min 796.7 Max 814.4 | 793.0 %RSD 2.1 Min 763.9 Max 810.2 | 797.1 %RSD 0.5 Min 791.2 Max 803.2 |
| Average Tablet Thickness (mm) | 5.6 %RSD 0.3 Min 5.5 Max 5.6 | 6.2 %RSD 0.6 Min 6.2 Max 6.3 | 5.4 %RSD 1.7 Min 5.2 Max 5.5 | 5.6 %RSD 0.4 Min 5.6 Max 5.6 |
| Compression Force (lbs) | 1800 | 800 | 10000 | 3500 |
| Disintegration time (mins) | 10.5 | 8* | 20 | 8 |

**Figure 1** shows that for the HPMC-AS formulation, dry granulation may slow dissolution after a pH switch (e.g., gastric to intestinal pH) in comparison to the dissolution of tablets prepared using a direct compression approach.

For the PVP VA/Soluplus formulation, the dissolution profiles for tablets prepared via a dry granulation approach may be slower in comparison to tablets prepared using a direct compression approach. The dissolution profile generated was very similar to that for a VS-6063 reference product (e.g., unformulated product).

**Reference Example 2. Dry Granulation of VS-6063 immediate release formulations**

One batch of the VS-6063 IR formulations was prepared **(Table 4)**, using a dry granulation approach:

**Table 4: Composition of 100mg VS-6063 tablet formulations**

| Composition | PVP VA/Soluplus | | |
|---|---|---|---|
| | % w/w | mg/tablet | g/per 200g batch |
| **Intragranular blend** | | | |

(continued)

| Composition | PVP VA/Soluplus | | |
|---|---|---|---|
| | % w/w | mg/tablet | g/per 200g batch |
| VS-6063* | 13.529 | 108.23 | 27.058 |
| (free base) | | (100.00) | |
| PVP VA | 20.293 | 162.35 | 40.586 |
| Soluplus | 20.293 | 162.35 | 40.586 |
| Pearlitol® Flash | 34.885 | 279.08 | 69.770 |
| Crospovidone | 10.000 | 80.00 | 20.000 |
| Magnesium Stearate | 0.500 | 4.00 | 1.000 |
| **Sub-total** | **99.50** | **796.01** | **199.00** |
| Extragranular blend | | | |
| Magnesium stearate | 0.500 | 4.00 | Final blend for tableting based on yield of granulate available |
| **Total** | **100.00** | **800.00** | |

The intragranular blend was prepared by screening VS-6063 and all excipients through 600μm sieves before use. The required amount of the functional excipients and VS-6063 was weighed out and pre-blended for 1 minute using a large plastic spatula, followed by mixing on the Turbula mixer at a setting of 32rpm for 5 minutes. The remaining screened components (except the magnesium stearate) were added to the pre-blend, mixed for 1 minute using a large plastic spatula, followed by mixing on the Turbula mixer at a setting of 32rpm for 15 minutes. Final blending on the Turbula mixer was conducted at a setting of 32rpm for 3 minutes after addition of magnesium stearate. The intragranular blend was then roller compacted.

After completion of the dry granulation process, the compacted ribbons were passed through 1.4mm, 1.18mm and 850μm sieves. The tablet blend was then prepared by mixing the granules with magnesium stearate using the Turbula mixer at 32rpm for 3 minutes.

**Table 5: Powder characterisation for 100mg VS-6063 tablet formulations**

| Test | PVP VA/Soluplus Prior to dry granulation | PVP VA/Soluplus Dry granulation softer ribbons | PVP VA/Soluplus Dry granulation harder ribbons |
|---|---|---|---|
| Bulk Density (g/mL) | 0.431 | 0.529 | 0.572 |
| Tapped Density (g/mL) | 0.610 | 0.761 | 0.748 |
| Hausner Ratio | 1.41 | 1.44 | 1.31 |
| Carr's Index (%) | 29.29 | 30.49 | 23.49 |
| Angle of Repose (°) | 22.39 | 35.83 | 36.49 |
| | | | |

The particle size analysis data indicate that a larger portion of the blend consisted of fines (approximately 45% <150μm), in comparison to roller compacted PVP VA/Soluplus formulations (approximately 35% <150μm), which generated harder ribbons.

The physical properties of the blend pre and post roller compaction was also determined and indicated that by using a dry granulation approach, the flowability may not be significantly improved **(Table 5).** The data also indicate that that by formation of softer ribbons, the powder flow properties may not be improved in comparison to granules formed from harder ribbons **(Table 5).**

In comparison to a direct compression approach, a higher compression force of 80001bs may be required to achieve the desired tablet hardness, for the dry granulation formulation. However, this may be lower in comparison to 10000lbs compression force that may be required for manufacture of tablets from the granules prepared from harder ribbons.

**Table 6: Tablet compression data for 100mg VS-6063 tablet formulations.**

| Test | PVP VA/Soluplus Dry granulation softer ribbons | PVP VA/Soluplus Dry granulation harder ribbons | PVP VA/Soluplus Direct Compression |
|---|---|---|---|
| Average Hardness (kP) | 10.6 %RSD 2.7 Min 10.4 Max 10.8 | 11.7 %RSD 8.8 Min 10.4 Max 12.7 | 11.2 |
| Average Tablet Weight (mg) | 799.8 %RSD 1.2 Min 779.1 Max 811.0 | 793.0 %RSD 2.1 Min 763.9 Max 810.2 | 797.1 %RSD 0.5 Min 791.2 Max 803.2 |
| Average Tablet Thickness (mm) | 5.4 %RSD 0.2 Min 5.4 Max 5.4 | 5.4 %RSD 1.7 Min 5.2 Max 5.5 | 5.6 %RSD 0.4 Min 5.6 Max 5.6 |
| Compression Force (lbs) | 8000 | 10000 | 3500 |
| Disintegration time (mins) | 13 | 20 | 8 |

To determine whether addition of further Crospovidone to the formulation would improve the disintegration time, the Crospovidone content was further increased to a total disintegrant content of 13%, where 3% was added to the extragranular blend. Tablets were compressed using 18.97 x 10.41mm oval tooling tablet tooling on the Carver tablet press. Compression force was adjusted to achieve a tablet hardness of 10 - 14kP. Tablet compression data is shown in **Table 6**.

**Table 7: Tablet compression data for 100mg VS-6063 tablet formulations.**

| Test | Provisional Acceptance Criteria | PVP VA/Soluplus Dry granulation with addition of 3% disintegrant |
|---|---|---|
| Average Hardness (kP) | 10-14kP | 11.6 %RSD 6.9 Min 10.8 Max 12.5 |
| Average Tablet Weight (mg) | Target ± 5.0%, RSD ≤2% | 800.2 %RSD 2.2 Min 779.2 Max 835.4 |
| Average Tablet Thickness (mm) | Run and record | 5.5 %RSD 2.0 Min 5.4 Max 5.7 |
| Compression Force (lbs) | In-process control, not part of the finished product specification | 8000 |
| Disintegration time (mins) | Run and record | 10.5 |

Compared with direct compression, granules made by dry granulation may not improve compressibility or powder flow. While using softer ribbons may improve the disintegration time for the resultant tablets, the dissolution profile was not improved in comparison to the reference product (**Figure 2**), indicating that the functionality of the precipitation inhibitors in the PVP VA/Soluplus formulation may be lost during dry granulation.

**Example 3. Dry Granulation of VS-6063 formulations**

One batch of the VS-6063 IR formulations has been prepared (**Table 8**), using a dry granulation approach:

**Table 8: Composition of 100mg VS-6063 IR tablet formulation**

| Composition | % w/w | mg/tablet | g/per 300g batch |
|---|---|---|---|
| **Intragra nular blend** | | | |
| VS-6063* (free base) | 13.529 | 108.23 (100.00) | 40.587 |

(continued)

| Composition | % w/w | mg/tablet | g/per 300g batch |
|---|---|---|---|
| **Intragra nular blend** | | | |
| HPMC AS-HF | 40.586 | 324.69 | 121.758 |
| Avicel PH 102 | 20.943 | 167.54 | 62.829 |
| Lactose Monohydrate (FastFlo 316) | 20.943 | 167.54 | 62.829 |
| Sodium starch glycolate | 3.000 | 24.00 | 9.000 |
| Magnesium Stearate | 0.500 | 4.00 | 1.500 |
| **Sub-total** | **99.501** | **796.00** | **298.503** |
| **Extragranular blend** | | | |
| Magnesium stearate | 0.500 | 4.00 | Final blend for tableting based on yield of granulate available |
| **Total** | **100.00** | **800.00** | |

The intragranular blend preparation was conducted by screening VS-6063 and all excipients through 600μm sieves before use. The required amount of the functional excipients and the API (*e.g.,* VS-6063) was weighed out and pre-blended for 1 minute using a large plastic spatula, followed by mixing on the Turbula mixer at a setting of 32rpm for 5 minutes. The remaining screened components (except the magnesium stearate) were added to the pre-blend, mixed for 1 minute using a large plastic spatula, followed by mixing on the Turbula mixer at a setting of 32rpm for 15 minutes. Final blending on the Turbula mixer was conducted at a setting of 32rpm for 3 minutes after addition of magnesium stearate. The intragranular blend was then roller compacted.

The granule properties for the IR tablet formulations are shown in **Table 9** and compared to the direct compression batch of the same composition:

**Table 9: Powder characterisation for 100mg VS-6063 tablet formulations**

| Test | HPMC-AS Dry granulation batch 1 | HPMC-AS Dry granulation batch 2 | HPMC-AS Dry granulation batch 3 | HPMC-AS Direct Compression |
|---|---|---|---|---|
| Bulk Density (g/mL) | Not tested | 0.470 | 0.522 | 0.282 |
| Tapped Density (g/mL) | Not tested | 0.640 | 0.679 | 0.570 |
| Hausner Ratio | Not tested | 1.36 | 1.30 | 2.02 |
| Carr's Index (%) | Not tested | 26.56 | 23.20 | 50.60 |
| Angle of Repose (°) | Not tested | 29.87 | 17.54 | 38.37 |

The particle size analysis data indicated a bi-modal particle size distribution for both batches.

The physical properties of the blends were determined and compared to the direct compression formulation (HPMC-AS formulation only). The data indicated that by using a dry granulation approach the flowability may be improved (**Table 9**). However, the Hausner ratio for these experiments was >1.3 and the Carr's index was >**23**%, indicating possibly poor powder flow, cohesiveness and high interparticulate friction. Still the data for angle of repose indicates a passable powder flow.

For both dry granulation formulations in comparison to a direct compression approach, a higher compression force may be required to achieve the desired tablet hardness.

**Table 10: Tablet compression data for 100mg VS-6063 tablet formulations.**

| Test | HPMC-AS Dry granulation batch 1 | HPMC-AS Dry granulation batch 2 | HPMC-AS Dry granulation batch 3 | HPMC-AS Direct Compression |
|---|---|---|---|---|
| Average Hardness (kP) | 12.0 %RSD 7.0 Min 10.6 Max 13.6 | 12.3 %RSD 3.3 Min 11.9 Max 12.8 | 12.7 %RSD 8.7 Min 11.9 Max 14.0 | 13.3 %RSD 3.8 Min 12.8 Max 14.1 |
| Average Tablet Weight (mg) | 799.9 %RSD 0.8 Min 780.4 Max 812.2 | 802.4 %RSD 0.6 Min 787.6 Max 812.9 | 805.4 %RSD 0.9 Min 797.8 Max 821.6 | 804.7 %RSD 0.7 Min 796.7 Max 814.4 |
| Average Tablet Thickness (mm) | 5.9 %RSD 0.9 Min 5.7 Max 6.0 | 5.7 %RSD 0.7 Min 5.6 Max 5.8 | 5.6 %RSD 0.3 Min 5.5 Max 5.6 | 6.2 %RSD 0.6 Min 6.2 Max 6.3 |
| Compression Force (lbs) | 1300 | 1500 | 1800 | 800 |
| Disintegration time (mins) | Not measured | Not measured | 10.5 | 8* |

The data from **Figure 3** suggests that for the HPMC-AS formulation by introducing a dry granulation approach, the dissolution profiles of all the batches after the pH switch are comparable to tablets prepared using a direct compression approach.

**Example 4. In Vivo Assessment of the Pharmacokinetics of VS-6063 in Various Formulations**

Abbreviations used:

$$\text{Relative bioavailability} = AUC_{inf}\text{ test formulation}/AUC_{inf}\text{ reference formulation}$$

$C_{max}$ Maximum concentration observed

$T_{max}$ Time of maximum concentration

$t_{1/2}$ Terminal half life

$AUC_{inf}$ Area under the concentration-time curve from time zero extrapolated to infinity

$AUC_{last}$ Area under the concentration-time curve from time zero to the last quantifiable concentration

*Study Design*

4 groups of 1 male beagle dog per group received an oral gavage dose of VS-6063 in various formulations in a 4-session crossover design with a minimum 3-day washout period between dose sessions. In each session, VS-6063, VS-6063-25% SDD in Soluplus/PVP-A, VS-6063-25% physical mixture with Soluplus/PVP-A, and VS-6063-10% SDD in EUDRAGIT L100-55 were administered at a target dose level of 10 mg API/kg. Blood samples (anticoagulant: $K_2$EDTA) were scheduled for collection from each animal prior to dosing and at 0.25, 0.5, 1, 2, 4, 6, 8, 12 and 24 hours after dosing in each dose session. Blood samples were processed for plasma and the plasma was analyzed for VS-6063 by LC-MS/MS. Plasma concentration-time data for individual animals were analyzed to determine the pharmacokinetic profiles of VS-6063.

4 groups of 1 male beagle dog per group received an oral gavage dose of VS-6063 in various formulations in a 4-session crossover design with a minimum 3-day washout period between dose sessions. In each session, VS-6063-25% physical mixture with Soluplus/PVP-A and VS-6063-25% SDD in Soluplus/PVP-A were administered at a

target dose level of 20 mg API/kg and VS-6063-50% physical mixture with Soluplus/PVP-A was administered at target dose levels of 10 and 20 mg API/kg. Blood samples (anticoagulant: $K_2EDTA$) were scheduled for collection from each animal prior to dosing and at 0.25, 0.5, 1, 2, 4, 6, 8, 12 and 24 hours after dosing in each dose session. Blood samples were processed for plasma and the plasma was analyzed for VS-6063 by LC-MS/MS. Plasma concentration-time data for individual animals were analyzed to determine the pharmacokinetic profiles of VS-6063.

**Dose Formulation**

VS-6063 was formulated (0.934 correction factor) at a target concentration of 10 mg API/mL in 0.5% (w/v) Methocel A4M in water with 0.1% (v/v) Tween 80. VS-6063-25% SDD in Soluplus/PVP-A was formulated (0.25 correction factor) at a target concentration of 10 mg API/mL in 0.5% (w/v) Methocel A4M in water. VS-6063-25% physical mixture with Soluplus/PVP-A was formulated (0.25 correction factor) at a target concentration of 10 mg API/mL in 0.5% (w/v) Methocel A4M in water. VS-6063-10% SDD in EUDRAGIT L100-55 was formulated (0.10 correction factor) at a target concentration of 10 mg API/mL in 0.5% (w/v) Methocel A4M in water.

VS-6063-25% physical mixture with Soluplus/PVP-A and VS-6063-25% SDD in Soluplus/PVP-A were formulated (0.25 correction factor) at a target concentration of 10 mg API/mL in 0.5% (w/v) Methocel A4M in water. VS-6063-50% Physical mixture with Soluplus/PVP-A was formulated (0.50 correction factor) at target concentrations of 5 mg API/mL and 10 mg API/mL in 0.5% (w/v) Methocel A4M in water.

**Dose Administration**

4 groups of 1 male beagle dog per group received an oral gavage dose of VS-6063 in various formulations in a 4-session crossover design with a minimum 3-day washout period between dose sessions. In each session, VS-6063, VS-6063-25% SDD in Soluplus/PVP-A, VS-6063-25% physical mixture with Soluplus/PVP-A and VS-6063-10% SDD in EUDRAGIT L100-55 were administered at a target dose level of 10 mg API/kg. Animals were fasted overnight prior to dose administration; water was not withheld. Food was returned at 4 hours post-dose.

Prior to dosing, the body weight of each animal was recorded. Doses (rounded to the nearest 0.01 mL) were calculated based on the pretreatment body weight (kg) and a dose volume of 1 mL/kg. Following the oral gavage dose, the gavage tube was flushed with 10 to 20 mL of water prior to removal of the tube. Dosing syringes were weighed immediately prior to and immediately after dosing each animal, and the quantity of formulation administered to each animal was determined from the difference in syringe weights.

4 groups of 1 male beagle dog per group received an oral gavage dose of VS-6063 in various formulations in a 4-session crossover design with a minimum 3-day washout period between dose sessions. In each session, VS-6063-25% physical mixture with Soluplus/PVP-A and VS-6063-25% SDD in Soluplus/PVP-A were administered at a target dose level of 20 mg API/kg and VS-6063-50% physical mixture with Soluplus/PVP-A was administered at target dose levels of 10 and 20 mg API/kg. Animals were fasted overnight prior to dose administration; water was not withheld. Food was returned at 4 hours post-dose.

Prior to dosing, the body weight of each animal was recorded. Doses (rounded to the nearest 0.01 mL) were calculated based on the pretreatment body weight (kg) and a dose volume of 2 mL/kg. Following the oral gavage dose, the gavage tube was flushed with 10 to 20 mL of water prior to removal of the tube. Dosing syringes were weighed immediately prior to and immediately after dosing each animal, and the quantity of formulation administered to each animal was determined from the difference in syringe weights.

**VS-6063 Concentrations in Plasma**

Mean (n=4) plasma concentration-time profiles are shown in **Figure 5** (rectilinear scale).

Following a single oral dose of VS-6063 at a target dose of 10 mg/kg over 4 sessions, plasma concentrations rose at a fairly rapid rate and maximum concentrations for individual animals were reached within 2 to 4 hours post-dose (mean 2.50 hours). Mean concentrations then declined in an apparent first-order manner. Concentrations of VS-6063 were quantifiable in all animals through the 24 hours post-dose (the final time point).

Following a single oral dose of VS-6063-25% SDD in Soluplus/PVP-A, VS-6063-25% physical mixture with Soluplus/PVP-A, and VS-6063-10% SDD in EUDRAGIT L100-55 at a target dose of 10 mg API/kg over 4 sessions, plasma concentrations rose at a fairly rapid rate and maximum concentrations for individual animals were reached within 0.5 to 4 hours post-dose (mean range of 1.75 to 1.88 hours). Mean concentrations then declined in an apparent first-order manner. Concentrations of VS-6063 were quantifiable in all animals through the 24 hours post-dose (the final time point).

## Plasma Pharmacokinetics for VS-6063

Plasma pharmacokinetic parameters for VS-6063 are shown in **Tables 13 and 14 .**

For VS-6063 at a target dose of 10 mg/kg over 4 sessions, the mean estimated half life was approximately 3.9 hours. Mean $C_{max}$ and mean $T_{max}$ were 614 ng/mL and 2.50 hours, respectively. Mean $AUC_{last}$ was 3100 ng*hr/mL and mean $AUC_{inf}$ was 3120 ng*hr/mL. (**Table 17**)

For VS-6063-25% SDD in Soluplus/PVP-A at a target dose of 10 mg API/kg over 4 sessions, the mean estimated half life was approximately 3.2 hours. Mean $C_{max}$ and mean $T_{max}$ were 1770 ng/mL and 1.75 hours, respectively. Mean $AUC_{last}$ was 8130 ng*hr/mL and mean $AUC_{inf}$ was 8170 ng*hr/mL. (**Table 18**)

For VS-6063-25% physical mixture with Soluplus/PVP-A at a target dose of 10 mg API/kg over 4 sessions, the mean estimated half life was approximately 3.7 hours. Mean $C_{max}$ and mean $T_{max}$ were 1460 ng/mL and 1.75 hours, respectively. Mean $AUC_{last}$ was 7320 ng*hr/mL and mean $AUC_{inf}$ was 7410 ng*hr/mL. (**Table 19**)

For VS-6063-10% SDD in EUDRAGIT L100-55 at a target dose of 10 mg API/kg over 4 sessions, the mean estimated half life was approximately 2.9 hours. Mean $C_{max}$ and mean $T_{max}$ were 1800 ng/mL and 1.88 hours, respectively. Mean $AUC_{last}$ was 8720 ng*hr/mL and mean $AUC_{inf}$ was 8740 ng*hr/mL. (**Table 20**)

Mean pharmacokinetic data suggests relative bioavailability was increased when comparing test formulations (VS-6063-25% SDD, VS-6063-25% physical mixture and VS-6063-10% SDD to the reference formulation (VS-6063), although variability among animals was significant. Mean relative bioavailability estimates were 2.97 (CV 72.7%) for VS-6063-25% physical mixture, 3.21 (CV 43.3%) for VS-6063-10% SDD, and 3.78 (CV 89.9%) for VS-6063-25% SDD. When comparing crossover data; Animal No. 4001 had the lowest exposure to the reference formulation (VS-6063) which contributed to overall variability among animals receiving the treatment (CV 52.8% for mean $AUC_{inf}$) as well as contributing to the highest relative bioavailability among test formulations.

Although emesis was observed in three of the four subjects dosed with VS-6063-25% physical mixture, mean relative bioavailability was similar to the other test formulations when compared to the reference formulation.

## VS-6063 Concentrations in Plasma

Mean plasma concentration-time profiles are shown in **Figure 6** (rectilinear scale).

Following a single oral dose of VS-6063-25% physical mix at a target dose of 20 mg API/kg over 4 sessions, plasma concentrations rose at a rapid rate and maximum concentrations were reached within 0.25 to 1 hour post-dose (mean 0.688 hr). Mean concentrations then declined in an apparent first-order manner. Concentrations of VS-6063 were quantifiable in all animals through the 24 hours post-dose (the final time point).

Following a single oral dose of VS-6063-25% SDD at a target dose of 20 mg API/kg over 4 sessions, plasma concentrations rose at a fairly rapid rate and maximum concentrations were reached within 0.25 to 2 hours post-dose (mean 1.31 hr). Mean concentrations then declined in an apparent first-order manner. Concentrations of VS-6063 were quantifiable in all animals through the 24 hours post-dose (the final time point).

Following a single oral dose of VS-6063-50% physical mixture at target doses of 10 mg API/kg and 20 mg API/kg over 4 sessions, plasma concentrations rose at a fairly rapid rate and maximum concentrations were reached within 0.5 to 2 hours post-dose (mean 1.38 hr) for the 10 mg API/kg dose and within 0.5 to 4 hours post-dose (mean 2.13 hr) for the 20 mg API/kg dose. Mean concentrations then declined in an apparent first-order manner. Concentrations of VS-6063 were quantifiable in all animals through the 24 hours post-dose (the final time point).

## Plasma Pharmacokinetics for VS-6063

Plasma pharmacokinetic parameters for VS-6063 are shown in **Tables 15 and 16** .

For VS-6063-25% physical mixture with Soluplus/PVP-A at a target dose of 20 mg API/kg over 4 sessions, the mean estimated half life was approximately 3.0 hours. Mean $C_{max}$ and mean $T_{max}$ were 1670 ng/mL and 0.688 hours, respectively. Mean $AUC_{last}$ was 8670 ng*hr/mL and mean $AUC_{inf}$ was 8700 ng*hr/mL. (**Table 21**)

For VS-6063-25% SDD in Soluplus/PVP-A at a target dose of 20 mg API/kg over 4 sessions, the mean estimated half life was approximately 3.1 hours. Mean $C_{max}$ and mean $T_{max}$ were 1300 ng/mL and 1.31 hours, respectively. Mean $AUC_{last}$ was 5920 ng*hr/mL and mean $AUC_{inf}$ was 5940 ng*hr/mL. (**Table 22**)

For VS-6063-50% physical mixture with Soluplus/PVP-A at a target dose of 10 mg API/kg over 4 sessions, the mean estimated half life was approximately 3.0 hours. Mean $C_{max}$ and mean $T_{max}$ were 680 ng/mL and 1.38 hours, respectively. Mean $AUC_{last}$ was 3390 ng*hr/mL and mean $AUC_{inf}$ was 3400 ng*hr/mL. (**Table 23**)

For VS-6063-50% physical mixture with Soluplus/PVP-A at a target dose of 20 mg API/kg over 4 sessions, the mean estimated half life was approximately 3.5 hours. Mean $C_{max}$ and mean $T_{max}$ were 1160 ng/mL and 2.13 hours, respectively. Mean $AUC_{last}$ was 6900 ng*hr/mL and mean $AUC_{inf}$ was 6930 ng*hr/mL. (**Table 24**)

Observations of emesis corresponded with lower exposure while in other instances there was no correlation.

Pharmacokinetic data suggests that VS-6063-50% physical mixture at target doses of 10 mg API/kg and 20 mg API/kg are dose proportional: mean dose-normalized $AUC_{inf}$ and $C_{max}$ may be similar.

Comparing data from both studies, VS-6063-25% physical mixture (20 mg API/kg) and VS-6063-25% SDD (20 mg API/kg) were less than dose proportional relative to dose-normalized mean $AUC_{inf}$ and $C_{max}$ with VS-6063-25% physical mixture (10 mg API/kg) and VS-6063-25% SDD (10 mg API/kg) as summarized below in **Table 11**.

**Table 11.**

| Formulation | Dose Level mg API/kg | Mean $C_{max}$/Dose | Mean $AUC_{inf}$/Dose |
|---|---|---|---|
| 25% Physical Mixture | 20 | 83.5 | 440 |
| 25% Physical Mixture | 10 | 146 | 741 |
| 25% SDD | 20 | 65.0 | 297 |
| 25% SDD | 10 | 177 | 817 |

Mean pharmacokinetic data suggests relative bioavailability is similar when comparing test formulations VS-6063-50% physical mixture at target doses of 10 mg API/kg and 20 mg API/kg to reference formulation VS-6063 (10 mg/kg).

**Table 12**.

| Formulation | Dose Level mg API/kg | Mean $AUC_{inf}$/Dose | Relative Bioavailability |
|---|---|---|---|
| VS-6063 (neat) | 10 | 312 | NA |
| 50% Physical Mixture | 10 | 340 | 1.09 |
| 50% Physical Mixture | 20 | 347 | 1.11 |

**Conclusions**

Mean pharmacokinetic data suggests relative bioavailability may be increased when comparing VS-6063-25% SDD in Soluplus (10 mg API/kg), VS-6063-25% physical mixture with Soluplus (10 mg API/kg), and VS-6063-10% SDD in EUDRAGIT L100-55 (10 mg API/kg) to VS-6063 (10 mg/kg), although there was variability among animals. Mean relative bioavailability estimates were 2.97 (CV 72.7%) for VS-6063-25% physical mixture, 3.21 (CV 43.3%) for VS-6063-10% SDD, and 3.78 (CV 89.9%) for VS-6063-25% SDD.

Mean pharmacokinetic data suggests that VS-6063-50% physical mixture with Soluplus/PVP-A at target doses of 10 mg API/kg and 20 mg API/kg are dose proportional. Mean dose-normalized mean $AUC_{inf}$ and mean $C_{max}$ may be generally similar.

Comparing data from both studies, VS-6063-25% physical mixture (20 mg API/kg) and VS-6063-25% SDD (20 mg API/kg) are less than dose proportional when comparing dose-normalized mean $AUC_{inf}$ and mean $C_{max}$ with VS-6063-25% physical mixture (10 mg API/kg) and VS-6063-25% SDD (10 mg API/kg). Dose normalized mean pharmacokinetic data suggests relative bioavailability may be similar when comparing VS-6063-50% physical mixture at target doses of 10 mg API/kg and 20 mg API/kg to VS-6063 (10 mg/kg).

**Table 13: Oral (Gavage) Dose Administration of VS-6063 in Various Formulations to Male Beagle Dogs**

| Session No. | Test Article Formulation | Group No. | Animal No. | Animal Weight (kg) | Formulation Admin (g) | Conc[a] (mg/g) | Dose Administered (mg) | Dose Administered (mg/kg) | Protocol Dose (mg/kg) | Dosing Variance (%) |
|---|---|---|---|---|---|---|---|---|---|---|
| 1 | VS-6063 | 1 | 1001 | 8.80 | 7.986 | 10 | 79.86 | 9.075 | 10 | -9.25 |
| | VS-6063-25% SDD in Soluplus/PVP-A | 2 | 2001 | 10.74 | 10.577 | 10 | 105.8 | 9.848 | 10 | -1.52 |
| | VS-6063-25% Phys Mix with Soluplus/PVP-A | 3 | 3001 | 10.34 | 10.280 | 10 | 102.8 | 9.942 | 10 | -0.58 |
| | VS-6063-10% SDD in EUDRAGIT L100-55 | 4 | 4001 | 9.14 | 8.688 | 10 | 86.88 | 9.505 | 10 | -4.95 |
| 2 | VS-6063-10%SDD in EUDRAGIT L100-55 | 1 | 1001 | 9.22 | 8.872 | 10 | 88.72 | 9.623 | 10 | -3.77 |
| | VS-6063 | 2 | 2001 | 10.46 | 9.794 | 10 | 97.94 | 9.363 | 10 | -6.37 |
| | VS-6063-25% SDD in Soluplus/PVP-A | 3 | 3001 | 11.10 | 10.951 | 10 | 109.51 | 9.866 | 10 | -1.34 |
| | VS-6063-25% Phys Mix with Soluplus/PVP-A | 4 | 4001 | 9.62 | 9.516 | 10 | 95.16 | 9.892 | 10 | -1.08 |
| 3 | VS-6063-25% Phys Mix with Soluplus/PVP-A | 1 | 1001 | 8.50 | 8.459 | 10 | 84.59 | 9.952 | 10 | -0.48 |
| | VS-6063-10% SDD in EUDRAGIT L100-55 | 2 | 2001 | 10.58 | 9.950 | 10 | 99.50 | 9.405 | 10 | -5.95 |
| | VS-6063 | 3 | 3001 | 10.48 | 10.462 | 10 | 104.6 | 9.983 | 10 | -0.17 |
| | VS-6063-25% SDD in Soluplus/PVP-A | 4 | 4001 | 9.38 | 9.280 | 10 | 92.80 | 9.893 | 10 | -1.07 |
| 4 | VS-6063-25%SDD in Soluplus/PVP-A | 1 | 1001 | 8.820 | 8.800 | 10 | 88.00 | 9.977 | 10 | -0.23 |
| | VS-6063-25% Phys Mix with Soluplus/PVP-A | 2 | 2001 | 10.800 | 10.692 | 10 | 106.9 | 9.900 | 10 | -1.00 |
| | VS-6063-10% SDD in EUDRAGIT L100-55 | 3 | 3001 | 10.580 | 10.390 | 10 | 103.9 | 9.820 | 10 | -1.80 |
| | VS-6063 | 4 | 4001 | 9.380 | 8.864 | 10 | 88.64 | 9.450 | 10 | -5.50 |

Assumes a density of 1 g/mL.

**Table 14. Mean (n=4) Plasma Pharmacokinetic Parameters for VS-6063 Following an Oral Gavage Dose of VS-6063 in Various Formulations to Male Beagle Dogs at 10 mg/kg**

| Formulation | | $C_{max}$ (ng/mL) | $T_{max}$ (hr) | $t_{1/2}$ (hr) | $AUC_{inf}$ (hr*ng/m L) | $AUC_{last}$ (hr*ng/ m L) | Relative Bioavail ability |
|---|---|---|---|---|---|---|---|
| VS-6063 | Mean | 614 | 2.50 | 3.86 | 3120 | 3100 | NA |
| | SD | 302 | 1.00 | 0.872 | 1650 | 1640 | NA |
| | % CV | 49.2 | 40.0 | 22.6 | 52.8 | 53.0 | NA |
| VS-6063-25% SDD in Soluplus/PVP-A | Mean | 1770 | 1.75 | 3.24 | 8170 | 8130 | 3.78 |
| | SD | 791 | 1.66 | 0.501 | 2490 | 2480 | 3.40 |
| | % CV | 44.8 | 94.8 | 15.5 | 30.5 | 30.5 | 89.9 |
| VS-6063-25% Phys Mix With Soluplus/PVP-A | Mean | 1460 | 1.75 | 3.71 | 7410 | 7320 | 2.97 |
| | SD | 520 | 1.66 | 0.551 | 3400 | 3310 | 2.16 |
| | % CV | 35.7 | 94.8 | 14.9 | 45.8 | 45.2 | 72.7 |
| VS-6063-10% SDD in EUDRAGIT L100-55 | Mean | 1800 | 1.88 | 2.93 | 8740 | 8720 | 3.21 |
| | SD | 495 | 1.55 | 0.351 | 3560 | 3540 | 1.39 |
| | % CV | 27.6 | 82.6 | 12.0 | 40.7 | 40.7 | 43.3 |
| Relative bioavailability =$AUC_{inf}$ test formulation/$AUC_{inf}$ reference formulation | | | | | | | |

**Table 15. Oral (Gavage) Dose Administration of VS-6063 in Various Formulations to Male Beagle Dogs**

| Session No. | Test Article Formulation | Group No. | Animal No. | Animal Weight (kg) | Formulation Admin (g) | Conc[a] (mg/g) | Dose Administered (mg) | Dose Administered (mg/kg) | Protocol Dose (mg/kg) | Dosing Variance (%) |
|---|---|---|---|---|---|---|---|---|---|---|
| 1 | VS-6063-25% Phys Mix with Soluplus/PVP-A | 1 | 1001 | 9.76 | 19.44 | 10 | 194.35 | 19.91 | 20 | -0.44 |
| | VS-6063-25% SDD in Soluplus/PVP-A | 2 | 2001 | 9.40 | 18.64 | 10 | 186.38 | 19.83 | 20 | -0.86 |
| | VS-6063-50% Phys Mix with Soluplus/PVP-A | 3 | 3001 | 8.64 | 17.01 | 5 | 85.03 | 9.84 | 10 | -1.59 |
| | VS-6063-50% Phys Mix with Soluplus/PVP-A | 4 | 4001 | 9.68 | 19.26 | 10 | 192.58 | 19.89 | 20 | -0.53 |
| 2 | VS-6063-50% Phys Mix with Soluplus/PVP-A | 1 | 1001 | 10.08 | 19.98 | 10 | 199.75 | 19.82 | 20 | -0.92 |
| | VS-6063-25% Phys Mix with Soluplus/PVP-A | 2 | 2001 | 9.08 | 18.33 | 10 | 183.25 | 20.18 | 20 | 0.91 |
| | VS-6063-25% SDD in Soluplus/PVP-A | 3 | 3001 | 9.00 | 17.95 | 10 | 179.52 | 19.95 | 20 | -0.27 |
| | VS-6063-50% Phys Mix with Soluplus/PVP-A | 4 | 4001 | 9.90 | 19.49 | 5 | 97.44 | 9.84 | 10 | -1.58 |
| 3 | VS-6063-50% Phys Mix with Soluplus/PVP-A | 1 | 1001 | 9.94 | 19.45 | 5 | 97.24 | 9.78 | 10 | -2.18 |
| | VS-6063-50% Phys Mix with Soluplus/PVP-A | 2 | 2001 | 8.68 | 17.08 | 10 | 170.83 | 19.68 | 20 | -1.60 |
| | VS-6063-25% PHYS mix with Soluplus/PVP-A | 3 | 3001 | 8.40 | 16.61 | 10 | 166.10 | 19.77 | 20 | -1.13 |
| | VS-6063-25% SDD in Soluplus/PVP-A | 4 | 4001 | 9.80 | 18.96 | 10 | 189.62 | 19.35 | 20 | -3.26 |
| 4 | VS-6063-25% SDD in Soluplus/PVP-A | 1 | 1001 | 10.50 | 20.45 | 10 | 204.49 | 19.48 | 20 | -2.62 |
| | VS-6063-50% Phys Mix with Soluplus/PVP-A | 2 | 2001 | 9.00 | 17.70 | 5 | 88.48 | 9.83 | 10 | -1.69 |

(continued)

| Session No. | Test Article Formulation | Group No. | Animal No. | Animal Weight (kg) | Formulation Admin (g) | Conc[a] (mg/g) | Dose Administered (mg) | Dose Administered (mg/kg) | Protocol Dose (mg/kg) | Dosing Variance (%) |
|---|---|---|---|---|---|---|---|---|---|---|
| | VS-6063-50% Phys Mix with Soluplus/PVP-A | 3 | 3001 | 8.92 | 17.65 | 10 | 176.47 | 19.78 | 20 | -1.08 |
| | VS-6063-25% Phys Mix with Soluplus/PVP-A | 4 | 4001 | 10.40 | 20.61 | 10 | 206.05 | 19.81 | 20 | -0.94 |

[a] Assumes a density of 1 g/mL.

**Table 16 Mean (n=4) Plasma Pharmacokinetic Parameters for VS-6063 Following an Oral Gavage Dose of VS-6063 in Various Formulations to Male Beagle Dogs at 10 or 20 mg API/kg**

| Formulation Dose | | $C_{max}$ (ng/mL ) | $T_{max}$ (hr) | $t_{1/2}$ (hr) | $AUC_{inf}$ (hr*ng/mL) | $AUC_{last}$ (hr*ng/mL) | $C_{max}$/Dose | $AUC_{inf}$/Dose |
|---|---|---|---|---|---|---|---|---|
| VS-6063-25% Phys Mix with Soluplus/PVP-A 20 mg API/kg | Mean | 1670 | 0.688 | 3.01 | 8700 | 8670 | 83.5 | 440 |
| | SD | 580 | 0.375 | 0.156 | 3400 | 3390 | 29.0 | 170 |
| | % CV | 34.7 | 54.5 | 5.2 | 39.1 | 39.1 | 34.7 | 39.1 |
| VS-6063-25% SDD in Soluplus/PVP-A 20 mg API/kg | Mean | 1300 | 1.31 | 3.12 | 5940 | 5920 | 64.9 | 297 |
| | SD | 672 | 0.851 | 0.289 | 1640 | 1640 | 33.6 | 82.1 |
| | % CV | 51.8 | 64.8 | 9.3 | 27.6 | 27.7 | 51.8 | 27.6 |
| VS-6063-50% Phys Mix with Soluplus/PVP-A 10 mg API/kg | Mean | 680 | 1.38 | 3.00 | 3400 | 3390 | 68.0 | 340 |
| | SD | 190 | 0.750 | 0.0208 | 1150 | 1150 | 19.0 | 115 |
| | % CV | 28.0 | 54.5 | 0.700 | 33.9 | 33.8 | 28.0 | 33.9 |
| VS-6063-50% Phys Mix with Soluplus/PVP-A 20 mg API/kg | Mean | 1160 | 2.13 | 3.51 | 6930 | 6900 | 57.9 | 347 |
| | SD | 516 | 1.44 | 0.813 | 3500 | 3500 | 25.8 | 175 |
| | % CV | 44.6 | 67.6 | 23.1 | 50.5 | 50.8 | 44.6 | 50.5 |

**Table 17 Plasma Pharmacokinetic Parameters for VS-6063 Following an Oral Gavage Dose of VS-6063 to Male Beagle Dogs at 10 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) |
|---|---|---|---|---|---|---|
| 1 | 1001 | 537 | 2 | 3.49 | 2830 | 2820 |
| 2 | 2001 | 987 | 4 | 3.42 | 5200 | 5180 |
| 3 | 3001 | 671 | 2 | 3.39 | 3240 | 3230 |
| 4 | 4001 | 260 | 2 | 5.17 | 1200 | 1180 |
| | Mean | 614 | 2.50 | 3.86 | 3120 | 3100 |
| | SD | 302 | 1.00 | 0.872 | 1650 | 1640 |
| | % CV | 49.2 | 40.0 | 22.6 | 52.8 | 53.0 |

**Table 18 Plasma Pharmacokinetic Parameters for VS-6063 Following an Oral Gavage Dose of VS-6063-25% SDD in Soluplus/PVP-A to Male Beagle Dogs at 10 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL ) | Relative Bioavailability |
|---|---|---|---|---|---|---|---|
| 4 | 1001 | 2390 | 0.5 | 3.58 | 9880 | 9840 | 3.49 |
| 1 | 2001 | 1280 | 4 | 2.58 | 7460 | 7450 | 1.43 |
| 2 | 3001 | 912 | 2 | 3.12 | 4950 | 4930 | 1.53 |
| 3 | 4001 | 2490 | 0.5 | 3.67 | 10400 | 10300 | 8.68 |
| | Mean | 1770 | 1.75 | 3.24 | 8170 | 8130 | 3.78 |
| | SD | 791 | 1.66 | 0.501 | 2490 | 2480 | 3.40 |
| | % CV | 44.8 | 94.8 | 15.5 | 30.5 | 30.5 | 89.9 |

**Table 19 Plasma Pharmacokinetic Parameters for VS-6063 Following an Oral Gavage Dose of VS-6063-25% Physical Mixture With Soluplus/PVP-A to Male Beagle Dogs at 10 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | Relative Bioavailabilit y |
|---|---|---|---|---|---|---|---|
| 3 | 1001[a] | 1810 | 0.5 | 3.26 | 6420 | 6350 | 2.27 |
| 4 | 2001 | 1750 | 4 | 4.40 | 12000 | 11800 | 2.31 |
| 1 | 3001 | 691 | 2 | 3.92 | 3890 | 3850 | 1.20 |
| 2 | 4001 | 1580 | 0.5 | 3.27 | 7320 | 7300 | 6.12 |
| | Mean | 1460 | 1.75 | 3.71 | 7410 | 7320 | 2.97 |
| | SD | 520 | 1.66 | 0.551 | 3400 | 3310 | 2.16 |
| | % CV | 35.7 | 94.8 | 14.9 | 45.8 | 45.2 | 72.7 |

[a] Regression R square value <0.9

**Table 20 Plasma Pharmacokinetic Parameters for VS-6063 Following an Oral Gavage Dose of VS-6063-10% SDD in EUDRAGIT L100-55 to Male Beagle Dogs at 10 mg API/kg**

| Session | Animal Number | $C_{max}$ (ng/mL) | $T_{max}$ (hr) | $t_{1/2}$ (hr) | $AUC_{inf}$ (hr*ng/mL ) | $AUC_{last}$ (hr*ng/mL ) | Relative Bioavailabilit y |
|---|---|---|---|---|---|---|---|
| 2 | 1001 | 2180 | 4 | 2.77 | 11400 | 11400 | 4.03 |
| 3 | 2001 | 2200 | 1 | 3.33 | 12200 | 12100 | 2.35 |
| 4 | 3001 | 1160 | 2 | 3.10 | 5700 | 5680 | 1.76 |
| 1 | 4001 | 1650 | 0.5 | 2.53 | 5640 | 5640 | 4.72 |
| | Mean | 1800 | 1.88 | 2.93 | 8740 | 8720 | 3.21 |
| | SD | 495 | 1.55 | 0.351 | 3560 | 3540 | 1.39 |
| | % CV | 27.6 | 82.6 | 12.0 | 40.7 | 40.7 | 43.3 |

**Table 21 Plasma Pharmacokinetic Parameters for VS-6063 Following an Oral Gavage Dose of VS-6063-25% Physical Mixture With Soluplus/PVP-A to Male Beagle Dogs at 20 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | $C_{max}$/ Dose | $AUC_{inf}$/Dos e |
|---|---|---|---|---|---|---|---|---|
| 1 | 1001 | 952 | 1 | 3.15 | 4770 | 4750 | 47.6 | 240 |
| 2 | 2001 | 1570 | 1 | 3.13 | 7200 | 7180 | 78.5 | 360 |
| 3 | 3001 | 1810 | 0.5 | 2.87 | 10300 | 10300 | 90.5 | 520 |
| 4 | 4001 | 2350 | 0.25 | 2.87 | 12500 | 12500 | 120 | 630 |
| | Mean | 1670 | 0.688 | 3.01 | 8700 | 8670 | 83.5 | 440 |
| | SD | 580 | 0.375 | 0.156 | 3400 | 3390 | 29.0 | 170 |
| | % CV | 34.7 | 54.5 | 5.2 | 39.1 | 39.1 | 34.7 | 39.1 |

**Table 22 Plasma Pharmacokinetic Parameters for VS-6063 Following an Oral Gavage Dose of VS-6063-25% SDD in Soluplus/PVP-A to Male Beagle Dogs at 20 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | $C_{max}$/ Dose | $AUC_{inf}$/Dos e |
|---|---|---|---|---|---|---|---|---|
| 4 | 1001 | 2240 | 0.25 | 2.73 | 7870 | 7860 | 112 | 394 |
| 1 | 2001 | 650 | 2 | 3.15 | 3930 | 3920 | 32.5 | 197 |
| 2 | 3001 | 1200 | 2 | 3.43 | 6380 | 6350 | 60.0 | 319 |
| 3 | 4001 | 1100 | 1 | 3.18 | 5580 | 5560 | 55.0 | 279 |

(continued)

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | $C_{max}$/ Dose | $AUC_{inf}$/Dose |
|---|---|---|---|---|---|---|---|---|
| | Mean | 1300 | 1.31 | 3.12 | 5940 | 5920 | 64.9 | 297 |
| | SD | 672 | 0.851 | 0.289 | 1640 | 1640 | 33.6 | 82.1 |
| | % cv | 51.8 | 64.8 | 9.3 | 27.6 | 27.7 | 51.8 | 27.6 |

**Table 23 Plasma Pharmacokinetic Parameters for VS-6063 Following an Oral Gavage Dose of VS-6063-50% Physical Mixture With Soluplus/PVP-A to Male Beagle Dogs at 10 mg API/kg**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | $C_{max}$/ Dose | $AUC_{inf}$/Dose |
|---|---|---|---|---|---|---|---|---|
| 3 | 1001 | 809 | 2 | 3.03 | 3970 | 3960 | 80.9 | 397 |
| 4 | 2001 | 398 | 0.5 | 3.01 | 1820 | 1810 | 39.8 | 181 |
| 1 | 3001 | 733 | 2 | 2.98 | 3350 | 3340 | 73.3 | 335 |
| 2 | 4001 | 778 | 1 | 3.00 | 4470 | 4450 | 77.8 | 447 |
| | Mean | 680 | 1.38 | 3.00 | 3400 | 3390 | 68.0 | 340 |
| | SD | 190 | 0.750 | 0.0208 | 1150 | 1150 | 19.0 | 115 |
| | % CV | 28.0 | 54.5 | 0.700 | 33.9 | 33.8 | 28.0 | 33.9 |

**Table 24 Plasma Pharmacokinetic Parameters for VS-6063 Following an Oral Gavage Dose of VS-6063-50% Physical Mixture With Soluplus/PVP-A to Male Beagle Dogs at 20 mg API/k**

| Session | Animal Number | $C_{max}$ (ng/mL) | $T_{max}$ (hr) | $t_{1/2}$ (hr) | $AUC_{inf}$ (hr*ng/mL) | $AUC_{last}$ (hr*ng/mL) | $C_{max}$/ Dose | $AUC_{inf}$/Dose |
|---|---|---|---|---|---|---|---|---|
| 2 | 1001 | 1210 | 0.5 | 3.32 | 6250 | 6230 | 60.5 | 312 |
| 3 | 2001 | 1710 | 2 | 2.88 | 11000 | 10900 | 85.5 | 549 |
| 4 | 3001 | 1250 | 2 | 3.14 | 7930 | 7900 | 62.5 | 396 |
| 1 | 4001 | 462 | 4 | 4.70 | 2570 | 2530 | 23.1 | 129 |
| | Mean | 1160 | 2.13 | 3.51 | 6930 | 6900 | 57.9 | 347 |
| | SD | 516 | 1.44 | 0.813 | 3500 | 3500 | 25.8 | 175 |
| | % CV | 44.6 | 67.600 | 23.100 | 50.5 | 50.8 | 44.6 | 50.5 |

**Example 5. Clinical Study Protocol: Phase I Study of Safety, Bioavailability, and Pharmacodynamics of Selected Formulations of VS-6063**

*Study Design*

In a Phase I, open-label, single center, single dose, partially randomized trial, the safety, bioavailability, and pharmacodynamics of prototype and reference formulations of VS-6063 were investigated in healthy male subjects.The study was comprised of four periods, each of which adhered to the same study design and involved 24 healthy male subjects between 40 and 65 years of age with a body mass index between 18.0 and 35.0 kg/$m^2$. Following a 28 day screening period, the subjects were admitted to the clinical unit the morning prior to dosing day (Day -1) and were dosed in the morning of Day 1. Subjects in Regimens A and B were administered VS-6063 following an overnight fast of about 10 hours, while subjects in Regimens C and D were administered VS-6063 within 30 minutes of starting a high fat breakfast, which was consumed within 25 minutes. Subjects remained on site until 36 hours after dosing. There was a minimum washout period of 7 days between the administration of each regimen. A follow-up phone call took place 7 to 10 days post final dose in periods 3 and 4 to ensure the ongoing well-being of the subjects.

**Table 25. Summary of pharmacokinetic results**

| | Regimen A | Regimen B | Regimen C | Regimen D |
|---|---|---|---|---|
| **Formulation**<br>**Dose**<br>**Fasted/Fed**<br>**Parameter** | **Reference**<br>**200 mg**<br>**Fasted**<br>**N=24** | **Prototype**<br>**100 mg**<br>**Fasted**<br>**N=24** | **Prototype**<br>**100 mg**<br>**Fed**<br>**N = 23** | **Reference**<br>**200 mg**<br>**Fed**<br>**N=9** |
| $T_{lag}{}^a$ (h) | 0.000 (0.00-0.00) | 0.042 (0.00-0.50) | 0.000 (0.00-0.52) | 0.000 (0.00-1.00) |
| $T_{max}{}^a$ (h) | 1.000 (0.50-3.00) | 2.000 (1.00-4.00) | 4.000 (2.00-10.02) | 4.000 (2.00-6.00) |
| $C_{max}$ (ng/mL) | 212 (72.8%) | 446 (32.3%) | 262 (43.5%) | 427 (66.7%) |
| $C_{12}$ (ng/mL) | 6.1 (78.0%) | 15.1 (88.8%) | 22.7 (80.6%) | 38.5 (174.0%) |
| $C_{24}$ (ng/mL) | 3.30 (59.9%) [n = 23] | 3.72 (63.4%) | 4.45 (74.2%) | 4.52 (93.2%) |
| $AUC_{last}$ (ng.h/mL) | 883 (69.2%) | 1910 (36.6%) | 1590 (44.6%) | 2490 (75.8%) |
| $AUC_{inf}$ (ng.h/mL) | 755 (46.0%) [n = 2] | 2350 (29.2%) [n = 11] | 1790 (37.0%) [n = 12] | 2970 (75.4%) [n = 7] |
| $AUC_{\%extrap}$ (%) | 1.581 (155.7%) [n = 2] | 1.028 (41.8%) [n = 11] | 1.232 (46.1%) [n = 12] | 0.574 (69.3%) [n = 7] |
| lambda-z | 0.16125 (214.7) [n = 2] | 0.10403 (31.4%) [n = 11] | 0.09156 (21.4%) [n = 12] | 0.13372 (40.1%) [n = 7] |
| $\alpha$ $t_{1/2}$ (h) (Distributional Component) | 1.880 (18.9%) | 1.989 (20.1%) | 2.082 (21.4%) | 2.154 (15.9%) |
| $t_{1/2}$ (h) | 4.299 (214.7%) [n = 2] | 6.663 (31.4%) [n = 11] | 7.571 (21.4%) [n = 12] | 5.183 (40.1%) [n = 7] |
| [a] Median (range) | | | | |

**Abbreviations:** $T_{lag}{}^a$: the elapsed time from dosing at which VS-6063 was first quantifiable in a concentration vs time profile; $C_{max}$: the maximum observed VS-6063 concentration; $T_{max}{}^a$: the time from dosing at which $C_{max}$ was present; $C_{12}$: the observed VS-6063 concentration at 12 h; $C_{24}$: the observed VS-6063 concentration at 24 h; $AUC_{last}$: the area under the concentration vs time curve from time zero to the time of the last quantifiable concentration; $AUC_{inf}$: the area under the concentration vs time curve from time zero extrapolated to infinity; $AUC_{\%extrap}$: the percentage of $AUC_{(0-inf)}$ accounted for by extrapolation; lambda-z: slope of the regression time passing through the apparent elimination phase in a concentration vs time plot; $t_{1/2}$: the apparent elimination half-life; $\alpha$ $t_{1/2}$: the distributional half-life of VS-6063 occurring over the time range between $C_{max}$ and approximately 12 h post-dose.

*Statistical Methods*

Formal statistical analysis was performed on the dose corrected pharmacokinetic (PK) parameters $AUC_{last}$, $AUC_{inf}$, and $C_{max}$ to assess relative bioavailability and the presence of food effects. The PK parameters were subjected to a natural logarithmic transformation and were analyzed using mixed model techniques. Adjusted geometric mean rations (GMRs) and 90% confidence intervals (CIs) for the adjusted GMRs were calculated for the comparisons of the different regimens. The CIs were based on the t-distribution and the variances estimated by the model using the Kenward-Roger method for calculating the degrees of freedom.

*Study Results*

Following administration of either a 200 mg VS-6063 reference formulation tablet (comprising VS-6063, microcrystalline cellulose PH 102, Fastflo 316, sodium starch glycolate, and magnesium stearate) or a 100 mg VS-6063 prototype formulation tablet (comprising VS-6063, HPMCAS-HF, microcrystalline cellulose PH 102, Fastflo 316, sodium starch glycolate, and magnesium stearate) in the fed and fasted states, plasma concentrations rose to a maximal level

immediately following dosing. This was followed by a biphasic reduction in plasma levels over the 24 h sampling period, as VS-6063 was distributed and eliminated from the systemic circulation. No real difference in half-life could be determined between the reference and prototype formulations in either the fed or fasted states.

In the fasted state, the 100 mg prototype formulation administered in Regimen B lead to significantly higher exposure in comparison to the 200 mg reference formulation in Regimen A. The adjusted GMRs and 90% CIs for Regimen B/Regimen A based on $C_{max}$ and $AUC_{last}$ were 421.33 (348.4%, 509.5%) and 433.05 (366.8%, 511.3%), respectively, equating to a 4-fold increase in exposure as a result of the change in formulation. In addition to the increase in bioavailability, there was evidence of a decrease in variability observed upon administration of the prototype formulation associated with exposure parameters from approximately 70% to 30%.

Comparison of the time taken to reach maximum plasma concentrations of VS-6063 was 2 to 4 times longer in the fed state than in the fasted state (as assessed by $C_{max}$ and $AUC_{last}$), indicating a food effect on the rate of absorption. In addition, determination of exposure following administration of the 200 mg VS-6063 reference formulation in both the fed and fasted state indicate a higher level of exposure in the fed state relative to the fasted state, which signifies the presence of a food effect for the reference formulation. Despite these effects, an increase in VS-6063 exposure was still observed when the prototype formulation was administered compared with the reference formulation in the fed state. The adjusted GMRs and 90% CIs for Regimen C/Regimen D based on $C_{max}$ and $AUC_{last}$ were 129.8 and 125.1, respectively.

Overall, the maximum exposure was achieved with the prototype formulation administered in the fasted state. Equivalent exposure in terms of $AUC_{inf}$ was also achieved with the prototype formulation in the fed state.

VS-6063 was well tolerated when administered in both the fed and fasted states in both the reference and prototype formulations. There were no serious adverse events (AEs) or severe AEs reported during the study, and no subject was withdrawn as a result of an AE. The overall incidence of total AEs was low, with 12 AEs reported by 9 subjects. The incidence of AEs was highest for Regimen C (5 subjects) compared to Regimens A, B, and D (3 subjects, 2 subjects, and 1 subject, respectively). The most common AE was headache, which was reported by 3 subjects. All other AEs were reported by 1 subject. No subject reported an AE that was related to VS-6063, and nearly all AEs were mild in severity. There were no clinically significant findings in any laboratory assessments, measurement of vital signs, ECGs, or physical examinations.

## Claims

1. A pharmaceutical composition comprising VS-6063, or a pharmaceutically acceptable salt thereof, admixed with a polymer excipient, wherein the polymer excipient is hydroxypropylmethyl cellulose acetate succinate (HPMCAS), wherein the pharmaceutical composition is configured in an oral dosage form in the form of a tablet, wherein the tablet is provided by direct compression or dry granulation of a physical blend or mixture of VS-6063, or a pharmaceutically acceptable salt thereof, admixed with the polymer excipient.

2. The pharmaceutical composition of claim 1, wherein the composition comprises VS-6063 free base or VS-6063 hydrochloride.

3. The pharmaceutical composition of claim 1 or 2, wherein:

   a) the ratio by weight of VS-6063, or a pharmaceutically acceptable salt thereof, to the polymer is about 1 to 1, 1 to 2, 1 to 3, or 1 to 4; or
   b) VS-6063, or a pharmaceutically acceptable salt thereof, is present in the composition in an amount that is about 10, 25, 30, 35, 40, 45, 50% or more w/w relative to an amount of polymer provided; or
   c) the composition comprises 5 to 50%, 5 to 30%, 10 to 30%, 10 to 20%, 12 to 15%, or 13% weight of VS-6063, or a pharmaceutically acceptable salt thereof, per weight of the composition.

4. The pharmaceutical composition of claim 1, wherein:

   a) the oral dosage form comprises 5 to 50%, 5 to 30%, 10 to 30%, 10 to 20%, 12 to 15%, or 13% weight of VS-6063, or a pharmaceutically acceptable salt thereof, per weight of the oral dosage form; or
   b) VS-6063, or a pharmaceutically acceptable salt thereof, is present at 13% per weight of the oral dosage form; or
   c) the polymer is present at 25 to 55%, 35 to 45%, 38 to 43%, or 40% per weight of the oral dosage form.

5. The pharmaceutical composition of claim 1, further comprising:

a) one or more fillers, for example microcrystalline cellulose, optionally wherein the microcrystalline cellulose is microcrystalline cellulose PH 102; or lactose, optionally wherein the lactose is lactose monohydrate; optionally wherein:

i) the filler is present at 5 to 80%, 10 to 70%, 20 to 60%, 30 to 60%, 30 to 50%, or 42% per weight of the oral dosage form; or
ii) the filler is a mixture of two fillers; optionally wherein:

I) one filler is microcrystalline cellulose PH 102, optionally present at 10 to 30%, 15 to 25%, 20% per weight of the oral dosage form, and/or one filler is lactose monohydrate, optionally present at 10 to 30%, 15 to 25%, or 20% per weight of the oral dosage form; or
II) the fillers are present at a 1:1 w/w ratio to one another; or

b) a disintegrant, optionally wherein the disintegrant is modified starch; optionally wherein:

i) the disintegrant is sodium starch glycolate; or
ii) the disintegrant is present at 0 to 5%, 1 to 5%, 2.5 to 5%, or 3% per weight of the oral dosage form; or

c) a lubricant, for example talc, silica, fats; optionally wherein:

i) the lubricant is magnesium stearate; or
ii) the lubricant is present at 0.1 to 2%, 0.2 to 1.5%, 1%, or 0.5% per weight of the oral dosage form.

**6.** An oral dosage form comprising:

VS-6063, or a pharmaceutically acceptable salt thereof, optionally wherein VS-6063 is VS-6063 free base and/or the pharmaceutically acceptable salt is VS-6063 hydrochloride, in an amount from about 5 to 50% by weight on a dry weight basis,
a pharmaceutically acceptable filler, for example microcrystalline cellulose, optionally wherein the filler is microcrystalline cellulose PH 102, in an amount from about 10 to 30% by weight on a dry weight basis,
a second pharmaceutically acceptable filler, for example lactose, optionally wherein the filler is lactose monohydrate, in an amount from about 10 to 30% by weight on a dry weight basis,
HPMCAS, optionally wherein the HPMCAS is HPMCAS-HF, in an amount from about 25 to 55% by weight on a dry weight basis,
a pharmaceutically acceptable disintegrant, for example starch, optionally wherein the disintegrant is modified starch, optionally wherein the disintegrant is sodium starch glycolate, in an amount from about 0 to 5% by weight on a dry weight basis, and
a pharmaceutically acceptable lubricant, for example magnesium stearate, in an amount from about 0.1 to 2% by weight on a dry weight basis;

optionally wherein the oral dosage form comprises:

VS-6063, or a pharmaceutically acceptable salt thereof, in an amount from about 10 to 15% by weight on a dry weight basis,
the pharmaceutically acceptable filler in an amount from about 15 to 25% by weight on a dry weight basis,
the second pharmaceutically acceptable filler in an amount from about 15 to 25% by weight on a dry weight basis,
the HPMCAS in an amount from about 35 to 55% by weight on a dry weight basis,
the pharmaceutically acceptable disintegrant in an amount from about 2.5 to 3.5% by weight on a dry weight basis, and
the pharmaceutically acceptable lubricant in an amount from about 0.2 to 1.5% by weight on a dry weight basis;
optionally wherein the oral dosage form is a compressed pharmaceutical tablet or a direct compressed pharmaceutical tablet, wherein the tablet is provided by direct compression or dry granulation of a physical blend or mixture of VS-6063, or a pharmaceutically acceptable salt thereof, admixed with the polymer excipient.

**7.** An oral dosage form comprising VS-6063, or a pharmaceutically acceptable salt thereof according to claim 1, for use in treating a subject having been identified with a cancer disorder, for example mesothelioma, triple negative breast cancer, ovarian cancer, or lung cancer; optionally wherein the cancer disorder is surgical resectable malignant pleural mesothelioma, advanced ovarian cancer, non-small cell lung cancer (NSCLC), KRAS mutant NSCLC, or

non-hematologic malignancies; optionally wherein:

a) the oral dosage form comprises 10 to 500 mg of VS-6063, or a pharmaceutically acceptable salt thereof; or
b) the oral dosage form is administered in combination with administration of an additional agent; optionally wherein the additional agent is a cancer therapeutic, optionally wherein the cancer therapeutic is taxane, optionally wherein the taxane is paclitaxel.

**8.** A method for preparing a direct compressed tablet in unit dosage form, which comprises:

(a) blending as a % by weight on a dry weight basis:

(i) 5 to 50% by weight on a dry weight basis of VS-6063;
(ii) HPMCAS; and
(iii) at least one excipient selected from a filler, a disintegrant, and a lubricant;
to form a VS-6063 formulation in the form of a tableting powder, capable of being directly compressed into a tablets; and

(b) compressing the formulation prepared during step (a) to form the compressed VS-6063 tablet in unit dosage form.

**9.** The method of claim 8, wherein:

a) a filler is present at 30 to 60% by weight on a dry weight basis; or
b) a disintegrant is present at 2.5 to 5% by weight on a dry weight basis; or
c) a lubricant is present at 0.5 to 2% by weight on a dry weight basis.

## Patentansprüche

**1.** Pharmazeutische Zusammensetzung, umfassend VS-6063 oder ein pharmazeutisch unbedenkliches Salz davon in Abmischung mit einem Polymerhilfsstoff, wobei es sich bei dem Polymerhilfsstoff um Hydroxypropylmethylcelluloseacetatsuccinat (HPMCAS) handelt, wobei die pharmazeutische Zusammensetzung in einer oralen Dosierungsform in Form einer Tablette konfiguriert ist, wobei die Tablette durch Direktverpressung oder Trockengranulierung eines physikalischen Blends oder einer physikalischen Mischung von VS-6063 oder eines pharmazeutisch unbedenklichen Salzes davon in Abmischung mit dem Polymerhilfsstoff bereitgestellt wird.

**2.** Pharmazeutische Zusammensetzung nach Anspruch 1, wobei die Zusammensetzung die freie Base VS-6063 oder VS-6063-Hydrochlorid umfasst.

**3.** Pharmazeutische Zusammensetzung nach Anspruch 1 oder 2, wobei:

a) das Gewichtsverhältnis von VS-6063 oder einem pharmazeutisch unbedenklichen Salz davon zu dem Polymer etwa 1 zu 1, 1 zu 2, 1 zu 3 oder 1 zu 4 beträgt oder
b) VS-6063 oder ein pharmazeutisch unbedenkliches Salz davon in der Zusammensetzung in einer Menge vorliegt, die etwa 10, 25, 30, 35, 40, 45, 50 % oder mehr w/w, bezogen auf eine bereitgestellte Polymermenge, beträgt, vorliegt oder
c) die Zusammensetzung 5 bis 50 Gew.-%, 5 bis 30 Gew.-%, 10 bis 30 Gew.-%, 10 bis 20 Gew.-%, 12 bis 15 Gew.-% oder 13 Gew.-% VS-6063 oder eines pharmazeutisch unbedenklichen Salzes davon pro Gewicht der oralen Dosierungsform umfasst.

**4.** Pharmazeutische Zusammensetzung nach Anspruch 1, wobei:

a) die Zusammensetzung 5 bis 50 Gew.-%, 5 bis 30 Gew.-%, 10 bis 30 Gew.-%, 10 bis 20 Gew.-%, 12 bis 15 Gew.-% oder 13 Gew.-% VS-6063 oder eines pharmazeutisch unbedenklichen Salzes davon, bezogen auf das Gewicht der oralen Dosierungsform, umfasst oder
b) VS-6063 oder ein pharmazeutisch unbedenkliches Salz davon in einer Menge von 13%, bezogen auf das Gewicht der oralen Dosierungsform, vorliegt oder
c) das Polymer in einer Menge von 25 bis 55 %, 35 bis 45 %, 38 bis 43 % oder 40 %, bezogen auf das Gewicht

der oralen Dosierungsform, vorliegt.

**5.** Pharmazeutische Zusammensetzung nach Anspruch 1, ferner umfassend:

a) einen oder mehrere Füllstoffe, beispielsweise mikrokristalline Cellulose, gegebenenfalls wobei es sich bei der mikrokristallinen Cellulose um mikrokristalline Cellulose PH 102 handelt; oder Lactose, gegebenenfalls wobei es sich bei der Lactose um Lactosemonohydrat handelt; gegebenenfalls wobei:

i) der Füllstoff in einer Menge von 5 bis 80 %, 10 bis 70 %, 20 bis 60 %, 30 bis 60 %, 30 bis 50 % oder 42 %, bezogen auf das Gewicht der oralen Dosierungsform, vorliegt oder
ii) es sich bei dem Füllstoff um eine Mischung von zwei Füllstoffen handelt; gegebenenfalls wobei I) es sich bei einem Füllstoff um mikrokristalline Cellulose PH 102 handelt, die gegebenenfalls in einer Menge von 10 bis 30 %, 15 bis 25 % oder 20 %, bezogen auf das Gewicht der oralen Dosierungsform, vorliegt, und/oder es sich bei einem Füllstoff um Lactosemonohydrat handelt, das gegebenenfalls in einer Menge von 10 bis 30 %, 15 bis 25 % oder 20 %, bezogen auf das Gewicht der oralen Dosierungsform, vorliegt; oder II) die Füllstoffe in einem Verhältnis von 1:1 w/w zueinander vorliegen; oder

b) ein Sprengmittel, gegebenenfalls wobei es sich bei dem Sprengmittel um modifizierte Stärke handelt; gegebenenfalls wobei:

i) es sich bei dem Sprengmittel um Natriumstärkeglykolat handelt oder
ii) das Sprengmittel in einer Menge von 0 bis 5 %, 1 bis 5 %, 2,5 bis 5 % oder 3 %, bezogen auf das Gewicht der oralen Dosierungsform, vorliegt; oder

c) ein Schmiermittel, beispielsweise Talk, Siliciumdioxid, Fette; gegebenenfalls wobei:

i) es sich bei dem Schmiermittel um Magnesiumstearat handelt; oder
ii) das Schmiermittel in einer Menge von 0,1 bis 2 %, 0,2 bis 1,5 %, 1 % oder 0,5 %, bezogen auf das Gewicht der oralen Dosierungsform, vorliegt.

**6.** Orale Dosierungsform, umfassend:

VS-6063 oder ein pharmazeutisch unbedenkliches Salz davon, gegebenenfalls wobei es sich bei VS-6063 um die freie Base VS-6063 handelt und/oder es sich bei dem pharmazeutisch unbedenklichen Salz um VS-6063-Hydrochlorid handelt, in einer Menge von etwa 5 bis 50 Gew.-% auf Trockengewichtsbasis,
einen pharmazeutisch unbedenklichen Füllstoff, beispielsweise mikrokristalline Cellulose, gegebenenfalls wobei es sich bei dem Füllstoff um mikrokristalline Cellulose PH 102 handelt, in einer Menge von etwa 10 bis 30 Gew.-% auf Trockengewichtsbasis,
einen zweiten pharmazeutisch unbedenklichen Füllstoff, beispielsweise Lactose, gegebenenfalls wobei es sich bei dem Füllstoff um Lactosemonohydrat handelt, in einer Menge von etwa 10 bis 30 Gew.-% auf Trockengewichtsbasis,
HPMCAS, gegebenenfalls wobei es sich bei dem HPMCAS um HPMCAS-HF handelt, in einer Menge von etwa 25 bis 55 Gew.-% auf Trockengewichtsbasis,
ein pharmazeutisch unbedenkliches Sprengmittel, beispielsweise Stärke, gegebenenfalls wobei es sich bei dem Sprengmittel um modifizierte Stärke handelt, gegebenenfalls wobei es sich bei dem Sprengmittel um Natriumstärkeglykolat handelt, in einer Menge von etwa 0 bis 5 Gew.-% auf Trockengewichtsbasis und
ein pharmazeutisch unbedenkliches Schmiermittel, beispielsweise Magnesiumstearat, in einer Menge von etwa 0,1 bis 2 Gew.-% auf Trockengewichtsbasis;
gegebenenfalls wobei die orale Dosierungsform VS-6063 oder ein pharmazeutisch unbedenkliches Salz davon in einer Menge von etwa 10 bis 15 Gew.-% auf Trockengewichtsbasis,
den pharmazeutisch unbedenklichen Füllstoff in einer Menge von etwa 10 bis 25 Gew.-% auf Trockengewichtsbasis,
den zweiten pharmazeutisch unbedenklichen Füllstoff in einer Menge von etwa 15 bis 25 Gew.-% auf Trockengewichtsbasis,
das HPMCAS in einer Menge von etwa 35 bis 55 Gew.-% auf Trockengewichtsbasis,
das pharmazeutisch unbedenkliche Sprengmittel in einer Menge von etwa 2,5 bis 3,5 Gew.-% auf Trockengewichtsbasis und
das pharmazeutisch unbedenkliche Schmiermittel in einer Menge von etwa 0,2 bis 1,5 Gew.-% auf Trockengewichtsbasis
umfasst;
gegebenenfalls wobei es sich bei der oralen Dosierungsform um eine verpresste pharmazeutische Tablette oder eine direkt verpresste pharmazeutische Tablette handelt, wobei die Tablette durch Direktverpressung oder Trockengranulierung eines physikalischen Blends oder einer physikalischen Mischung von VS-6063 oder eines pharmazeutisch unbedenklichen Salzes davon in Abmischung mit dem Polymerhilfsstoff bereitgestellt wird.

**7.** Orale Dosierungsform, umfassend VS-6063 oder ein pharmazeutisch unbedenkliches Salz davon nach Anspruch 1, zur Verwendung bei der Behandlung eines Individuums, bei dem eine Krebserkrankung, beispielsweise Mesotheliom, dreifach negativer Brustkrebs, Eierstockkrebs oder Lungenkrebs, festgestellt wurde; gegebenenfalls wobei es sich bei der Krebserkrankung um operativ reserzierbares malignes Pleuramesotheliom, fortgeschrittenen Eierstockkrebs, nichtkleinzelligen Lungenkrebs (NSCLC), KRAS-Mutante-NSCLC oder nichthämatologische Malignitäten handelt; gegebenenfalls wobei:

a) die orale Dosierungsform 10 bis 500 mg von VS-6063 oder einem pharmazeutisch unbedenklichen Salz davon umfasst oder
b) die orale Dosierungsform in Kombination mit der Verabreichung eines zusätzlichen Mittels verabreicht wird; gegebenenfalls wobei es sich bei dem zusätzlichen Mittel um ein Krebstherapeutikum handelt, gegebenenfalls wobei es sich bei dem Krebstherapeutikum um ein Taxan handelt, gegebenenfalls wobei es sich bei dem Taxan um Paclitaxel handelt.

**8.** Verfahren zur Herstellung einer direkt verpressten Tablette in Dosierungseinheitsform, das Folgendes umfasst:

(a) Mischen, als Gew.-% auf Trockengewichtsbasis:

(i) 5 bis 50 Gew.-% VS-6063 auf Trockengewichtsbasis;
(ii) HPMCAS und
(iii) mindestens einen Hilfsstoff, der aus einem Füllstoff, einem Sprengmittel und einem Schmiermittel ausgewählt ist;
zur Bildung einer VS-6063-Formulierung in Form eines Tablettierpulvers, das direkt zu einer Tablette verpresst werden kann; und

(b) Verpressen der in Schritt (a) hergestellten Formulierung zur Bildung der verpressten VS-6063-Tablette in Dosierungseinheitsform.

**9.** Verfahren nach Anspruch 8, wobei:

a) ein Füllstoff in einer Menge von 30 bis 60 Gew.-% auf Trockengewichtsbasis vorliegt oder
b) ein Sprengmittel in einer Menge von 2,5 bis 5 Gew.-% auf Trockengewichtsbasis vorliegt oder
c) ein Schmiermittel in einer Menge von 0,5 bis 2 Gew.-% auf Trockengewichtsbasis vorliegt.

**Revendications**

**1.** Composition pharmaceutique comprenant le VS-6063, ou un sel pharmaceutiquement acceptable correspondant, mélangé avec un excipient de type polymère, l'excipient de type polymère étant un acétate succinate d'hydroxypropylméthylcellulose (HPMCAS), la composition pharmaceutique étant configurée en une forme galénique orale sous la forme d'un cachet, le cachet étant obtenu par compression directe ou par granulation à sec d'un assemblage ou d'un mélange physique de VS-6063, ou d'un sel pharmaceutiquement acceptable correspondant, mélangé avec l'excipient de type polymère.

**2.** Composition pharmaceutique selon la revendication 1, la composition comprenant la base libre du VS-6063 ou le chlorhydrate du VS-6063.

**3.** Composition pharmaceutique selon la revendication 1 ou 2,

a) le rapport en poids du VS-6063, ou d'un sel pharmaceutiquement acceptable correspondant, sur le polymère étant d'environ 1 sur 1, 1 sur 2, 1 sur 3, ou 1 sur 4 ; ou

b) le VS-6063, ou un sel pharmaceutiquement acceptable correspondant, étant présent dans la composition en une quantité qui est d'environ 10, 25, 30, 35, 40, 45, 50 % ou plus p/p par rapport à une quantité de polymère mis à disposition ; ou
c) la composition comprenant 5 à 50 %, 5 à 30 %, 10 à 30 %, 10 à 20 %, 12 à 15 %, ou 13 % de VS-6063, ou d'un sel pharmaceutiquement acceptable correspondant, par poids de la composition.

**4.** Composition pharmaceutique selon la revendication 1,

a) la forme galénique orale comprenant 5 à 50 %, 5 à 30 %, 10 à 30 %, 10 à 20 %, 12 à 15 %, ou 13 % de VS-6063, ou d'un sel pharmaceutiquement acceptable correspondant, par poids de la forme galénique orale ; ou
b) le VS-6063, ou un sel pharmaceutiquement acceptable correspondant, étant présent à raison de 13 % en poids de la forme galénique orale ; ou
c) le polymère étant présent à raison de 25 à 55 %, 35 à 45 %, 38 à 43 %, ou 40 % par poids de la forme galénique orale.

**5.** Composition pharmaceutique selon la revendication 1, comprenant en outre :

a) une ou plusieurs charges, par exemple une cellulose microcristalline, éventuellement la cellulose microcristalline étant la cellulose microcristalline PH 102 ; ou du lactose, éventuellement le lactose étant du monohydrate de lactose ; éventuellement :

i) la charge étant présente à raison de 5 à 80 %, 10 à 70 %, 20 à 60 %, 30 à 60 %, 30 à 50 %, ou 42 % par poids de la forme galénique orale ; ou
ii) la charge étant un mélange de deux charges ; éventuellement :

I) une charge étant la cellulose microcristalline PH 102, éventuellement présente à raison de 10 à 30 %, 15 à 25 %, 20 % par poids de la forme galénique orale, et/ou une charge étant le monohydrate de lactose, éventuellement présent à raison de 10 à 30 %, 15 à 25 %, ou 20 % par poids de la forme galénique orale ; ou
II) les charges étant présentes à raison d'un rapport p/p de 1 : 1 l'une par rapport à l'autre ; ou

b) un désintégrant, éventuellement le désintégrant étant un amidon modifié ; éventuellement :

i) le désintégrant étant un glycolate d'amidon sodique ; ou
ii) le désintégrant étant présent à raison de 0 à 5 %, 1 à 5 %, 2,5 à 5 %, ou 3 % par poids de la forme galénique orale ; ou

c) un lubrifiant, par exemple un talc, une silice, des graisses ; éventuellement :

i) le lubrifiant étant du stéarate de magnésium ; ou
ii) le lubrifiant étant présent à raison de 0,1 à 2 %, 0,2 à 1,5 %, 1 %, ou 0,5 % par poids de la forme galénique orale.

**6.** Forme galénique orale comprenant :

du VS-6063, ou un sel pharmaceutiquement acceptable correspondant, éventuellement le VS-6063 étant la base libre du VS-6063 et/ou le sel pharmaceutiquement acceptable étant le chlorhydrate de VS-6063, en une quantité d'environ 5 à 50 % en poids sur une base de poids sec, une charge pharmaceutiquement acceptable, par exemple une cellulose microcristalline, éventuellement la charge étant la cellulose microcristalline PH 102, en une quantité d'environ 10 à 30 % en poids sur la base du poids sec, une deuxième charge pharmaceutiquement acceptable, par exemple du lactose, éventuellement la charge étant le monohydrate de lactose, en une quantité d'environ 10 à 30 % en poids sur une base de poids sec,
un HPMCAS, éventuellement le HPMCAS étant le HPMCAS-HF, en une quantité d'environ 25 à 55 % en poids sur la base du poids sec,
un désintégrant pharmaceutiquement acceptable, par exemple un amidon, éventuellement le désintégrant étant un amidon modifié, éventuellement le désintégrant étant un glycolate d'amidon sodique, en une quantité d'environ 0 à 5 % en poids sur une base de poids sec, et
un lubrifiant pharmaceutiquement acceptable, par exemple du stéarate de magnésium, en une quantité d'environ

0,1 à 2 % en poids sur une base de poids sec ;
éventuellement la forme galénique orale comprenant :

du VS-6063, ou un sel pharmaceutiquement acceptable correspondant, en une quantité d'environ 10 à 15 % en poids sur une base de poids sec,
la charge pharmaceutiquement acceptable en une quantité d'environ 15 à 25 % en poids sur une base de poids sec,
la deuxième charge pharmaceutiquement acceptable en une quantité d'environ 15 à 25 % en poids sur une base du poids sec,
le HPMCAS en une quantité d'environ 35 à 55 % en poids sur une base du poids sec,
le désintégrant pharmaceutiquement acceptable en une quantité d'environ 2,5 à 3,5 % en poids sur une base de poids sec, et
le lubrifiant pharmaceutiquement acceptable en une quantité d'environ 0,2 à 1,5 % en poids sur une base du poids sec ;
éventuellement, la forme galénique orale étant un cachet pharmaceutique comprimé ou un cachet pharmaceutique comprimé direct, le cachet étant obtenu par compression directe ou par granulation à sec d'un assemblage ou d'un mélange physique de VS-6063, ou d'un sel pharmaceutiquement acceptable correspondant, mélangé avec l'excipient de type polymère.

**7.** Forme galénique orale comprenant du VS-6063, ou un sel pharmaceutiquement acceptable correspondant selon la revendication 1, pour une utilisation dans le traitement d'un sujet chez qui on a identifié un trouble cancéreux, par exemple un mésothéliome, un cancer du sein triplement négatif, un cancer de l'ovaire, ou un cancer du poumon ; éventuellement le trouble cancéreux étant un mésothéliome pleural malin résécable chirurgical, un cancer de l'ovaire avancé, un cancer du poumon non à petites cellules (NSCLC), un NSCLC à mutant KRAS, ou des tumeurs malignes non hématologiques ; éventuellement :

a) la forme galénique orale comprenant 10 à 500 mg de VS-6063, ou d'un sel pharmaceutiquement acceptable correspondant ; ou
b) la forme galénique orale étant administrée en combinaison avec l'administration d'un agent supplémentaire ; éventuellement l'agent supplémentaire étant un composé thérapeutique anticancéreux, éventuellement le composé thérapeutique anticancéreux étant un taxane, éventuellement le taxane étant le paclitaxel.

**8.** Procédé pour la préparation d'un cachet comprimé direct sous une forme galénique unitaire, qui comprend :

(a) l'assemblage en % en poids sur une base de poids sec :

(i) de 5 à 50 % en poids sur une base de poids sec de VS-6063 ;
(ii) un HPMCAS ; et
(iii) au moins un excipient choisi parmi une charge, un désintégrant, et un lubrifiant ;
pour former une formulation de VS-6063 sous forme d'une poudre pour cachet, apte à être directement comprimée en cachets ; et

(b) la compression de la formulation préparée pendant l'étape (a) pour former le cachet de VS-6063 comprimé sous une forme galénique unitaire.

**9.** Procédé selon la revendication 8,

a) une charge étant présente à raison de 30 à 60 % en poids sur une base de poids sec ; ou
b) un désintégrant étant présent à raison de 2,5 à 5 % en poids sur une base de poids sec ; ou
c) un lubrifiant étant présent à raison de 0,5 à 2 % en poids sur la base du poids sec.

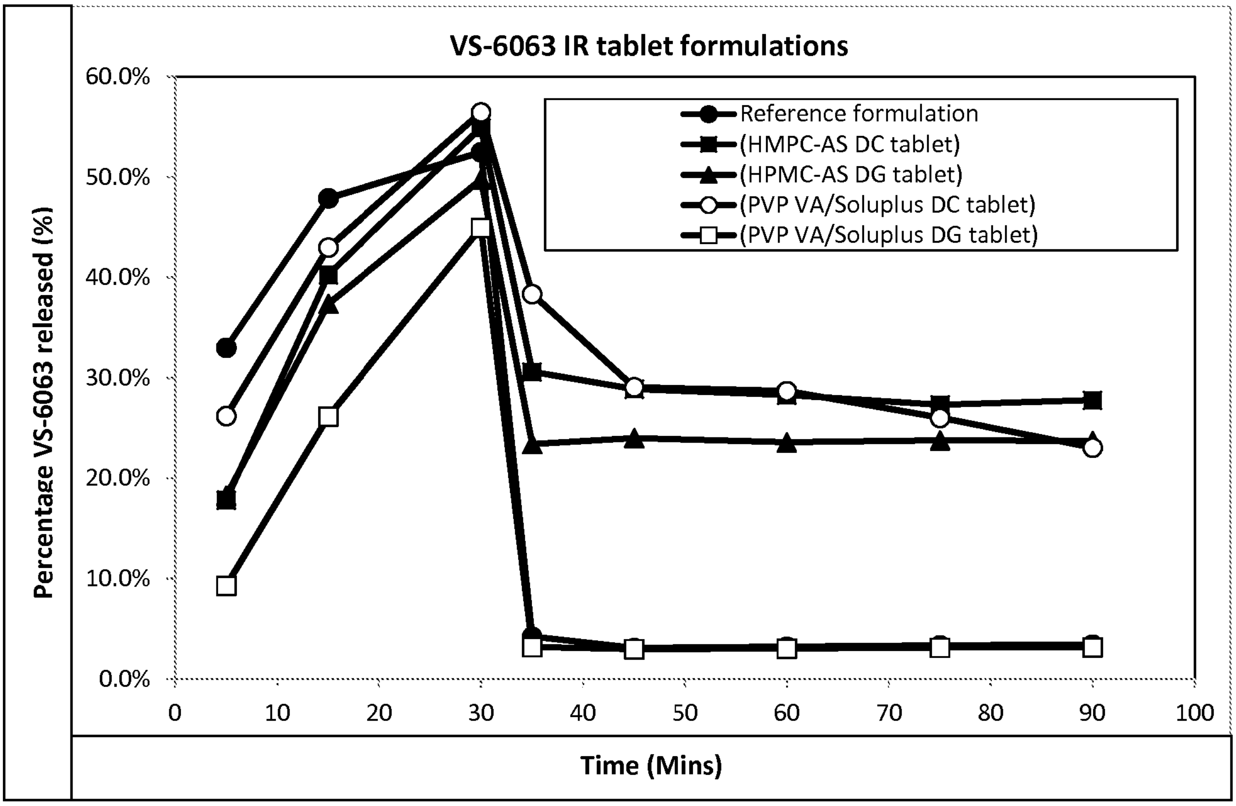
VS-6063 IR tablet formulations
Percentage VS-6063 released (%)
60.0%
50.0%
40.0%
30.0%
20.0%
10.0%
0.0%
0
10
20
30
40
50
60
70
80
90
100
Time (Mins)
Reference formulation
(HMPC-AS DC tablet)
(HPMC-AS DG tablet)
(PVP VA/Soluplus DC tablet)
(PVP VA/Soluplus DG tablet)

FIG. 1

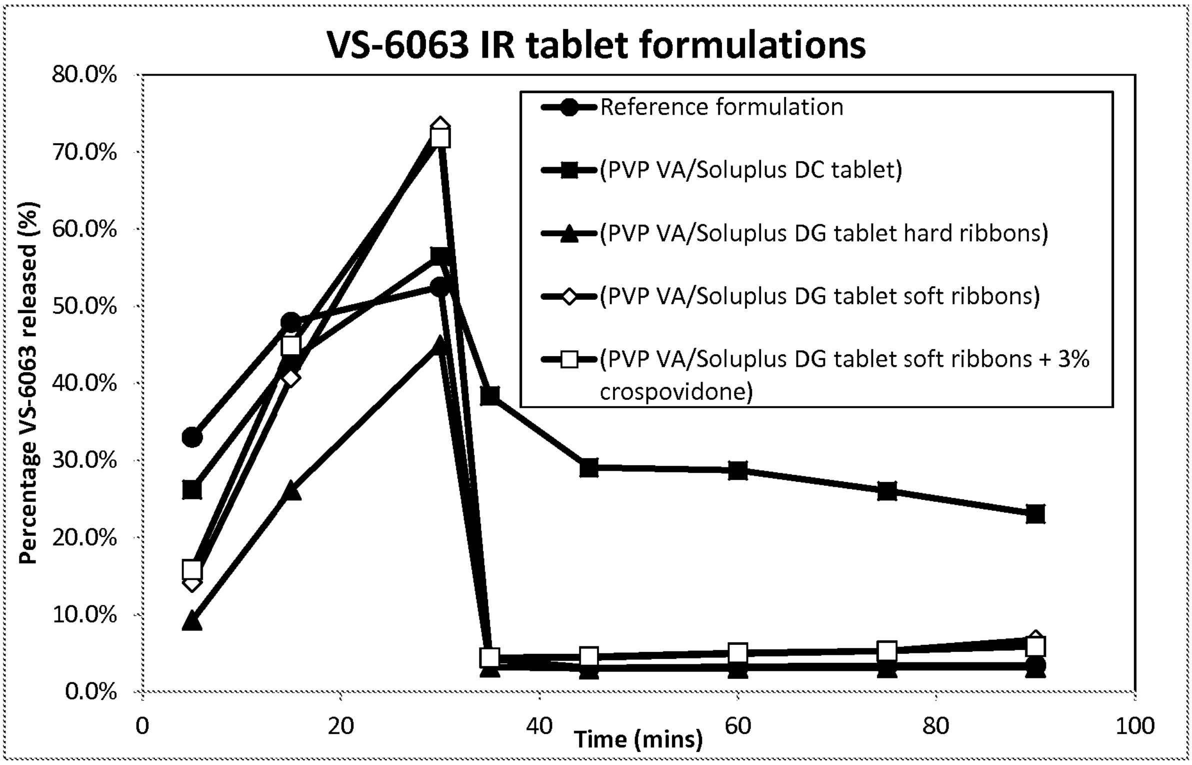
VS-6063 IR tablet formulations
Percentage VS-6063 released (%)
80.0%
70.0%
60.0%
50.0%
40.0%
30.0%
20.0%
10.0%
0.0%
0
20
40
60
80
100
Time (mins)
Reference formulation
(PVP VA/Soluplus DC tablet)
(PVP VA/Soluplus DG tablet hard ribbons)
(PVP VA/Soluplus DG tablet soft ribbons)
(PVP VA/Soluplus DG tablet soft ribbons + 3% crospovidone)

FIG. 2

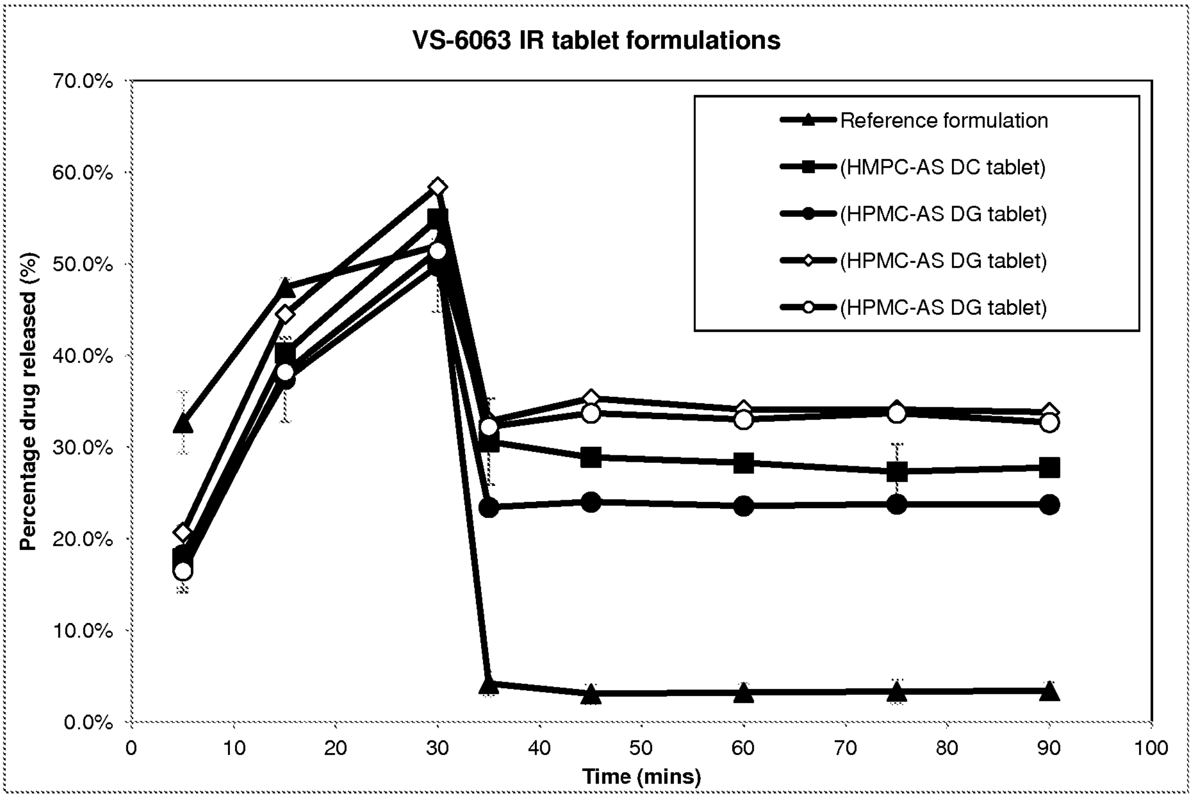
VS-6063 IR tablet formulations
Percentage drug released (%)
70.0%
60.0%
50.0%
40.0%
30.0%
20.0%
10.0%
0.0%
0
10
20
30
40
50
60
70
80
90
100
Time (mins)
Reference formulation
(HMPC-AS DC tablet)
(HPMC-AS DG tablet)
(HPMC-AS DG tablet)
(HPMC-AS DG tablet)

FIG. 3

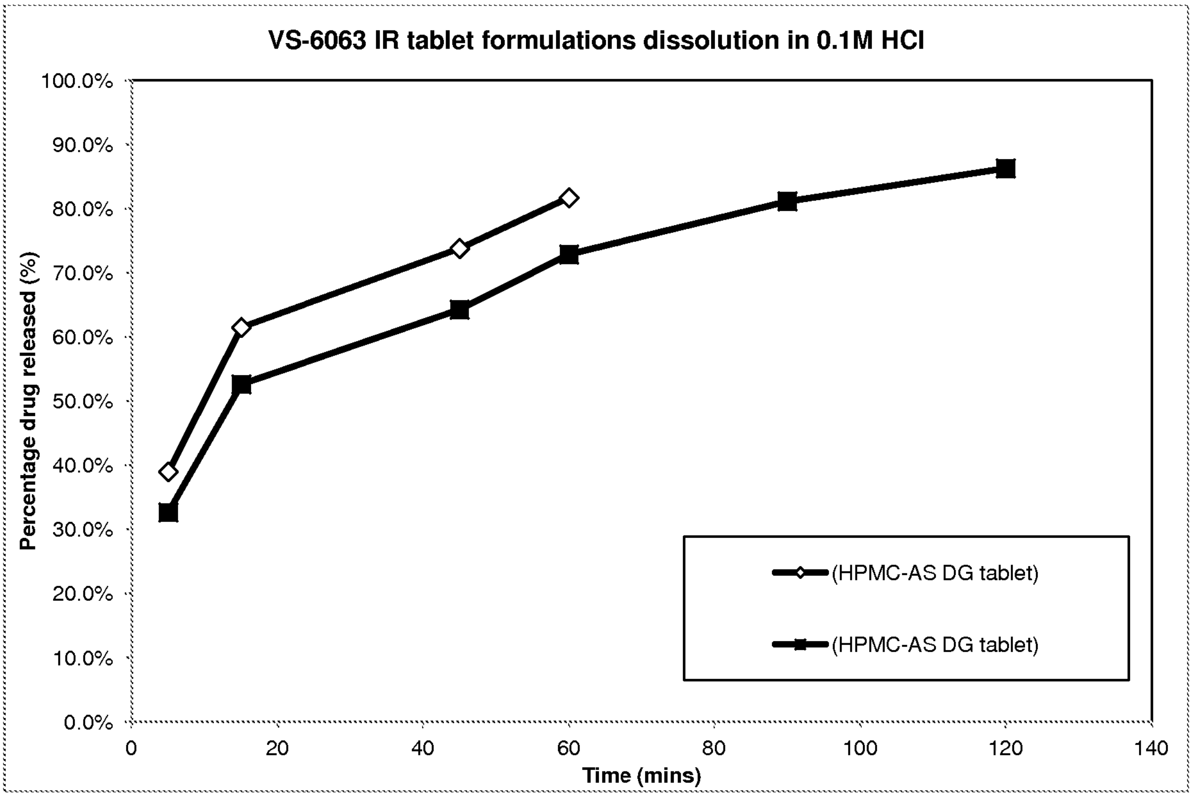
VS-6063 IR tablet formulations dissolution in 0.1M HCl
Percentage drug released (%)
100.0%
90.0%
80.0%
70.0%
60.0%
50.0%
40.0%
30.0%
20.0%
10.0%
0.0%
0
20
40
60
80
100
120
140
Time (mins)
(HPMC-AS DG tablet)
(HPMC-AS DG tablet)

FIG. 4

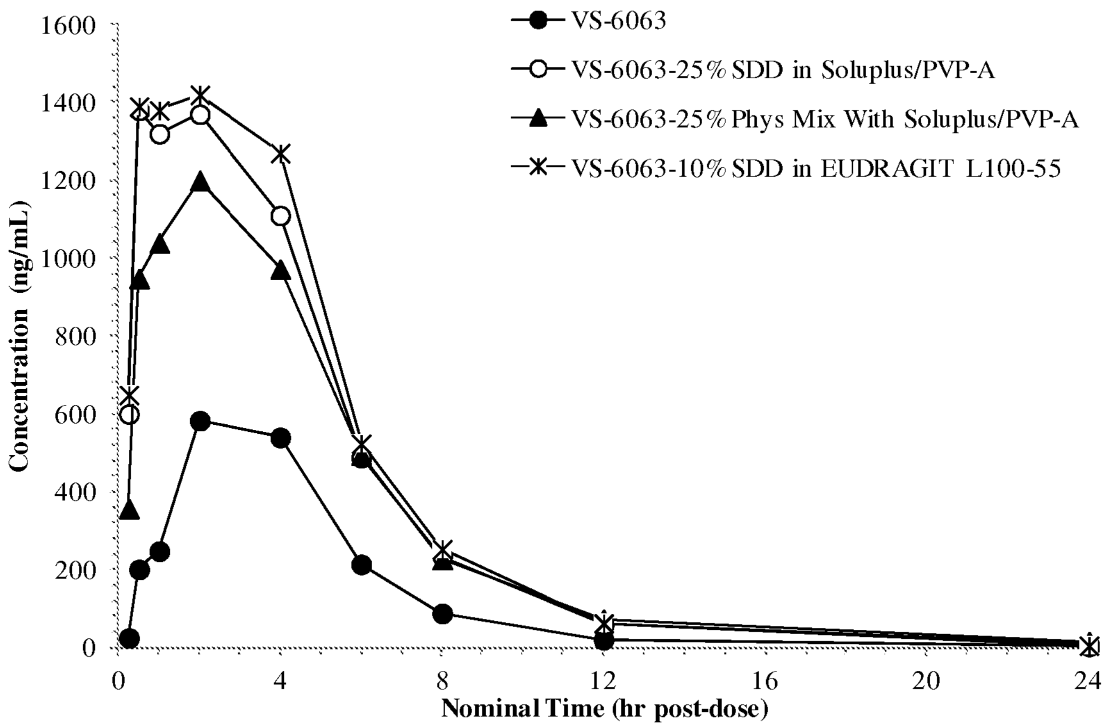

1600
1400
1200
1000
800
600
400
200
0
Concentration (ng/mL)
0
4
8
12
16
20
24
Nominal Time (hr post-dose)
VS-6063
VS-6063-25% SDD in Soluplus/PVP-A
VS-6063-25% Phys Mix With Soluplus/PVP-A
VS-6063-10% SDD in EUDRAGIT L100-55


FIG. 5

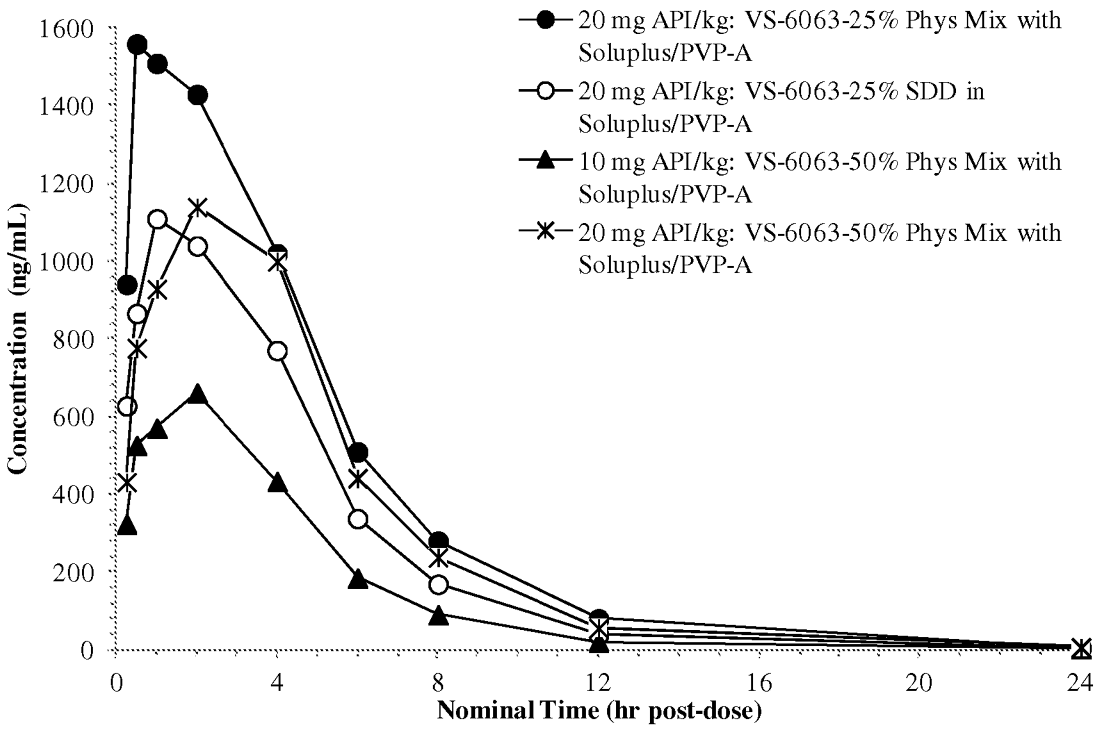
20 mg API/kg: VS-6063-25% Phys Mix with Soluplus/PVP-A
20 mg API/kg: VS-6063-25% SDD in Soluplus/PVP-A
10 mg API/kg: VS-6063-50% Phys Mix with Soluplus/PVP-A
20 mg API/kg: VS-6063-50% Phys Mix with Soluplus/PVP-A
Concentration (ng/mL)
1600
1400
1200
1000
800
600
400
200
0
0
4
8
12
16
20
24
Nominal Time (hr post-dose)

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8247411 B
- WO 2008129380 A
- US 7928109 B


### Non-patent literature cited in the description


- **CLARK ; BRUGGE.** *Science,* 1995, vol. 268, 233-239
- **OWENS et al.** *Cancer Research,* 1995, vol. 55, 2752-2755
- **XU et al.** *Cell Growth and Diff.,* 1996, vol. 7, 413-418
- **KANG et al.** *JNCI Journal of the National Cancer Institute,* 2013, vol. 105, 1485-1495
- **REHAB et al.** *Powder Technology,* 2012, vol. 237, 406-414
- **CURATOLO et al.** *Pharmaceutical Research,* 2009, vol. 26, 1419-1431
- **DINUZIO et al.** *Drug Development and Industrial Pharmacy,* 2010, vol. 36, 1064-1078
- **FRIESEN et al.** *Molecular Pharmaceuticals,* 2008, vol. 5, 1003-1019
- **HELLRIEGEL et al.** *Clinical Pharmacology and Therapeutics,* 1996, vol. 60, 601-607
- Remington's Pharmaceutical Sciences. Mack Publishing Company, 1985, 1418
- Journal of Pharmaceutical Science. 1977, vol. 66, 2
- Handbook of Pharmaceutical Excipients. Pharmaceutical Press
- **FINGL et al.** *The Pharmacological Basis of Therapeutics,* 1975